(19) Europäisches Patentamt
European Patent Office
Office européen des brevets

(11) EP 4 257 597 A1

(12) **EUROPEAN PATENT APPLICATION**
published in accordance with Art. 153(4) EPC

(43) Date of publication:
**11.10.2023 Bulletin 2023/41**

(21) Application number: **21900082.5**

(22) Date of filing: **02.12.2021**

(51) International Patent Classification (IPC):
***C07K 14/605*** *(2006.01)* ***A61K 38/26*** *(2006.01)*
***A61P 3/00*** *(2006.01)* ***A61P 3/10*** *(2006.01)*

(52) Cooperative Patent Classification (CPC):
**A61K 38/26; A61P 3/00; A61P 3/10; C07K 14/605**

(86) International application number:
**PCT/CN2021/135180**

(87) International publication number:
**WO 2022/117056 (09.06.2022 Gazette 2022/23)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **02.12.2020 CN 202011409947**
**02.12.2020 CN 202011402979**
**21.04.2021 CN 202110432060**
**27.05.2021 CN 202110587056**

(71) Applicant: **Dongbao Purple Star (Hangzhou) Biopharmaceutical Co., Ltd.**
**Hangzhou, Zhejiang 310018 (CN)**

(72) Inventors:
- **PAN, Zhixiang**
  **Shanghai 200131 (CN)**
- **JIANG, Zhigan**
  **Shanghai 200131 (CN)**
- **HE, Haiying**
  **Shanghai 200131 (CN)**
- **HU, Guoping**
  **Shanghai 200131 (CN)**
- **LI, Jian**
  **Shanghai 200131 (CN)**
- **CHEN, Shuhui**
  **Shanghai 200131 (CN)**

(74) Representative: **Rösler Rasch van der Heide & Partner**
**Bodenseestraße 18**
**81241 München (DE)**

Remarks:
The complete document including Reference Table(s) and the Sequence Listing(s) can be downloaded from the EPO website

# (54) LACTAM-MODIFIED POLYPEPTIDE COMPOUNDS

(57) A class of lactam-modified polypeptide compounds and the use thereof in the preparation of a drug for treating related diseases.



Processed by Luminess, 75001 PARIS (FR)

## Description

The present application claims the right of the following priorities:

CN202011402979.7, the application date is December 02, 2020;
CN202011409947.X, the application date is December 02, 2020;
CN202110432060.0, the application date is April 21, 2021;
CN202110587056.1, the application date is May 27, 2021.

TECHNICAL FIELD

The present disclosure relates to a class of polypeptide compounds containing lactam modification, and the use thereof in the manufacture of a medicament for treating related diseases.

BACKGROUND

It is estimated that there are 110 million diabetics in China, accounting for 24% of diabetics in the world. With economic development and lifestyle changes, the prevalence of diabetes in China has increased to 12.8% (as high as 19.9% in some provinces and cities), which is accompanied by obesity or cardiovascular disease (CVD). Recent studies have found that a glucose-dependent insulinotropic peptide (GIP)/glucagon-like peptide-1 (GLP-1) dual agonist can be used for treating diabetes.
GLP-1 plays a role in protecting islet β cells in islets and effectively controls postprandial blood glucose by stimulating the islet β cells to release insulin in a glucose-dependent manner. Due to GLP-1's unique mechanism of action, the risk of hypoglycemia is greatly reduced. Although GLP-1R agonists have demonstrated excellent glucose-lowering effect in clinical practice, there are still many type 2 diabetics who have not achieved their glycemic and weight goals. Therefore, combining GLP-1R with other glucose-lowering targets such as GIP for treating type 2 diabetes is an urgent and promising need. GIP is a polypeptide secreted by neuroendocrine K cells of small intestine, and GIP's physiological actions are mediated by GIPR including non-glucose-dependent insulinotropic effects, stimulation of glucagon secretion, and enhancement of lipid metabolism, etc. Although the beneficial effects of GIPR agonists appear to be attenuated in hyperglycemic symptoms in type 2 diabetics, studies have shown that the diminished insulinotropic effect of GIP can be fully restored after a period of normalization of plasma glucose levels. This indicates that co-agonism of GLP-1R/GIPR can exert a synergistic glucose-lowering effect. A GIP and GLP-1 dual agonist has a synergistic effect on the regulation of glucose/lipid metabolism and has better therapeutic effects on lowering blood glucose, reducing body weight, and alleviating liver fat.

CONTENT OF THE PRESENT INVENTION

The present disclosure provides a compound represented by the following formula or a pharmaceutically acceptable salt thereof,

SEQ ID NO1: YAibEGT FTSDY SIAibLD $KK^1AZ_0K_0$ $Z_1FZ_2E_0W$ $LZ_3AGG$ PSSGA $PPPS_0$
SEQ ID NO2: YAibEGT FTSDY SIAibLD $KE_0AQK_0$ AFVK'W LIAGG PSSGA $PPPS_0$
SEQ ID NO3: YAibEGT FTSDY $SIE_0LD$ $KK_0AQK^1$ AFVQW LIAGG PSSGA $PPPS_0$
SEQ ID NO4: YAibEGT FTSDY $SIE_0LD$ $K_0IAQK^1$ AFVQW LIAGG PSSGA $PPPS_0$

wherein

Aib has a structure of

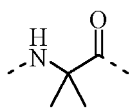
;

$S_0$ is selected from

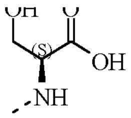 and 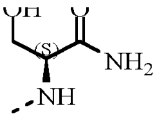 ;

$Z_0$ is selected from glutamine (Q) and asparagine (N);
$Z_1$ is selected from alanine (A) and glutamic acid (E);
$Z_2$ is selected from valine (V) and isoleucine (I);
$Z_3$ is selected from isoleucine (I) and leucine (L);
$E_0$ and $K_0$ indicate that the carboxyl group on the side chain of the glutamic acid and the amino group on the side chain of the lysine jointly form a lactam structure, and in this case,
$K_0Z_1FZ_2E_0$ has a structure of

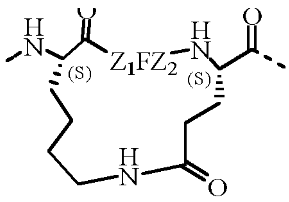

,

$E_0AQK_0$ has a structure of

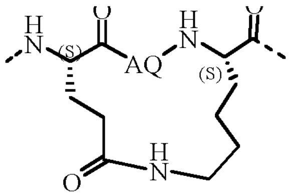

,

$E_0LDKK_0$ has a structure of

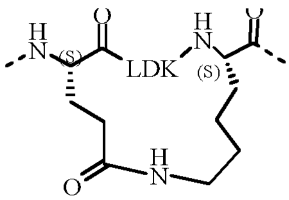

,

and $E_0LDK_0$ has a structure of

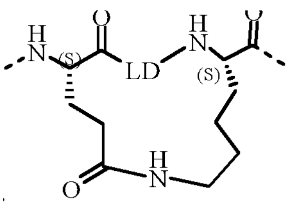

;

$K^1$ indicates that the amino group on the side chain of the lysine is connected to $-X-X_1-X_2$, and has a structure of

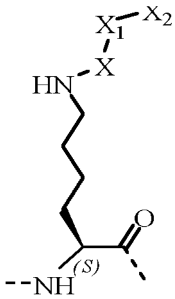

;

X is selected from

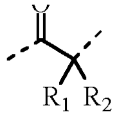

;

$R_1$ and $R_2$ are each independently selected from Hand $CH_3$;
$X_1$ is selected from

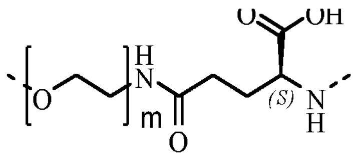

,

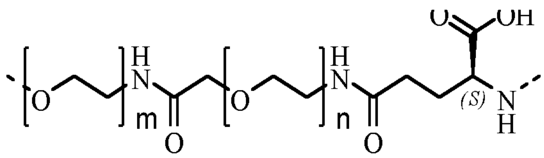

,

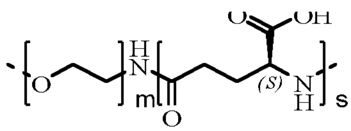

,

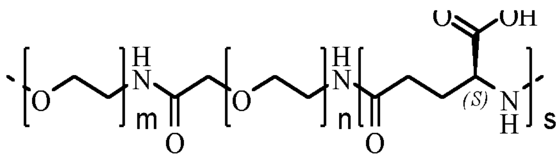

and

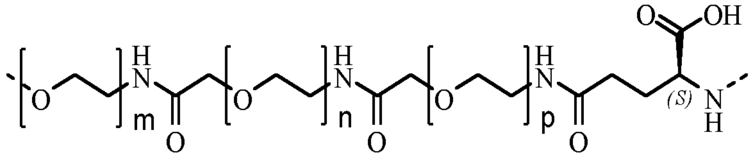

;

$X_2$ is selected from

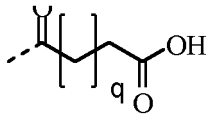

;

m, n and p are each independently selected from 2 and 3;
s is selected from 2 and 3;
q is selected from 15, 16, 17, 18 and 19.

In some embodiments of the present disclosure, the compound or the pharmaceutically acceptable salt thereof is selected from:

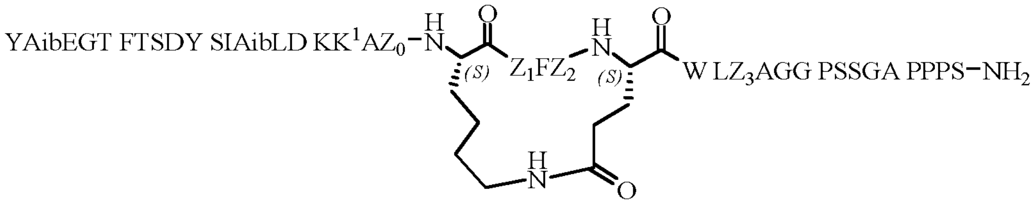

**(P-1)**

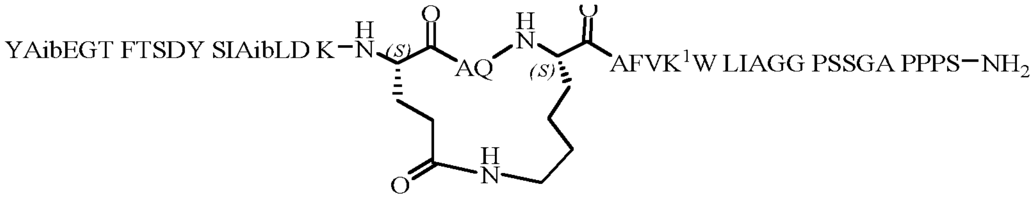

**(P-2)**

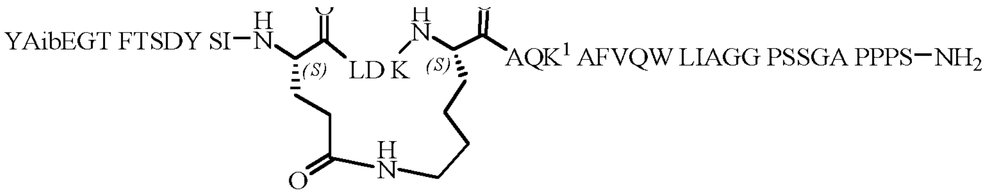

**(P-3)**

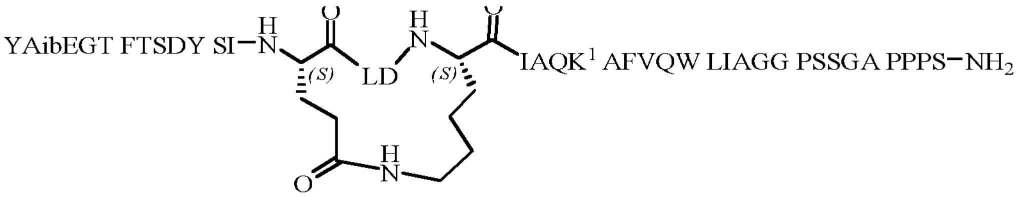

**(P-4)**

wherein

Aib, $Z_0$, $Z_1$, $Z_2$, $Z_3$ and $K^1$ are as defined in the present disclosure.

In some embodiments of the present disclosure, the X is selected from $-C(=O)-CH_2-$, and other variables are as defined in the present disclosure.

In some embodiments of the present disclosure, the m, n and p are each independently selected from 2, and other variables are as defined in the present disclosure.

In some embodiments of the present disclosure, the s is selected from 2, and other variables are as defined in the present disclosure.

In some embodiments of the present disclosure, the q is selected from 15 and 17, and other variables are as defined in the present disclosure.

In some embodiments of the present disclosure, the $X_1$ is selected from

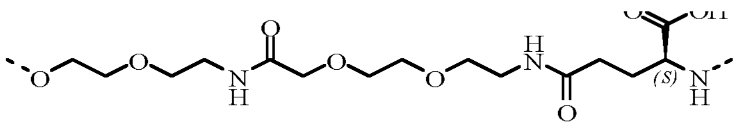

and

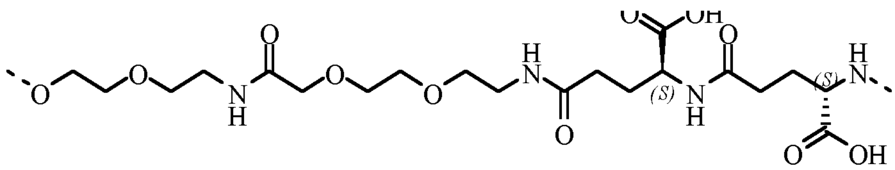

,

and other variables are as defined in the present disclosure.

In some embodiments of the present disclosure, the $X_2$ is selected from

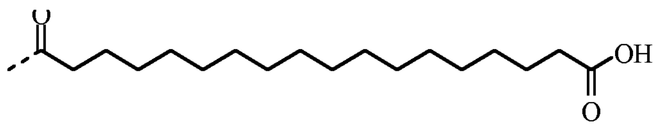

and

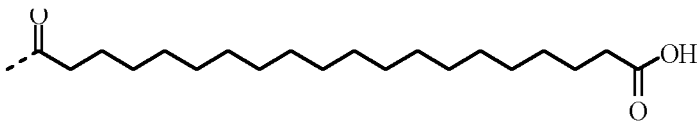

,

and other variables are as defined in the present disclosure.

In some embodiments of the present disclosure, the $-X-X_1-X_2$ is selected from

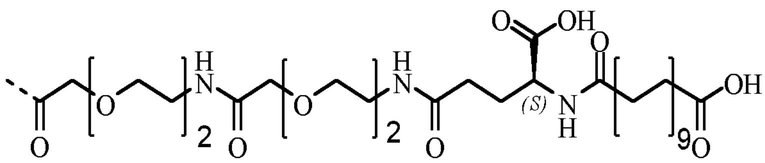

,

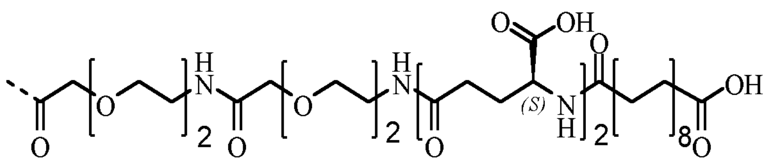

and

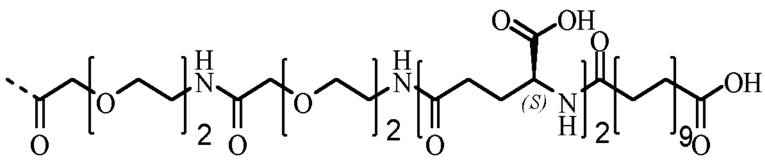

,

and other variables are as defined in the present disclosure.

The present disclosure provides a compound represented by the following formula or a pharmaceutically acceptable salt thereof,

YAibEGT FTSDY SIAibLD KK$^1$AQK$_0$ AFVE$_0$W LIAGG PSSGA PPPS$_0$
YAibEGT FTSDY SIAibLD KK$^1$ANK$_0$ AFVE$_0$W LIAGG PSSGA PPPS$_0$
YAibEGT FTSDY SIAibLD KK$^1$AQK$_0$ EFVE$_0$W LIAGG PSSGA PPPS$_0$
YAibEGT FTSDY SIAibLD KK$^1$AQK$_0$ AFIE$_0$W LIAGG PSSGA PPPS$_0$
YAibEGT FTSDY SIAibLD KK$^1$AQK$_0$ AFVE$_0$W LLAGG PSSGA PPPS$_0$

wherein

Aib has a structure of

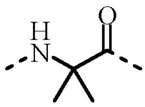

;

$S_0$ is selected from

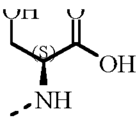

and

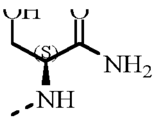

;

$K_0$ and $E_0$ indicate that the amino group on the side chain of the lysine and the carboxyl group on the side chain of the glutamic acid jointly form a lactam structure, and have a structure of

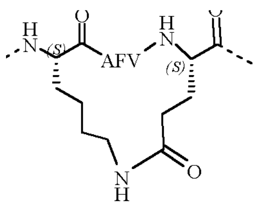

,

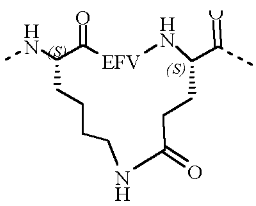

or

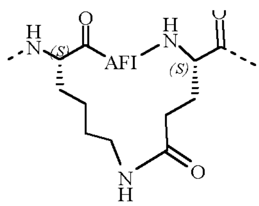

;

$K^1$ indicates that the amino group on the side chain of the lysine is connected to -X-$X_1$-$X_2$, and has a structure of

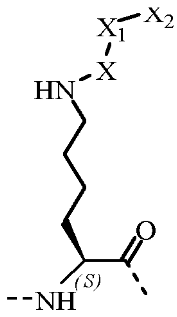

;

X is selected from

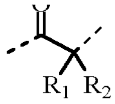

;

$R_1$ and $R_2$ are each independently selected from H;
$X_1$ is selected from

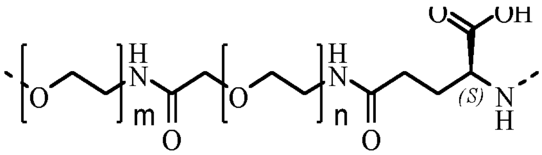

and

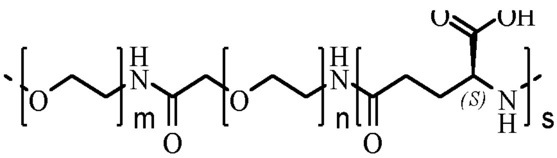

;

$X_2$ is selected from

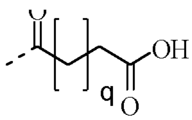

;

m and n are each independently selected from 2 and 3;
s is selected from 2 and 3;
q is selected from 15, 16, 17, 18 and 19.

In some embodiments of the present disclosure, the X is selected from $-C(=O)-CH_2-$, and other variables are as defined in the present disclosure.

In some embodiments of the present disclosure, the m and n are each independently selected from 2, and other variables are as defined in the present disclosure.

In some embodiments of the present disclosure, the s is selected from 2, and other variables are as defined in the present disclosure.

In some embodiments of the present disclosure, the q is selected from 15 and 17, and other variables are as defined in the present disclosure.

In some embodiments of the present disclosure, the $X_1$ is selected from

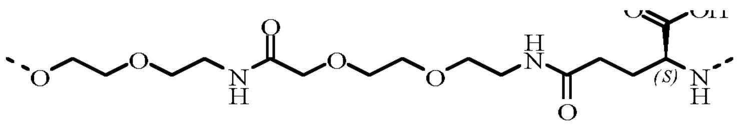

and

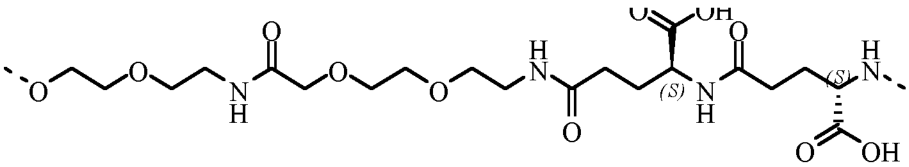

,

and other variables are as defined in the present disclosure.

In some embodiments of the present disclosure, the $X_2$ is selected from

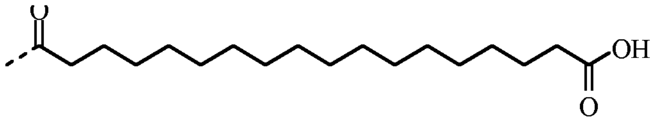

and

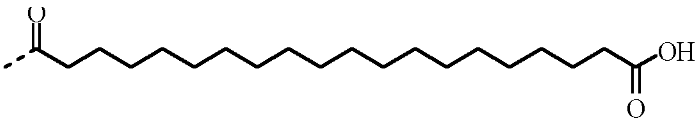

,

and other variables are as defined in the present disclosure.

In some embodiments of the present disclosure, the $-X-X_1-X_2$ is selected from

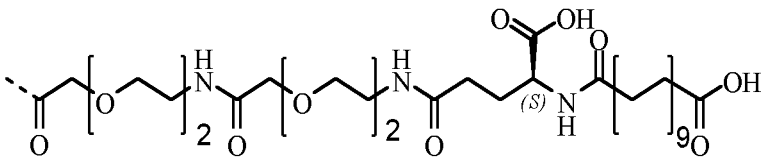

,

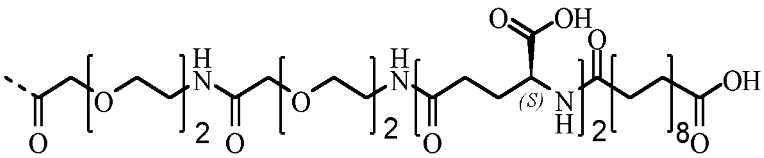

and

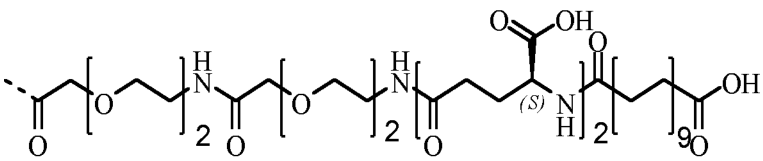

,

and other variables are as defined in the present disclosure.

The present disclosure provides a compound represented by the following formula or a pharmaceutically acceptable salt thereof,

YAibEGT FTSDY SIAibLD $KK^1AZ_0K_0$ $Z_1FZ_2E_0W$ $LZ_3AGG$ PSSGA $PPPS_0$
YAibEGT FTSDY SIAibLD $KE_0AQK_0$ AFVK'W LIAGG PSSGA $PPPS_0$
YAibEGT FTSDY $SIE_0LD$ $KK_0AQK^1$ AFVQW LIAGG PSSGA $PPPS_0$

wherein

Aib has a structure of

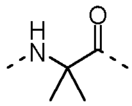
;

$S_0$ is selected from

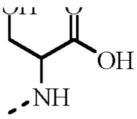
and
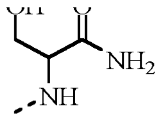
;

$Z_0$ is selected from glutamine (Q) and asparagine (N);
$Z_1$ is selected from alanine (A) and glutamic acid (E);
$Z_2$ is selected from valine (V) and isoleucine (I);
$Z_3$ is selected from isoleucine (I) and leucine (L);
$E_0$ and $K_0$ indicate that the carboxyl group on the side chain of the glutamic acid and the amino group on the side chain of the lysine jointly form a lactam structure, and in this case, $K_0Z_1FZ_2E_0$ has a structure of

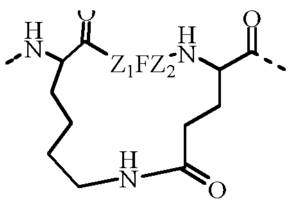
,

$E_0AQK_0$ has a structure of

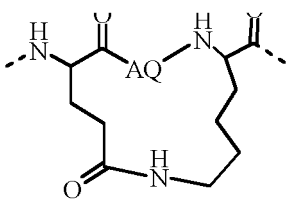
,

$E_0LDKK_0$ has a structure of

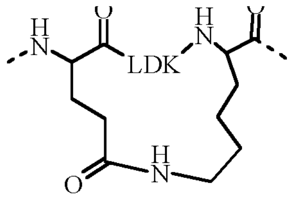

;

$K^1$ indicates that the amino group on the side chain of the lysine is connected to -X-$X_1$-$X_2$, and has a structure of

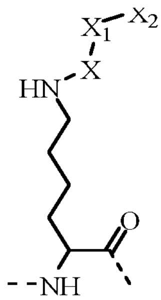

;

X is selected from

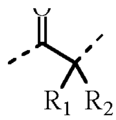

;

$R_1$ and $R_2$ are each independently selected from H;
$X_1$ is selected from

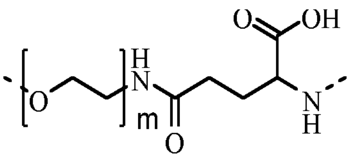

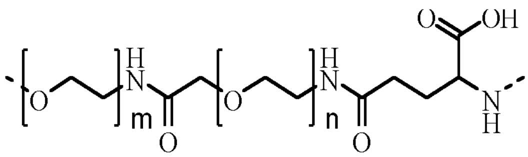

,

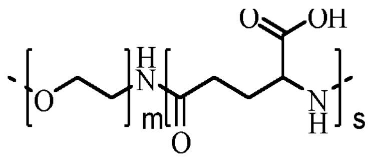

,

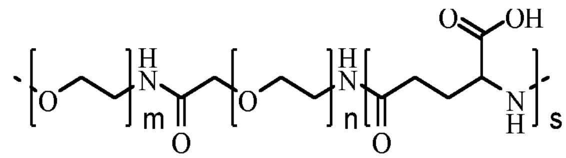

,

and

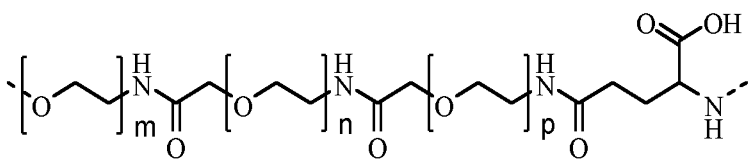

;

$X_2$ is selected from

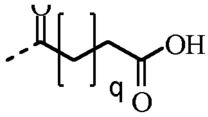

;

m, n and p are each independently selected from 2 and 3;
s is independently selected from 2 and 3;
q is independently selected from 15, 16, 17, 18 and 19.

In some embodiments of the present disclosure, the X is selected from $-C(=O)-CH_2-$, and other variables are as defined in the present disclosure.

In some embodiments of the present disclosure, the m, n and p are each independently selected from 2, and other variables are as defined in the present disclosure.

In some embodiments of the present disclosure, the s is independently selected from 2, and other variables are as defined in the present disclosure.

In some embodiments of the present disclosure, the q is independently selected from 15 and 17, and other variables are as defined in the present disclosure.

In some embodiments of the present disclosure, the q is independently selected from 17, and other variables are as defined in the present disclosure.

In some embodiments of the present disclosure, the $X_1$ is selected from

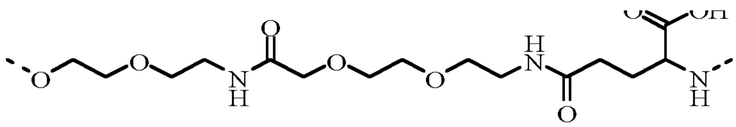

and

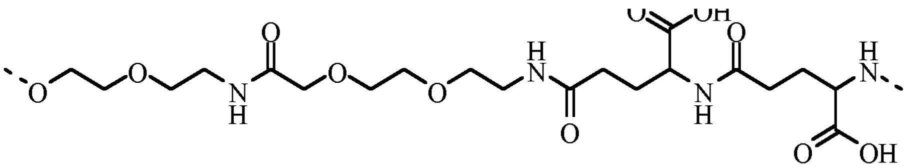

,

and other variables are as defined in the present disclosure.

In some embodiments of the present disclosure, the $X_2$ is selected from

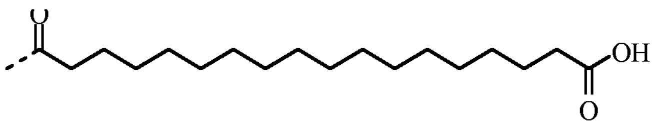

and

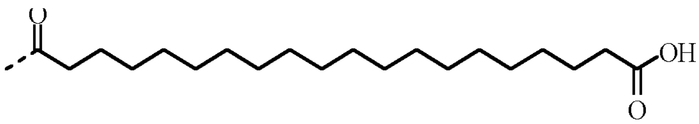

,

and other variables are as defined in the present disclosure.

In some embodiments of the present disclosure, the $-X-X_1-X_2$ is selected from

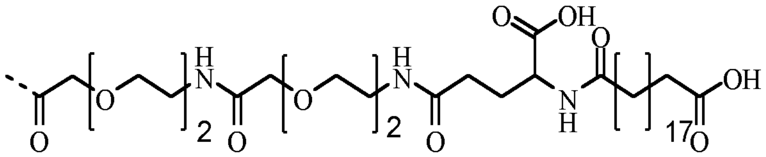

,

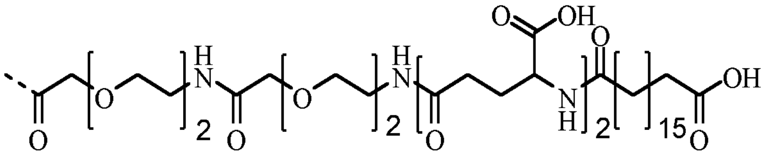

and

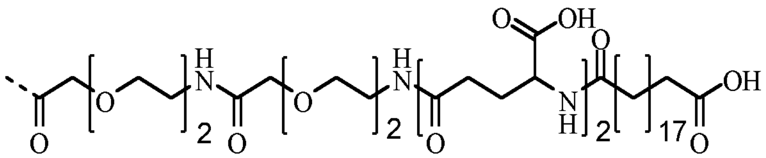

,

and other variables are as defined in the present disclosure.

The present disclosure provides a compound represented by the following formula or a pharmaceutically acceptable salt thereof,

YAibEGT FTSDY SIAibLD $KK^1AQK_0$ $AFVE_0W$ LIAGG PSSGA $PPPS_0$

wherein

Aib has a structure of

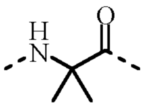

;

$S_0$ is selected from

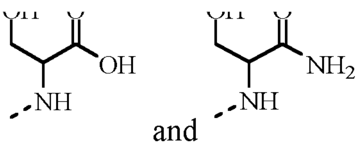

;

$K_0$ and $E_0$ indicate that the amino group on the side chain of the lysine and the carboxyl group on the side chain of the glutamic acid jointly form a lactam structure, and have a structure of

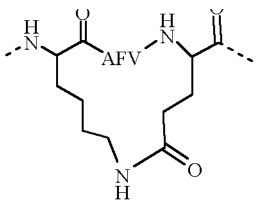

;

$K^1$ indicates that the amino group on the side chain of the lysine is connected to $-X-X_1-X_2$, and has a structure of

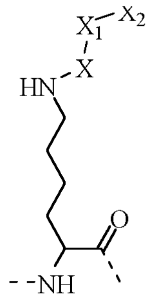

;

X is selected from

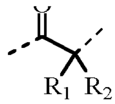

;

$R_1$ and $R_2$ are each independently selected from H;

$X_1$ is selected from

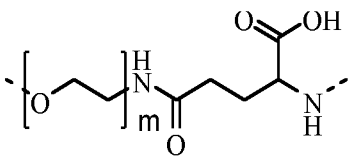

,

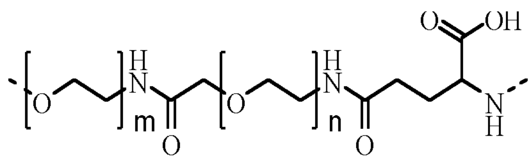

and

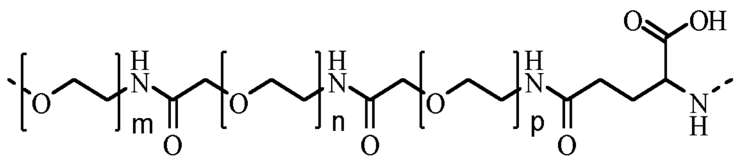

;

$X_2$ is selected from

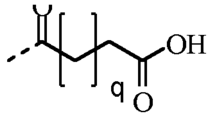

;

m, n and p are each independently selected from 2 and 3;

q is independently selected from 15, 16, 17, 18 and 19.

In some embodiments of the present disclosure, the X is selected from -C(=O)-$CH_2$-, and other variables are as defined in the present disclosure.

In some embodiments of the present disclosure, the m, n and p are each independently selected from 2, and other variables are as defined in the present disclosure.

In some embodiments of the present disclosure, the q is independently selected from 17, and other variables are as defined in the present disclosure.

In some embodiments of the present disclosure, the $X_1$ is selected from

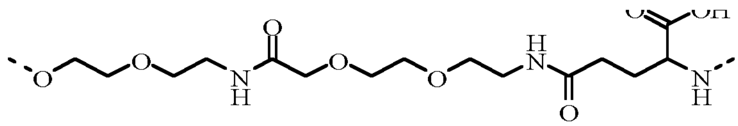

,

and other variables are as defined in the present disclosure.

In some embodiments of the present disclosure, the $X_2$ is selected from

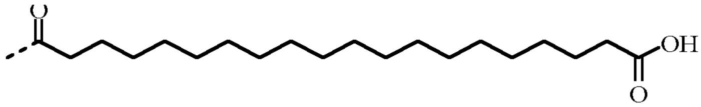

,

and other variables are as defined in the present disclosure.

In some embodiments of the present disclosure, the $-X-X_1-X_2$ is selected from

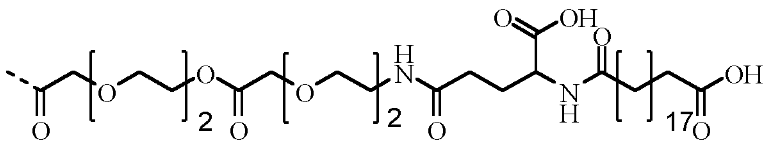

,

and other variables are as defined in the present disclosure.

The present disclosure provides a compound represented by the following formula or a pharmaceutically acceptable salt thereof,

YAibEGT FTSDY SI$E_0$LD $K_0$IAQ$K^1$ AFVQW LIAGG PSSGA PPP$S_0$

wherein

Aib has a structure of

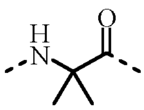

;

$S_0$ is selected from

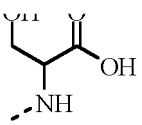

and

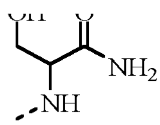

;

$E_0$ and $K_0$ indicate that the carboxyl group on the side chain of the glutamic acid and the amino group on the side chain of the lysine jointly form a lactam structure, and have a structure of

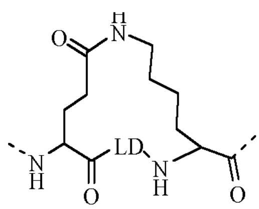

;

$K^1$ indicates that the amino group on the side chain of the lysine is connected to -X-$X_1$-$X_2$, and has a structure of

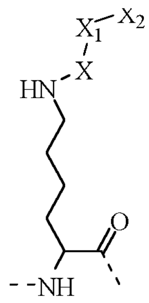

;

X is selected from

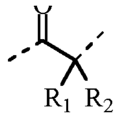

;

$R_1$ and $R_2$ are each independently selected from H;
$X_1$ is selected from

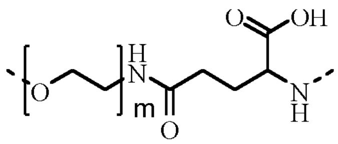

,

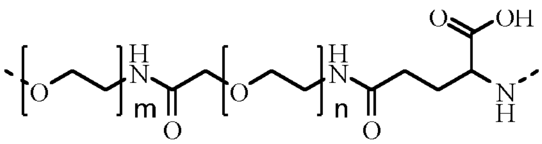

and

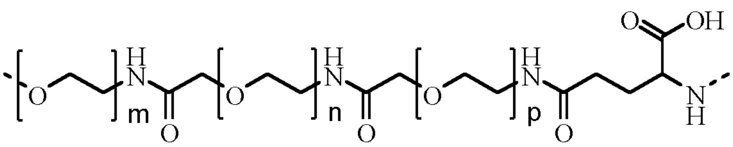

;

$X_2$ is selected from

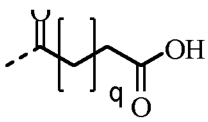

;

m, n and p are each independently selected from 2 and 3;
q is independently selected from 15, 16, 17, 18 and 19.

In some embodiments of the present disclosure, the X is selected from -C(=O)-$CH_2$-, and other variables are as defined in the present disclosure.

In some embodiments of the present disclosure, the m, n and p are each independently selected from 2, and other variables are as defined in the present disclosure.

In some embodiments of the present disclosure, the q is independently selected from 17, and other variables are as defined in the present disclosure.

In some embodiments of the present disclosure, the $X_1$ is selected from

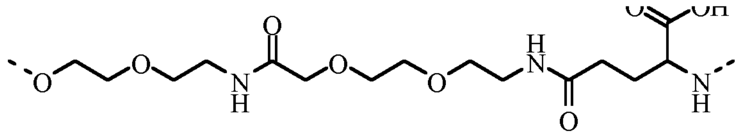

,

and other variables are as defined in the present disclosure.

In some embodiments of the present disclosure, the $X_2$ is selected from

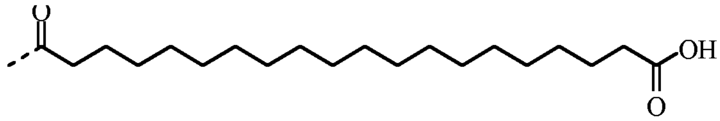

,

and other variables are as defined in the present disclosure.

In some embodiments of the present disclosure, the -X-$X_1$-$X_2$ is selected from

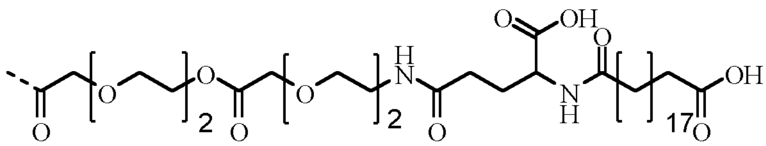

,

and other variables are as defined in the present disclosure.

There are still some embodiments of the present disclosure which are obtained by any combination of the above variables.

The present disclosure also provides a compound represented by the following formula or a pharmaceutically acceptable salt thereof,

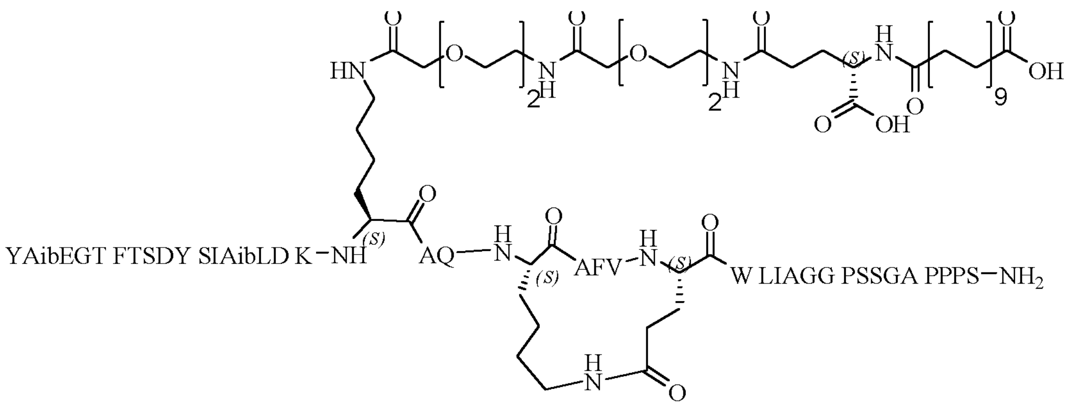

**WX-001**

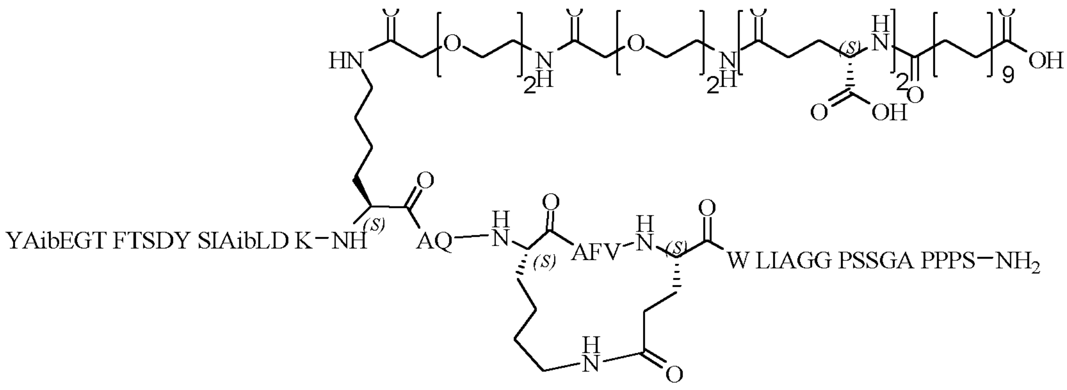

**WX-002**

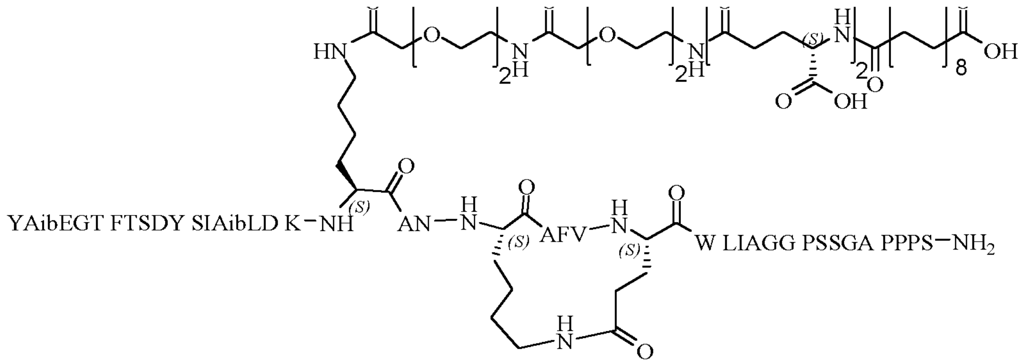

**WX-003**

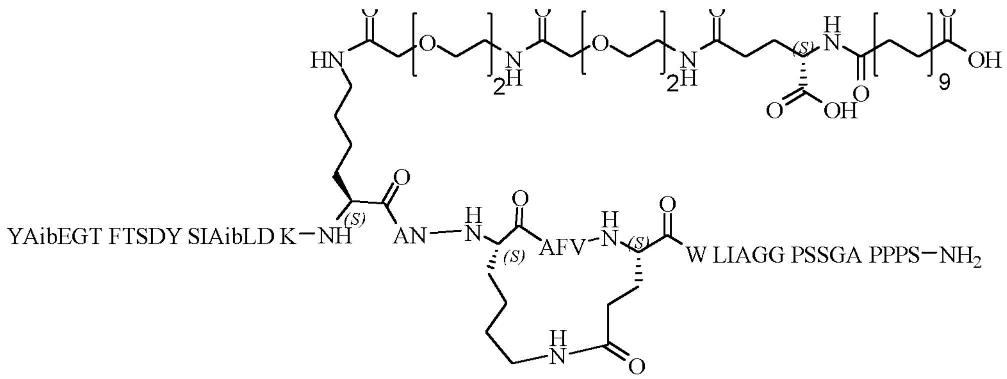

**WX-004**

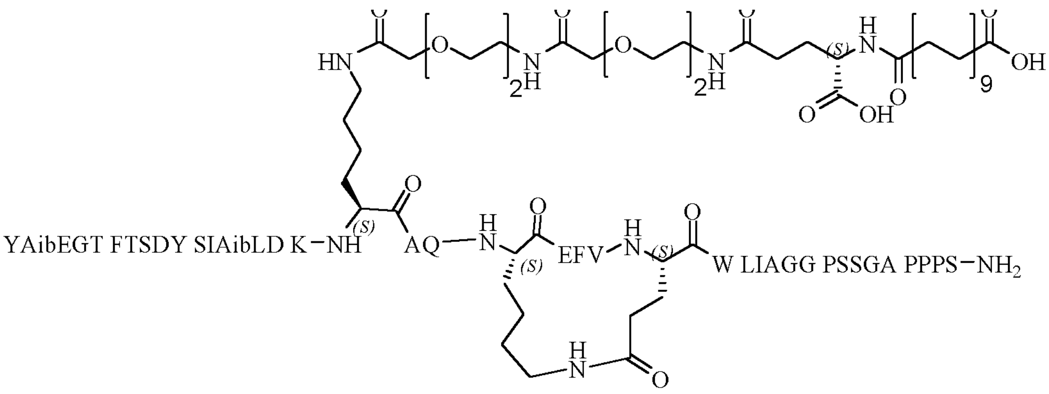

**WX-005**

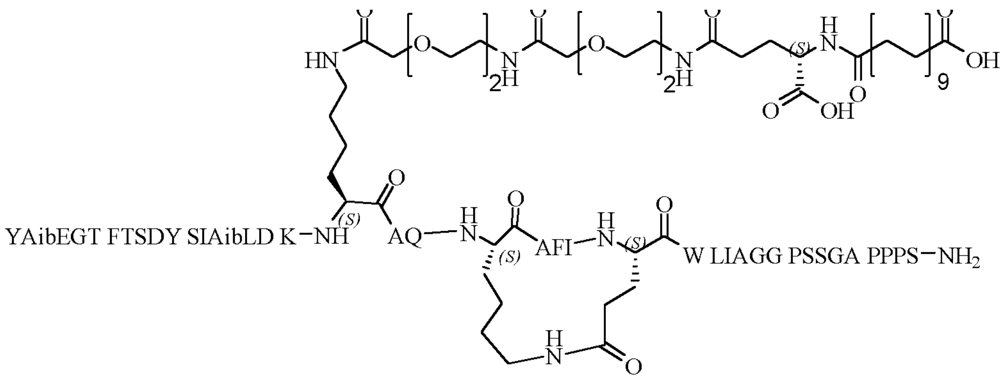

**WX-006**

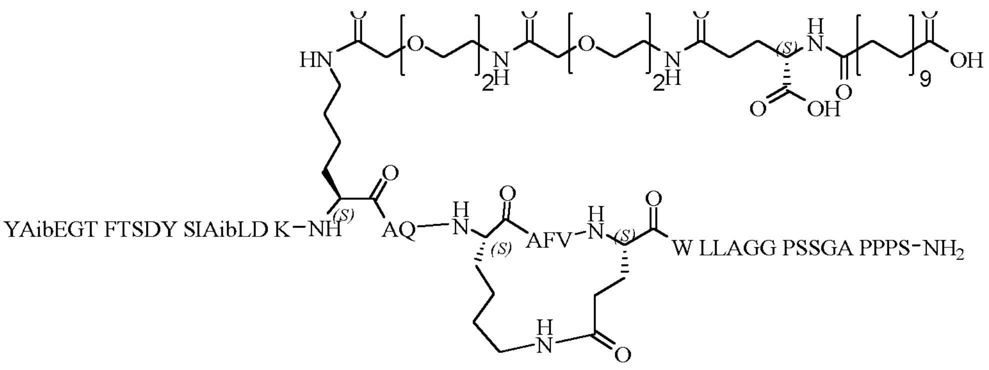

**WX-007**

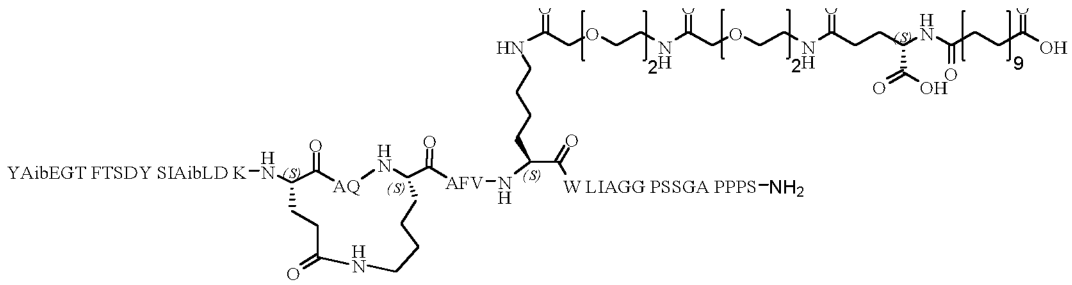

**WX-008**

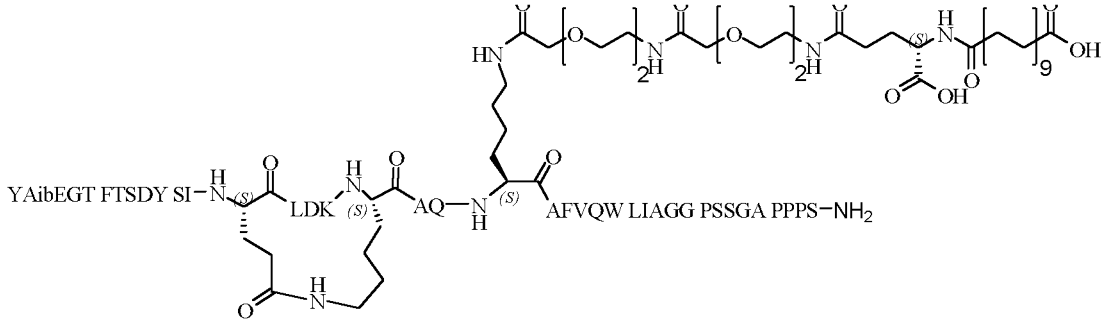

**WX-009**

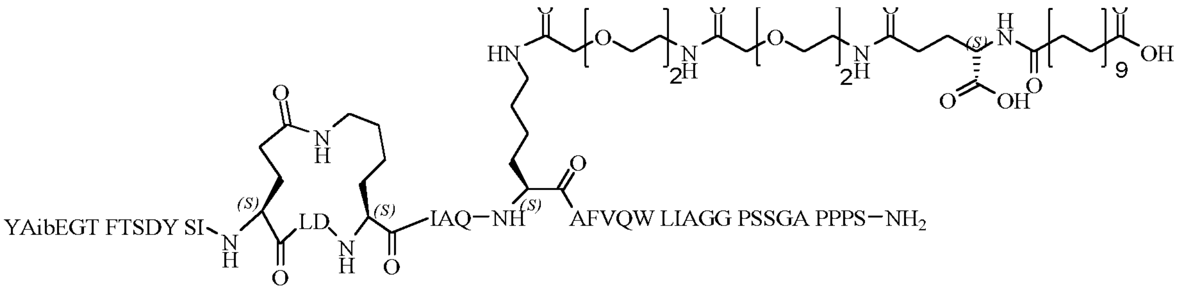

**WX-010**

The present disclosure also provides a compound represented by the following formula or a pharmaceutically acceptable salt thereof,

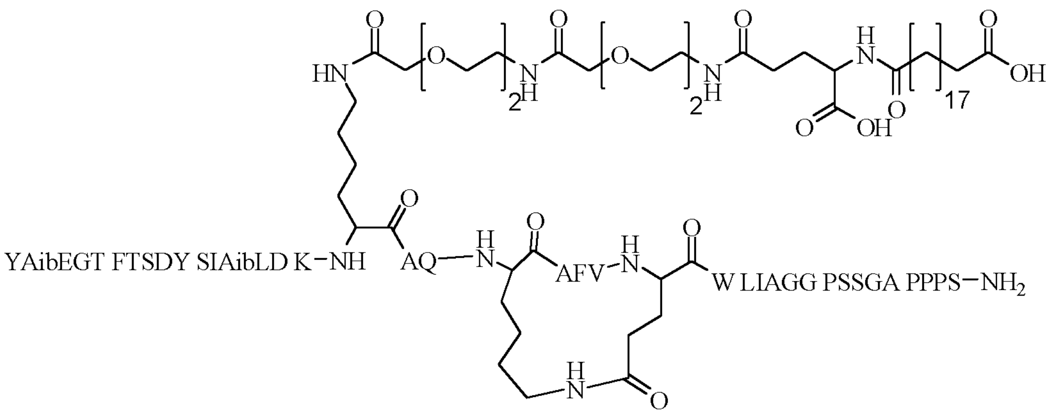

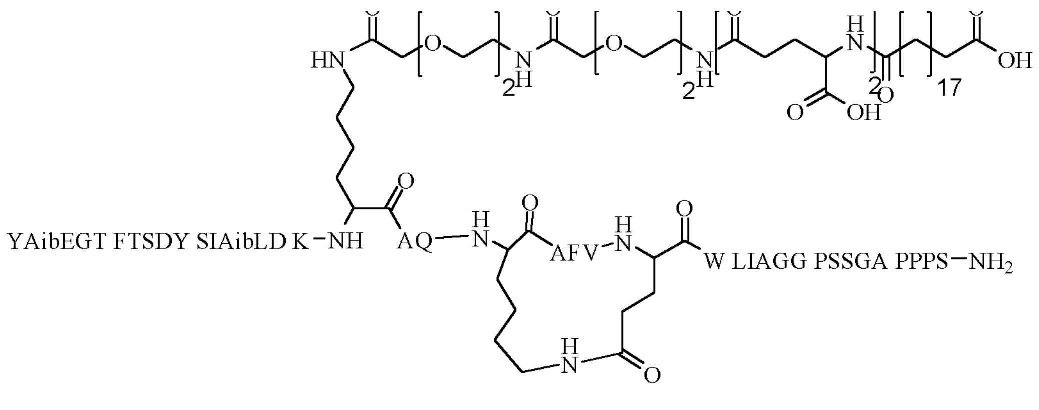

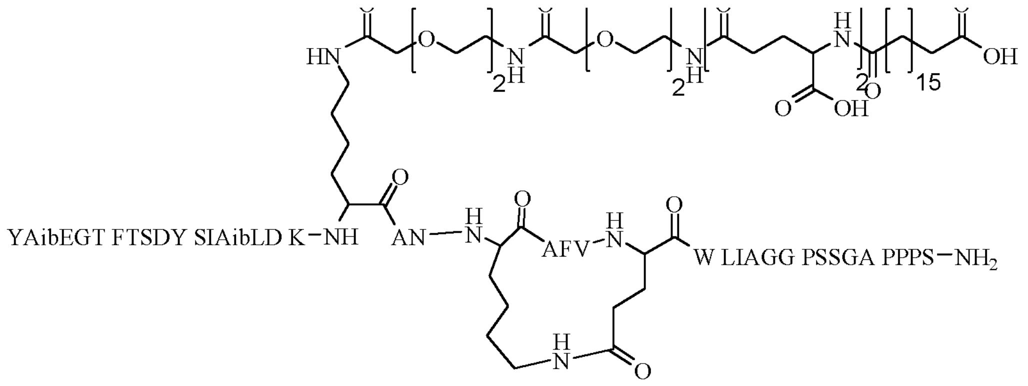

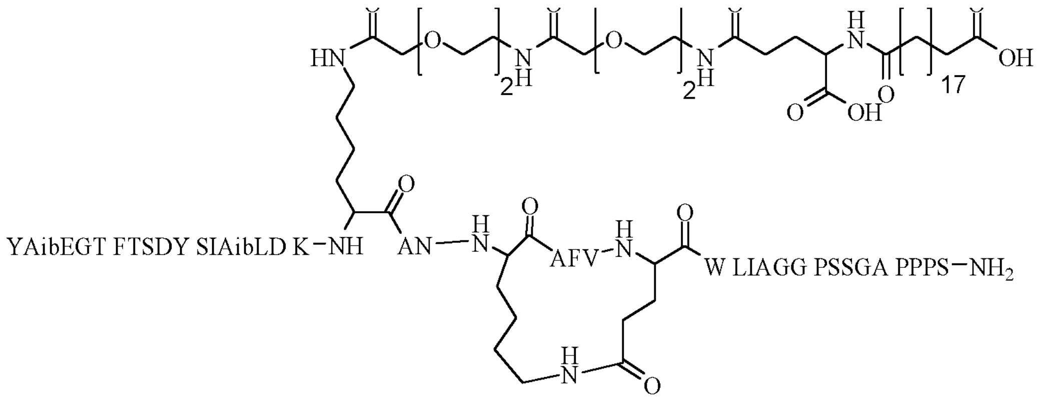

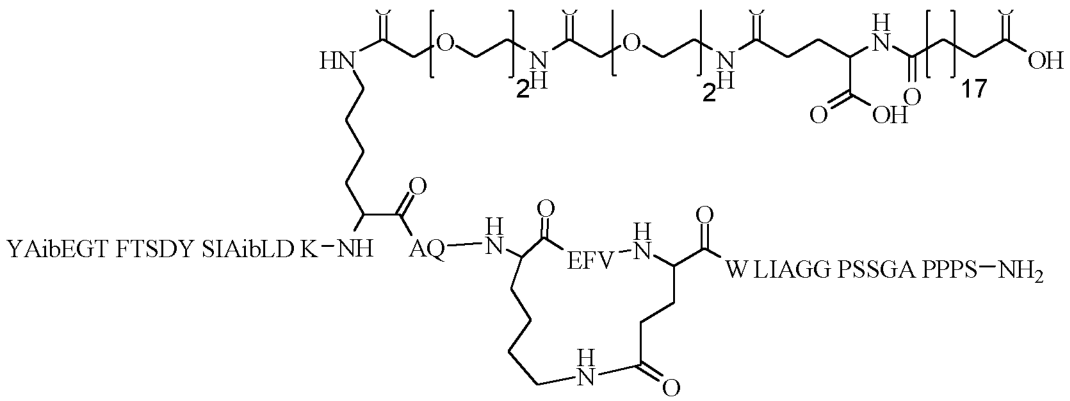

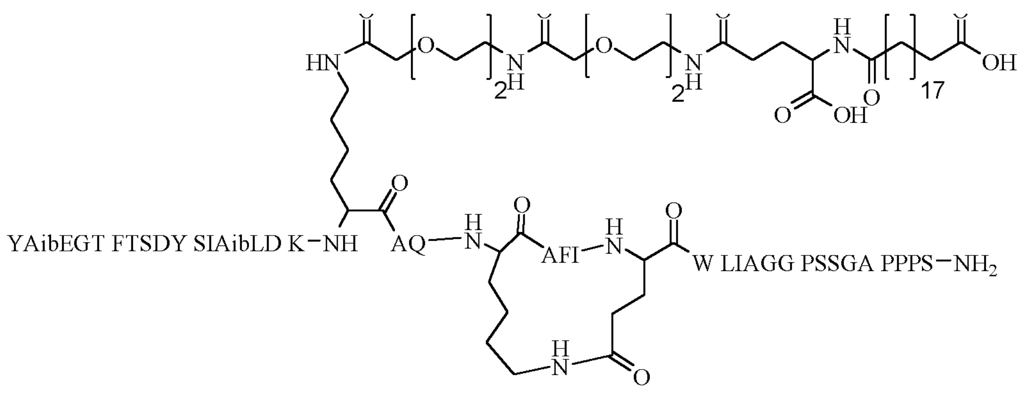

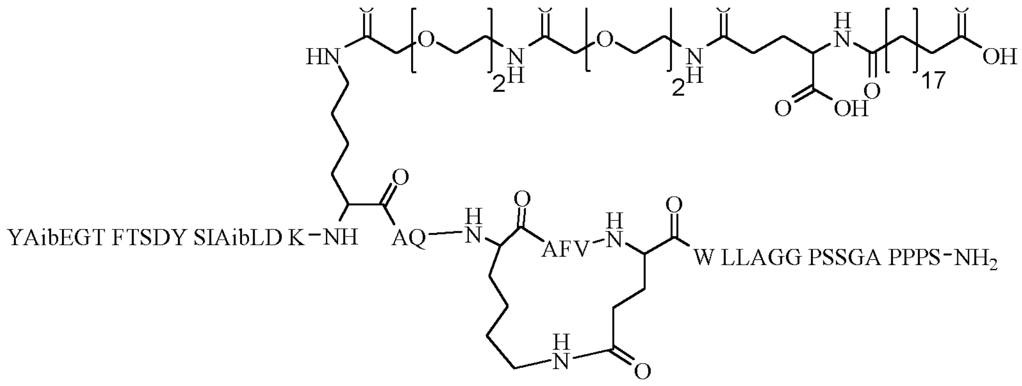

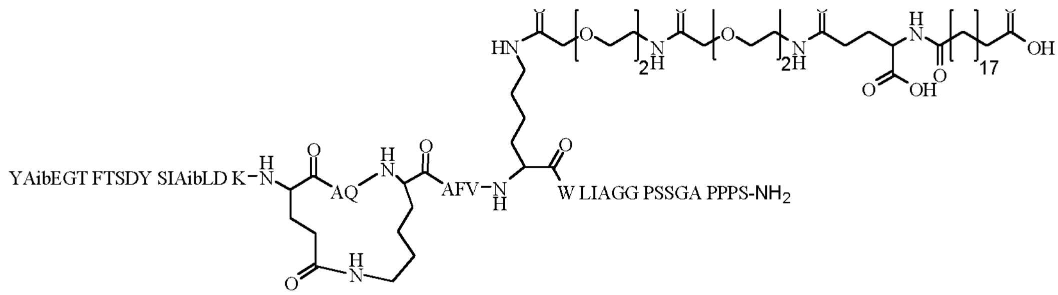

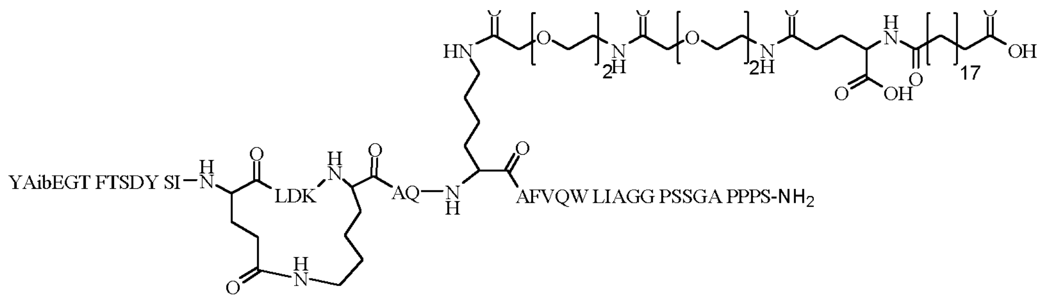

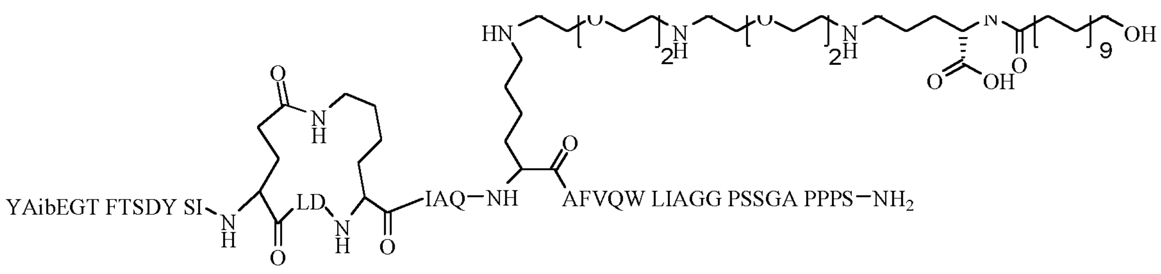

In some embodiments of the present disclosure, provided is a use of the compound or the pharmaceutically acceptable salt thereof in the manufacture of a medicament for treating obesity and diabetes.

The present disclosure also provides the following test method:

**Experimental Example: Inhibition Test of hERG Potassium Channels**

**A.** Main Material

CHO-hERG cell line (Chinese hamster ovary cells stably expressing hERG channels), constructed in-house at Shanghai Institute of Materia Medica, Chinese Academy of Sciences; patch clamp amplifier Axopatch 200B, TASER® International.

**B. Method**

a) Cell Culture

CHO cells stably expressing hERG are cultured in cell culture dishes with a diameter of 35 mm, placed in an incubator with 5% $CO_2$ at 37°C, and passaged at a ratio of 1:5 every 48 hours. Culture medium formulation: 90% F12 (Invitrogen), 10% fetal bovine serum (Gibco), 100 μg/mL G418 (Invitrogen) and 100 μg/mL Hygromycin B (Invitrogen). On the day of the test, the cell culture medium was aspirated, rinsed once with extracellular fluid, and then 0.25% Trypsin-EDTA (Invitrogen) solution was added, followed by digestion at room temperature for 3 to 5 minutes. After the digestion solution was aspirated, the cells were resuspended with extracellular fluid, and transferred to a laboratory dish for electrophysiological recording for further use.

b) Preparation of Intracellular and Extracellular Fluid

Extracellular fluid needs to be prepared once a month. Intracellular fluid needs to be subpackaged and frozen at -20°C.

Extracellular fluid (mM): 145 NaCl, 4 KCl, 2 $CaCl_2$, 1 $MgCl_2$, 10 Glucose and 10 HEPES, adjusted to pH = 7.4 with NaOH, with an osmotic pressure of 295 mOsm.

Intracellular fluid (mM): 120 KCl, 31.25 KOH, 5.374 $CaCl_2$, 1.75 $MgCl_2$, 4 $Na_2ATP$, 10 HEPES and 10 EGTA, adjusted to pH = 7.2 with KOH, with an osmotic pressure of 285 mOsm.

c) Preparation of Compounds

The compound is dissolved in DMSO into a 20 mM stock solution. On the day of the test, the compound stock solution is serially 3-fold diluted with DMSO, i.e., 10 μL of the compound stock solution is added to 20 μL of DMSO to obtain 6 intermediate concentrations of the compound serially diluted with DMSO, respectively. The intermediate concentrations are 20, 6.66, 2.22, 0.74, 0.24 and 0.082 mM, respectively. Then 10 μL of the intermediate concentration of the compound is taken and added to 4990 μL of extracellular fluid, and 500-fold diluted to obtain the final concentration to be tested. The highest test concentration is 40 μM, which are 40, 13.3, 4.44, 1.48, 0.49 and 0.16 μM, respectively. The content of DMSO in the final concentration to be tested does not exceed 0.2%, and this concentration of DMSO has no effect on hERG potassium channels.

d) Electrophysiological Recording Process

In CHO (Chinese Hamster Ovary) cells stably expressing hERG potassium channels, hERG potassium channel current is recorded by whole cell patch clamp technique at room temperature. The glass microelectrode is made of a glass electrode filament (BF150-86-10, Sutter) pulled by a glass microelectrode puller. The tip resistance after pouring the electrode internal solution into the electrode is about 2-5 MΩ. The glass microelectrode is inserted into the amplifier probe to connect to an Axopatch 200B (Molecular Devices) patch clamp amplifier. The clamping potential and data record are controlled and recorded by the computer with pClamp 10 software, and the sampling frequency is 10 kHz, and the wave filtering frequency is 2 kHz. After whole-cell recordings are obtained, the cells are clamped at -80 mV, and the step voltage of hERG potassium current (I hERG) is evoked from -80 mV to +20 mV with a depolarizing voltage for 2 s, then repolarized to -50 mV, and returned to - 80 mV after 1 s. This voltage stimulation is given every 10 s, and the administration process is started after confirming that the hERG potassium current is stable (1 min). Serial administration is started from a low test concentration of the compounds, with each test concentration administered for at least 1 minute. At least 3 cells ($n \geq 3$) are tested for each concentration of the compounds, and at least 2 cells ($n \geq 2$) are tested for each concentration of the positive compounds.

e) Data Analysis

In each complete current recording, the inhibition percentage for each compound effect concentration can be calculated based on the percentage of peak current in the negative control. The dose-effect relationship curve is obtained by fitting the standard Hill equation, and the specific equation is as follows:

$$I_{(C)} = I_b + (I_{fr} - I_b) * c^n / (IC_{50}^n + c^n)$$

c is the compound test concentration, n is the slope

Both curve fitting and inhibition rate calculation are analyzed and completed by Qpatch analysis software. If the inhibition rate at the lowest concentration exceeds the half inhibition rate or the inhibition rate at the highest concentration does not reach the half inhibition rate, the corresponding $IC_{50}$ of the compound is lower than the lowest concentration or the $IC_{50}$ value is greater than the highest concentration.

**C. Test Results and Conclusions**

The compounds in the present disclosure have no hERG-related risks.

**Experimental Example: Inhibition for Cytochrome P450 Isoenzyme**

**A. Experimental Purpose**

To determine the inhibitory effect of the test compounds on the activity of human liver microsomal cytochrome P450 isozymes (CYP1A2, CYP2C9, CYP2C19, CYP2D6 and CYP3A4).

**B. Experimental Operation**

First, the test compound (10 mM) is gradient diluted to prepare a working solution (100×final concentration). Concentrations of the working solution are 5 mM, 1.5 mM, 0.5 mM, 0.15 mM, 0.05 mM, 0.015 mM and 0.005 mM, respectively. At the same time, positive inhibitors of the P450 isozymes (CYP1A2, CYP2C9, CYP2C19, CYP2D6 and CYP3A4) and the working solution of the specific substrate mixture thereof are prepared; human liver microsomes frozen in a -80°C refrigerator are thawed on ice. Once the human liver microsomes are completely dissolved, the solution is diluted with PBS (phosphate buffer solution) to prepare a working solution with a certain concentration (0.253mg/mL); 20 μL of substrate mixture solution is added to a reaction plate (20 μL of PB is added to the Blank well). At the same time, 158 μL of human liver microsome working solution is added to the reaction plate, and the reaction plate is placed on ice for use; at this time, 2 μL of the test compound (N=1) and positive inhibitors (N=2) of each concentration are added to the corresponding well, and the group without inhibitors (the test compound or positive inhibitor) is added with the corresponding organic solvent, as the sample of the control group; after pre-incubating in a 37°C water bath for 10 minutes, 20 μL of NADPH solution is added to the reaction plate, and incubated in a 37°C water bath for 10 minutes; 400 μL of cold acetonitrile solution is added to terminate the reaction; the reaction plate is placed on a shaker and shaken for 10 minutes; the mixture is centrifuged at 4,000 rpm for 20 minutes; 200 μL of the supernatant is taken and added to 100 μL of water to dilute the sample; finally the reaction plate is sealed and shaken up for LC/MS/MS detection.

**C. Experimental Results and Conclusions**

The compounds in the present disclosure have no CYP inhibition-related risks.

**Technical Effect**

The compounds in the present disclosure have strong agonist activity on GLP-1R/GIPR; the compounds in the present disclosure have excellent pharmacokinetic properties; the compounds in the present disclosure have excellent plasma stability; the compounds in the present disclosure have very high plasma-protein binding; the compounds in the present disclosure have excellent *in vivo* efficacy.

**Definition and Description**

Unless otherwise specified, the following terms and phrases used herein have the following meanings. A specific term or phrase should not be considered indefinite or unclear in the absence of a particular definition, but should be understood according to the common meaning. When a trade name appears herein, it is intended to refer to its corresponding commercial product or active ingredient thereof.

The term "pharmaceutically acceptable" is used herein in terms of those compounds, materials, compositions, and/or dosage forms, which are suitable for use in contact with human and animal tissues within the scope of reliable medical judgment, without excessive toxicity, irritation, anaphylactic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic

acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by contacting the compound with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

As used herein, A or Ala represents alanine, and has a structure of

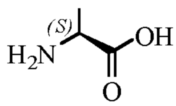

;

R or Arg represents arginine, and has a structure of

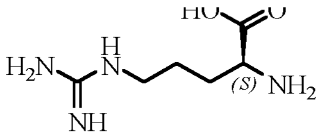

;

N or Asn represents asparagine, and has a structure of

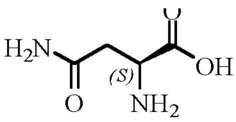

;

D or Asp represents aspartic acid, and has a structure of

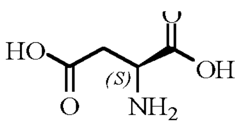

;

C or Cys represents cysteine, and has a structure of

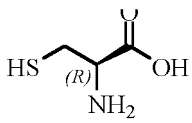

;

Q or Gln represents glutamine, and has a structure of

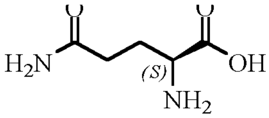
;

E or Glu represents glutamic acid, and has a structure of

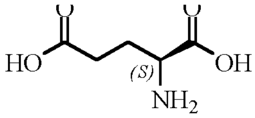
;

G or Gly represents glycine, and has a structure of

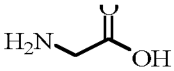
;

H or His represents histidine, and has a structure of

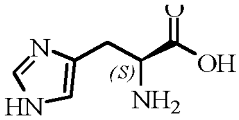
;

I or Ile represents isoleucine, and has a structure of

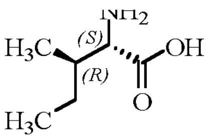
;

L or Leu represents leucine, and has a structure of

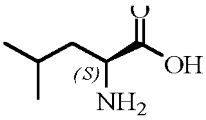
;

K or Lys represents lysine, and has a structure of

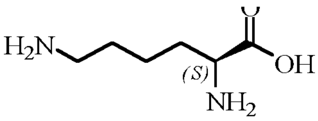
;

M or Met represents methionine, and has a structure of

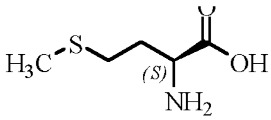

;

F or Phe represents phenylalanine, and has a structure of

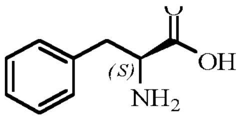

;

P or Pro represents proline, and has a structure of

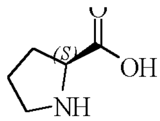

;

S or Ser represents serine, and has a structure of

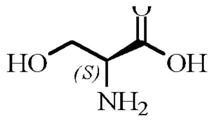

;

T or Thr represents threonine, and has a structure of

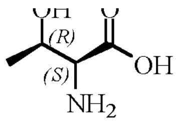

;

W or Trp represents tryptophan, and has a structure of

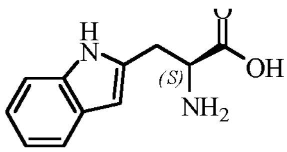

;

Y or Tyr represents tyrosine, and has a structure of

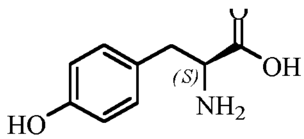

;

V or Val represents valine, and has a structure of

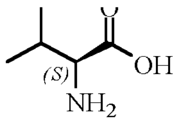
.

The term "treating" includes inhibiting, slowing, stopping or reversing the progression or severity of an existing symptom or disease.

Unless otherwise specified, the term "isomer" is intended to include a geometric isomers, a *cis-trans* isomer, a stereoisomer, an enantiomer, an optical isomer, diastereoisomers and a tautomeric isomer.

The compounds of the present disclosure may exist in specific geometric or stereoisomeric forms. The present disclosure contemplates all such compounds, including *cis* and *trans* isomers, (-)-and (+)-enantiomers, (*R*)-and (*S*)-enantiomers, diastereoisomers, (*D*)-isomers, (*L*)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term "*cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and or "(±)" refers to racemic.

Unless otherwise specified, the absolute configuration of a stereogenic center is represented by a wedged solid bond (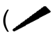) and a wedged dashed bond (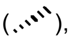), and the relative configuration of a stereogenic center is represented by a straight solid bond (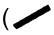) and a straight dashed bond (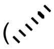), a wave line (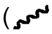) is used to represent a wedged solid bond (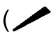) or a wedged dashed bond (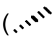), or the wave line (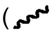) is used to represent a straight solid bond (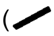) and a straight dashed bond (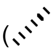).

Unless otherwise specified, the terms "enriched in one isomer", "enriched in isomers", "enriched in one enantiomer" or "enriched in enantiomers" refer to the content of one of the isomers or enantiomers is less than 100%, and the content of such isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, as well as *D* and *L* isomers, can be prepared by chiral synthesis or chiral reagents or other conventional techniques. If one enantiomer of a compound of the present disclosure is desired, the pure desired enantiomer can be prepared by asymmetric synthesis or derivatization of chiral auxiliary followed by separating the resulting diastereomeric mixture and cleaving the auxiliary group. Alternatively, when the molecule contains a basic functional group (such as an amino) or an acidic functional group (such as a carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more atom(s) that constitute the compound. For example, the compound can be labeled with a radioactive isotope, such as tritium ($^{3}H$), iodine-125 ($^{125}I$) or C-14 ($^{14}C$). For another example, deuterated drugs can be formed by replacing hydrogen with heavy hydrogen, the bond formed by deuterium and carbon is stronger than that by ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

Unless otherwise specified, the term "$C_{1-3}$ alkyl" refers to a linear or branched saturated hydrocarbon group

consisting of 1 to 3 carbon atoms. The $C_{1-3}$ alkyl includes $C_{1-2}$ and $C_{2-3}$ alkyl, etc.; it can be monovalent (such as methyl), divalent (such as methylene) or multivalent (such as methine). Examples of $C_{1-3}$ alkyl include but are not limited to methyl (Me), ethyl (Et), propyl (including n-propyl and isopropyl), etc.

Unless otherwise specified, "S-$NH_2$" in the compounds of the present disclosure is used to indicate the replacement of the carboxyl on serine with an amide.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: φ/ω scan, and after collecting the relevant data, the crystal structure can be further analyzed by direct method (Shelxs97).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the examples of the present disclosure.

The solvents used in the present disclosure are commercially available.

The present diclosure uses the following abbreviations: "aq" represents water; "eq" represents equivalent; "DCM" represents dichloromethane; "PE" represents petroleum ether; "DMSO" represents dimethyl sulfoxide; "MeOH" represents methanol; "BOC" represents *tert*-butoxycarbonyl, which is an amine protecting group; "r.t." represents room temperature; "O/N" represents overnight; "THF" represents tetrahydrofuran; "$Boc_2O$" represents di-*tert*-butyl dicarbonate; "TFA" represents trifluoroacetic acid; "DIEA" represents diisopropylethylamine; "DMF" represents *N,N*-dimethylformamide; "HBTU" represents O-(benzotriazol-1-yl)-*N,N,N',N'*-tetramethyluronium hexafluorophosphate; "HOBT" represents 1-hydroxybenzotriazole; "HOAT" represents 1-hydroxy-7-azabenzotriazole; "DIC" represents *N,N'*-diisopropylcarbodiimide; "DBU" represents 1,8-diazabicyclo[5.4.0]undec-7-ene; "PhSiH" represents phenylsilane; "$Pd(PPh_3)_4$" represents tetrakis(triphenylphosphine)palladium.

DETAILED DESCRIPTION OF THE PREFERRED EMBODIMENT

The present disclosure is described in detail by the examples below, but it does not mean that there are any adverse restrictions on the present disclosure. The present disclosure has been described in detail herein, and specific embodiments thereof have also been disclosed; for those skilled in the art, it is obvious to make various modifications and improvements to the embodiments of the present disclosure without departing from the spirit and scope of the present disclosure.

## Intermediate B-1

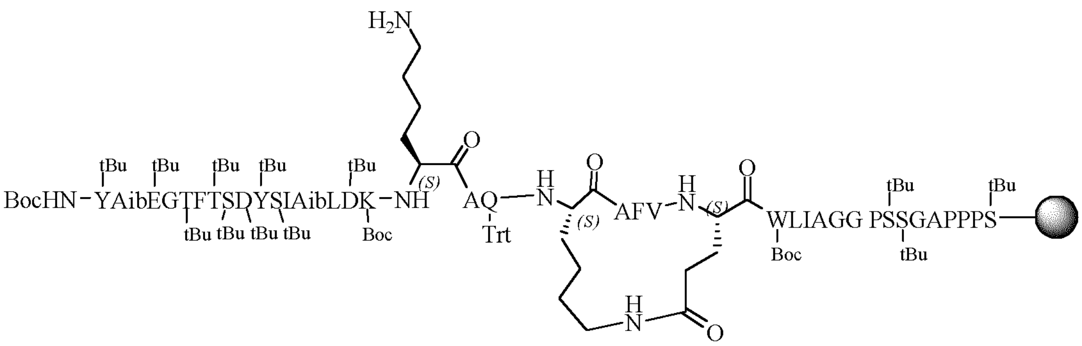

B-1

**Step 1:** 1.1 g of 4-(2',4'-dimethoxyphenyl-Fmoc-aminmethyl)-phenoxyacetamido-methylbenzhydryl amine resin (loading value, i.e., Sub =0.28 mmol/g) was weighed and added into a reaction column, and then DMF (50 mL) was added to the reaction column, bubbled with nitrogen for 2 hours. The waste was drained until no liquid flowed out, and the resin was washed with DMF (50 mL) 5 times (1 minute each time), and the waste was drained until no liquid flowed out.

**Step 2:** 20% of piperidine/DMF (50 mL) was added to the reaction column, bubbled with nitrogen for 20 minutes, and then the waste was drained until no liquid flowed out. The resin was washed with DMF (50 mL) 5 times (1 minute

each time), and the waste was drained until no liquid flowed out. The resin was tested with ninhydrin and showed blue.

**Step 3: Coupling of Amino Acids**

**3.1 Coupling of Fmoc-Ser(tBu)-OH**

1. Fmoc-Ser(tBu)-OH (3.0 eq) was weighed and added to the above resin, and DIEA (6.00 eq) plus 10 mL of DMF were added to the reaction column, bubbled with nitrogen, and HBTU (2.85 eq) was added after the amino acid was dissolved. The nitrogen was adjusted to make the resin bulge evenly.
2. The reaction was carried out for 0.5 hours at 25°C. The resin was tested with ninhydrin and then showed colorless and transparent.
3. The reaction solution was removed, and the resin was washed with DMF 5 times (50 mL each time, 1 min each time). The waste was drained until no liquid flowed out.

**3.2 Coupling of Fmoc-Pro-OH**

1. 20% of piperidine/DMF (50 mL) was added to the reaction column, bubbled with nitrogen for 20 minutes, and then the waste was drained until no liquid flowed out. The resin was washed with DMF (50 mL) 5 times (1 minute each time), and the waste was drained until no liquid flowed out. The resin was tested with ninhydrin and showed blue.
2. Fmoc-Pro-OH (3.0 eq) was weighed and added to the above resin, and DIEA (6.00 eq) plus 10 mL of DMF were added to the reaction column, bubbled with nitrogen, and HBTU (2.85 eq) was added after the amino acid was dissolved. The nitrogen was adjusted to make the resin bulge evenly.
3. The reaction was carried out for 0.5 hours at 25°C. The resin was tested with ninhydrin and then showed colorless and transparent.
4. The reaction solution was removed, and the resin was washed with DMF 5 times (50 mL each time, 1 min each time). The waste was drained until no liquid flowed out.

**Step 3.2 was repeated to complete the coupling of the following amino acids**

| NO. | Raw Material | Coupling Reagent |
|---|---|---|
| 3.3 | Fmoc-Pro-OH (3.00 eq) | HBTU (2.85 eq) and DIEA (6.00 eq) |
| 3.4 | Fmoc-Pro-OH (3.00 eq) | HBTU (2.85 eq) and DIEA (6.00 eq) |
| 3.5 | Fmoc-Ala-OH (3.00 eq) | HBTU (2.85 eq) and DIEA (6.00 eq) |
| 3.6 | Fmoc-Gly-OH (3.00 eq) | HBTU (2.85 eq) and DIEA (6.00 eq) |
| 3.7 | Fmoc-Ser(tBu)-OH (3.00 eq) | HBTU (2.85 eq) and DIEA (6.00 eq) |
| 3.8 | Fmoc-Ser(tBu)-OH (3.00 eq) | HBTU (2.85 eq) and DIEA (6.00 eq) |
| 3.9 | Fmoc-Pro-OH (3.00 eq) | HBTU (2.85 eq) and DIEA (6.00 eq) |
| 3.10 | Fmoc-Gly-OH (3.00 eq) | HBTU (2.85 eq) and DIEA (6.00 eq) |
| 3.11 | Fmoc-Gly-OH (3.00 eq) | HBTU (2.85 eq) and DIEA (6.00 eq) |
| 3.12 | Fmoc-Ala-OH (3.00 eq) | HBTU (2.85 eq) and DIEA (6.00 eq) |
| 3.13 | Fmoc-Ile-OH (3.00 eq) | HBTU (2.85 eq) and DIEA (6.00 eq) |
| 3.14 | Fmoc-Leu-OH (3.00 eq) | HBTU (2.85 eq) and DIEA (6.00 eq) |
| 3.15 | Fmoc-Trp(Boc)-OH (3.00 eq) | HBTU (2.85 eq) and DIEA (6.00 eq) |
| 3.16 | Fmoc-Glu(OAll)-OH (2.00 eq) | HBTU (1.90 eq) and DIEA (4.00 eq) |
| 3.17 | Fmoc-Val-OH (3.00 eq) | HBTU (2.85 eq) and DIEA (6.00 eq) |
| 3.18 | Fmoc-Phe-OH (3.00 eq) | HBTU (2.85 eq) and DIEA (6.00 eq) |
| 3.19 | Fmoc-Ala-OH (3.00 eq) | HBTU (2.85 eq) and DIEA (6.00 eq) |

(continued)

| NO. | Raw Material | Coupling Reagent |
|---|---|---|
| 3.20 | Fmoc-Lys(Alloc)-OH (2.00 eq) | HOBT (2.00 eq) and DIC (2.00 eq) |
| 3.21 | Fmoc-Gln(Trt)-OH (3.00 eq) | HBTU (2.85 eq) and DIEA (6.00 eq) |
| 3.22 | Fmoc-Ala-OH (3.00 eq) | HBTU (2.85 eq) and DIEA (6.00 eq) |
| 3.23 | Fmoc-Lys(Dde)-OH(3.00 eq) | HBTU (2.85 eq) and DIEA (6.00 eq) |
| 3.24 | Fmoc-Lys(Boc)-OH (3.00 eq) | HBTU (2.85 eq) and DIEA (6.00 eq) |
| 3.25 | Fmoc-Asp(tBu)-OH (6.00 eq) | HATU (5.70 eq) and DIEA (12.0 eq) |
| 3.26 | Fmoc-Leu-OH (6.00 eq) | HATU (5.70 eq) and DIEA (12.0 eq) |
| 3.27 | Fmoc-Aib-OH (6.00 eq) | HATU (5.70 eq) and DIEA (12.0 eq) |
| 3.28 | Fmoc-Ile-OH (6.00 eq) | HOAT (6.00 eq) and DIC (6.00 eq) |
| 3.29 | Fmoc-Ser(tBu)-OH (6.00 eq) | HATU (5.70 eq) and DIEA (12.0 eq) |
| 3.30 | Fmoc-Tyr(tBu)-OH (6.00 eq) | HATU (5.70 eq) and DIEA (12.0 eq) |
| 3.31 | Fmoc-Asp(tBu)-OH (6.00 eq) | HATU (5.70 eq) and DIEA (12.0 eq) |
| 3.32 | Fmoc-Ser(tBu)-OH (6.00 eq) | HATU (5.70 eq) and DIEA (12.0 eq) |
| 3.33 | Fmoc-Thr(tBu)-OH (6.00 eq) | HATU (5.70 eq) and DIEA (12.0 eq) |
| 3.34 | Fmoc-Phe-OH (6.00 eq) | HATU (5.70 eq) and DIEA (12.0 eq) |
| 3.35 | Fmoc-Thr(tBu)-OH (6.00 eq) | HATU (5.70 eq) and DIEA (12.0 eq) |
| 3.36 | Fmoc-Gly-OH (6.00 eq) | HATU (5.70 eq) and DIEA (12.0 eq) |
| 3.37 | Fmoc-Glu(tBu)-OH (6.00 eq) | HOBT (6.00 eq) and DIC (6.00 eq) |
| 3.38 | Fmoc-Aib-OH (3.00 eq) | HATU (2.85 eq) and DIEA (6.00 eq) |
| 3.39 | Boc-Tyr(tBu)-OH (3.00 eq) | HATU (2.85 eq) and DIEA (6.00 eq) |

**3.40 Deprotection of Alloc and OAll**

1. $PhSiH_3$ (20.0 eq) and DCM (10 mL) were added to the reaction column, after bubbling with nitrogen, $Pd(PPh_3)_4$ (0.2 eq) was added, and bubbled with nitrogen for 20 min. The reaction was carried out twice, and the waste was drained until no liquid flowed out.
2. The resin was washed with DMF 5 times (50 mL each time, 1 min each time). The waste was drained until no liquid flowed out.

**3.41 Ring Closure of Amide**

1. DIEA (3.00 eq) plus 10 mL of DMF were added to the reaction column, bubbled with nitrogen, and HATU (1.5 eq) was added after the amino acid was dissolved. The nitrogen was adjusted to make the resin bulge evenly.
2. The reaction was carried out for 0.5 hours at 25°C. The resin was tested with ninhydrin and then showed colorless and transparent.
3. The resin was washed with DMF 5 times (50 mL each time, 1 min each time). The waste was drained until no liquid flowed out.

**3.42 Deprotection of Dde**

1. 3% of hydrazine hydrate/DMF (50 mL) was added to the reaction column, bubbled with nitrogen for 15

minutes, and then the waste was drained until no liquid flowed out. The resin was washed with DMF 5 times (50 mL each time, 1 min each time) and the waste was drained until no liquid flowed out to obtain intermediate **B-1**. The resin was tested with ninhydrin and showed blue.

**Intermediate B-2**

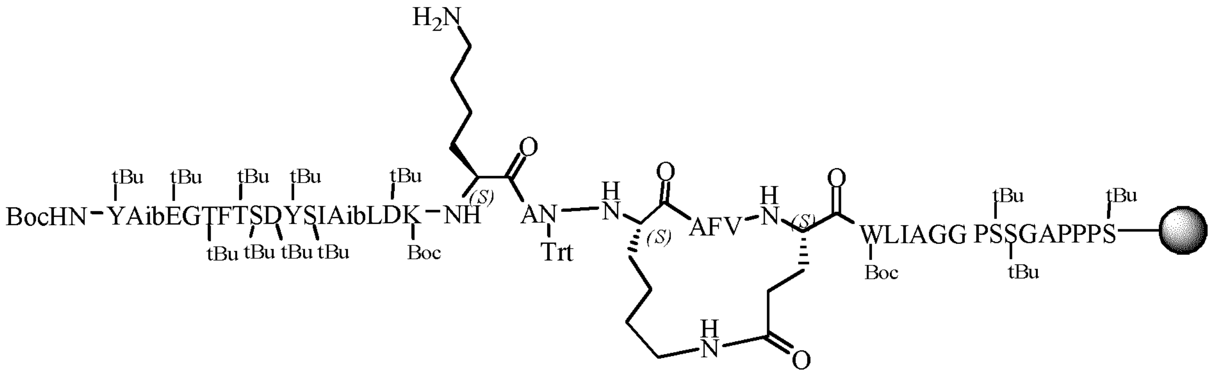

**B-2**

Referring to the synthesis method of **B-1**, **B-2** was obtained after the following operations such as coupling of amino acids, deprotection of Alloc and OAll, ring closure of amide, and deprotection of Dde.

| NO. | Raw Material | Coupling Reagent |
|---|---|---|
| 1 | Fmoc-Ser(tBu)-OH (3.00 eq) | HBTU (2.85 eq) and DIEA (6.00 eq) |
| 2 | Fmoc-Pro-OH (3.00 eq) | HBTU (2.85 eq) and DIEA (6.00 eq) |
| 3 | Fmoc-Pro-OH (3.00 eq) | HBTU (2.85 eq) and DIEA (6.00 eq) |
| 4 | Fmoc-Pro-OH (3.00 eq) | HBTU (2.85 eq) and DIEA (6.00 eq) |
| 5 | Fmoc-Ala-OH (3.00 eq) | HBTU (2.85 eq) and DIEA (6.00 eq) |
| 6 | Fmoc-Gly-OH (3.00 eq) | HBTU (2.85 eq) and DIEA (6.00 eq) |
| 7 | Fmoc-Ser(tBu)-OH (3.00 eq) | HBTU (2.85 eq) and DIEA (6.00 eq) |
| 8 | Fmoc-Ser(tBu)-OH (3.00 eq) | HBTU (2.85 eq) and DIEA (6.00 eq) |
| 9 | Fmoc-Pro-OH (3.00 eq) | HBTU (2.85 eq) and DIEA (6.00 eq) |
| 10 | Fmoc-Gly-OH (3.00 eq) | HBTU (2.85 eq) and DIEA (6.00 eq) |
| 11 | Fmoc-Gly-OH (3.00 eq) | HBTU (2.85 eq) and DIEA (6.00 eq) |
| 12 | Fmoc-Ala-OH (3.00 eq) | HBTU (2.85 eq) and DIEA (6.00 eq) |
| 13 | Fmoc-Ile-OH (3.00 eq) | HBTU (2.85 eq) and DIEA (6.00 eq) |
| 14 | Fmoc-Leu-OH (3.00 eq) | HBTU (2.85 eq) and DIEA (6.00 eq) |
| 15 | Fmoc-Trp(Boc)-OH (3.00 eq) | HBTU (2.85 eq) and DIEA (6.00 eq) |
| 16 | Fmoc-Glu(OAll)-OH (2.00 eq) | HBTU (1.90 eq) and DIEA (4.00 eq) |
| 17 | Fmoc-Val-OH (3.00 eq) | HBTU (2.85 eq) and DIEA (6.00 eq) |
| 18 | Fmoc-Phe-OH (3.00 eq) | HBTU (2.85 eq) and DIEA (6.00 eq) |
| 19 | Fmoc-Ala-OH (3.00 eq) | HBTU (2.85 eq) and DIEA (6.00 eq) |
| 20 | Fmoc-Lys(Alloc)-OH (2.00 eq) | HOBT (2.00 eq) and DIC (2.00 eq) |
| 21 | Fmoc-Asn(Trt)-OH (3.00 eq) | HBTU (2.85 eq) and DIEA (6.00 eq) |

(continued)

| NO. | Raw Material | Coupling Reagent |
|---|---|---|
| 22 | Fmoc-Ala-OH (3.00 eq) | HBTU (2.85 eq) and DIEA (6.00 eq) |
| 23 | Fmoc-Lys(Dde)-OH(3.00 eq) | HBTU (2.85 eq) and DIEA (6.00 eq) |
| 24 | Fmoc-Lys(Boc)-OH (3.00 eq) | HBTU (2.85 eq) and DIEA (6.00 eq) |
| 25 | Fmoc-Asp(tBu)-OH (6.00 eq) | HATU (5.70 eq) and DIEA (12.0 eq) |
| 26 | Fmoc-Leu-OH (6.00 eq) | HATU (5.70 eq) and DIEA (12.0 eq) |
| 27 | Fmoc-Aib-OH (6.00 eq) | HATU (5.70 eq) and DIEA (12.0 eq) |
| 28 | Fmoc-Ile-OH (6.00 eq) | HOAT (6.00 eq) and DIC (6.00 eq) |
| 29 | Fmoc-Ser(tBu)-OH (6.00 eq) | HATU (5.70 eq) and DIEA (12.0 eq) |
| 30 | Fmoc-Tyr(tBu)-OH (6.00 eq) | HATU (5.70 eq) and DIEA (12.0 eq) |
| 31 | Fmoc-Asp(tBu)-OH (6.00 eq) | HATU (5.70 eq) and DIEA (12.0 eq) |
| 32 | Fmoc-Ser(tBu)-OH (6.00 eq) | HATU (5.70 eq) and DIEA (12.0 eq) |
| 33 | Fmoc-Thr(tBu)-OH (6.00 eq) | HATU (5.70 eq) and DIEA (12.0 eq) |
| 34 | Fmoc-Phe-OH (6.00 eq) | HATU (5.70 eq) and DIEA (12.0 eq) |
| 35 | Fmoc-Thr(tBu)-OH (6.00 eq) | HATU (5.70 eq) and DIEA (12.0 eq) |
| 36 | Fmoc-Gly-OH (6.00 eq) | HATU (5.70 eq) and DIEA (12.0 eq) |
| 37 | Fmoc-Glu(tBu)-OH (6.00 eq) | HOBT (6.00 eq) and DIC (6.00 eq) |
| 38 | Fmoc-Aib-OH (3.00 eq) | HATU (2.85 eq) and DIEA (6.00 eq) |
| 39 | Boc-Tyr(tBu)-OH (3.00 eq) | HATU (2.85 eq) and DIEA (6.00 eq) |

## Intermediate B-3

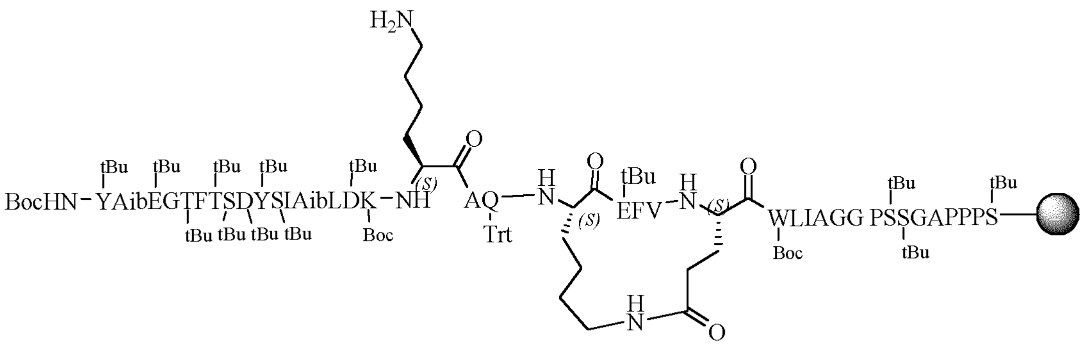

B-3

Referring to the synthesis method of **B-1**, **B-3** was obtained after the following operations such as coupling of amino acids, deprotection of Alloc and OAll, ring closure of amide, and deprotection of Dde.

| NO. | Raw Material | Coupling Reagent |
|---|---|---|
| 1 | Fmoc-Ser(tBu)-OH (3.00 eq) | HBTU (2.85 eq) and DIEA (6.00 eq) |
| 2 | Fmoc-Pro-OH (3.00 eq) | HBTU (2.85 eq) and DIEA (6.00 eq) |
| 3 | Fmoc-Pro-OH (3.00 eq) | HBTU (2.85 eq) and DIEA (6.00 eq) |
| 4 | Fmoc-Pro-OH (3.00 eq) | HBTU (2.85 eq) and DIEA (6.00 eq) |
| 5 | Fmoc-Ala-OH (3.00 eq) | HBTU (2.85 eq) and DIEA (6.00 eq) |

(continued)

| NO. | Raw Material | Coupling Reagent |
|---|---|---|
| 6 | Fmoc-Gly-OH (3.00 eq) | HBTU (2.85 eq) and DIEA (6.00 eq) |
| 7 | Fmoc-Ser(tBu)-OH (3.00 eq) | HBTU (2.85 eq) and DIEA (6.00 eq) |
| 8 | Fmoc-Ser(tBu)-OH (3.00 eq) | HBTU (2.85 eq) and DIEA (6.00 eq) |
| 9 | Fmoc-Pro-OH (3.00 eq) | HBTU (2.85 eq) and DIEA (6.00 eq) |
| 10 | Fmoc-Gly-OH (3.00 eq) | HBTU (2.85 eq) and DIEA (6.00 eq) |
| 11 | Fmoc-Gly-OH (3.00 eq) | HBTU (2.85 eq) and DIEA (6.00 eq) |
| 12 | Fmoc-Ala-OH (3.00 eq) | HBTU (2.85 eq) and DIEA (6.00 eq) |
| 13 | Fmoc-Ile-OH (3.00 eq) | HBTU (2.85 eq) and DIEA (6.00 eq) |
| 14 | Fmoc-Leu-OH (3.00 eq) | HBTU (2.85 eq) and DIEA (6.00 eq) |
| 15 | Fmoc-Trp(Boc)-OH (3.00 eq) | HBTU (2.85 eq) and DIEA (6.00 eq) |
| 16 | Fmoc-Glu(OAll)-OH (2.00 eq) | HBTU (1.90 eq) and DIEA (4.00 eq) |
| 17 | Fmoc-Val-OH (3.00 eq) | HBTU (2.85 eq) and DIEA (6.00 eq) |
| 18 | Fmoc-Phe-OH (3.00 eq) | HBTU (2.85 eq) and DIEA (6.00 eq) |
| 19 | Fmoc-Glu(tBu)-OH (3.00 eq) | HBTU (2.85 eq) and DIEA (6.00 eq) |
| 20 | Fmoc-Lys(Alloc)-OH (2.00 eq) | HOBT (2.00 eq) and DIC (2.00 eq) |
| 21 | Fmoc-Gln(Trt)-OH (3.00 eq) | HBTU (2.85 eq) and DIEA (6.00 eq) |
| 22 | Fmoc-Ala-OH (3.00 eq) | HBTU (2.85 eq) and DIEA (6.00 eq) |
| 23 | Fmoc-Lys(Dde)-OH(3.00 eq) | HBTU (2.85 eq) and DIEA (6.00 eq) |
| 24 | Fmoc-Lys(Boc)-OH (3.00 eq) | HBTU (2.85 eq) and DIEA (6.00 eq) |
| 25 | Fmoc-Asp(tBu)-OH (6.00 eq) | HATU (5.70 eq) and DIEA (12.0 eq) |
| 26 | Fmoc-Leu-OH (6.00 eq) | HATU (5.70 eq) and DIEA (12.0 eq) |
| 27 | Fmoc-Aib-OH (6.00 eq) | HATU (5.70 eq) and DIEA (12.0 eq) |
| 28 | Fmoc-Ile-OH (6.00 eq) | HOAT (6.00 eq) and DIC (6.00 eq) |
| 29 | Fmoc-Ser(tBu)-OH (6.00 eq) | HATU (5.70 eq) and DIEA (12.0 eq) |
| 30 | Fmoc-Tyr(tBu)-OH (6.00 eq) | HATU (5.70 eq) and DIEA (12.0 eq) |
| 31 | Fmoc-Asp(tBu)-OH (6.00 eq) | HATU (5.70 eq) and DIEA (12.0 eq) |
| 32 | Fmoc-Ser(tBu)-OH (6.00 eq) | HATU (5.70 eq) and DIEA (12.0 eq) |
| 33 | Fmoc-Thr(tBu)-OH (6.00 eq) | HATU (5.70 eq) and DIEA (12.0 eq) |
| 34 | Fmoc-Phe-OH (6.00 eq) | HATU (5.70 eq) and DIEA (12.0 eq) |
| 35 | Fmoc-Thr(tBu)-OH (6.00 eq) | HATU (5.70 eq) and DIEA (12.0 eq) |
| 36 | Fmoc-Gly-OH (6.00 eq) | HATU (5.70 eq) and DIEA (12.0 eq) |
| 37 | Fmoc-Glu(tBu)-OH (6.00 eq) | HOBT (6.00 eq) and DIC (6.00 eq) |
| 38 | Fmoc-Aib-OH (3.00 eq) | HATU (2.85 eq) and DIEA (6.00 eq) |
| 39 | Boc-Tyr(tBu)-OH (3.00 eq) | HATU (2.85 eq) and DIEA (6.00 eq) |

## Intermediate B-4

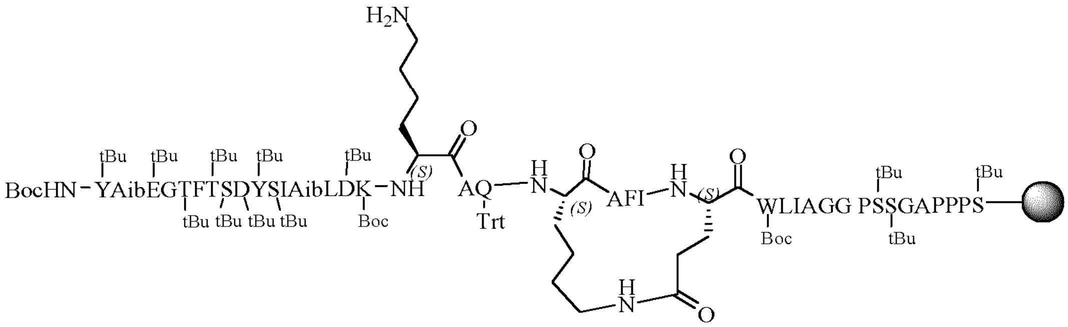

**B-4**

Referring to the synthesis method of **B-1**, **B-4** was obtained after the following operations such as coupling of amino acids, deprotection of Alloc and OAll, ring closure of amide, and deprotection of Dde.

| NO. | Raw Material | Coupling Reagent |
|---|---|---|
| 1 | Fmoc-Ser(tBu)-OH (3.00 eq) | HBTU (2.85 eq) and DIEA (6.00 eq) |
| 2 | Fmoc-Pro-OH (3.00 eq) | HBTU (2.85 eq) and DIEA (6.00 eq) |
| 3 | Fmoc-Pro-OH (3.00 eq) | HBTU (2.85 eq) and DIEA (6.00 eq) |
| 4 | Fmoc-Pro-OH (3.00 eq) | HBTU (2.85 eq) and DIEA (6.00 eq) |
| 5 | Fmoc-Ala-OH (3.00 eq) | HBTU (2.85 eq) and DIEA (6.00 eq) |
| 6 | Fmoc-Gly-OH (3.00 eq) | HBTU (2.85 eq) and DIEA (6.00 eq) |
| 7 | Fmoc-Ser(tBu)-OH (3.00 eq) | HBTU (2.85 eq) and DIEA (6.00 eq) |
| 8 | Fmoc-Ser(tBu)-OH (3.00 eq) | HBTU (2.85 eq) and DIEA (6.00 eq) |
| 9 | Fmoc-Pro-OH (3.00 eq) | HBTU (2.85 eq) and DIEA (6.00 eq) |
| 10 | Fmoc-Gly-OH (3.00 eq) | HBTU (2.85 eq) and DIEA (6.00 eq) |
| 11 | Fmoc-Gly-OH (3.00 eq) | HBTU (2.85 eq) and DIEA (6.00 eq) |
| 12 | Fmoc-Ala-OH (3.00 eq) | HBTU (2.85 eq) and DIEA (6.00 eq) |
| 13 | Fmoc-Ile-OH (3.00 eq) | HBTU (2.85 eq) and DIEA (6.00 eq) |
| 14 | Fmoc-Leu-OH (3.00 eq) | HBTU (2.85 eq) and DIEA (6.00 eq) |
| 15 | Fmoc-Trp(Boc)-OH (3.00 eq) | HBTU (2.85 eq) and DIEA (6.00 eq) |
| 16 | Fmoc-Glu(OAll)-OH (2.00 eq) | HBTU (1.90 eq) and DIEA (4.00 eq) |
| 17 | Fmoc-Ile-OH (3.00 eq) | HBTU (2.85 eq) and DIEA (6.00 eq) |
| 18 | Fmoc-Phe-OH (3.00 eq) | HBTU (2.85 eq) and DIEA (6.00 eq) |
| 19 | Fmoc-Ala-OH (3.00 eq) | HBTU (2.85 eq) and DIEA (6.00 eq) |
| 20 | Fmoc-Lys(Alloc)-OH (2.00 eq) | HOBT (2.00 eq) and DIC (2.00 eq) |
| 21 | Fmoc-Gln(Trt)-OH (3.00 eq) | HBTU (2.85 eq) and DIEA (6.00 eq) |
| 22 | Fmoc-Ala-OH (3.00 eq) | HBTU (2.85 eq) and DIEA (6.00 eq) |
| 23 | Fmoc-Lys(Dde)-OH(3.00 eq) | HBTU (2.85 eq) and DIEA (6.00 eq) |
| 24 | Fmoc-Lys(Boc)-OH (3.00 eq) | HBTU (2.85 eq) and DIEA (6.00 eq) |
| 25 | Fmoc-Asp(tBu)-OH (6.00 eq) | HATU (5.70 eq) and DIEA (12.0 eq) |
| 26 | Fmoc-Leu-OH (6.00 eq) | HATU (5.70 eq) and DIEA (12.0 eq) |
| 27 | Fmoc-Aib-OH (6.00 eq) | HATU (5.70 eq) and DIEA (12.0 eq) |
| 28 | Fmoc-Ile-OH (6.00 eq) | HOAT (6.00 eq) and DIC (6.00 eq) |

(continued)

| NO. | Raw Material | Coupling Reagent |
|---|---|---|
| 29 | Fmoc-Ser(tBu)-OH (6.00 eq) | HATU (5.70 eq) and DIEA (12.0 eq) |
| 30 | Fmoc-Tyr(tBu)-OH (6.00 eq) | HATU (5.70 eq) and DIEA (12.0 eq) |
| 31 | Fmoc-Asp(tBu)-OH (6.00 eq) | HATU (5.70 eq) and DIEA (12.0 eq) |
| 32 | Fmoc-Ser(tBu)-OH (6.00 eq) | HATU (5.70 eq) and DIEA (12.0 eq) |
| 33 | Fmoc-Thr(tBu)-OH (6.00 eq) | HATU (5.70 eq) and DIEA(12.0 eq) |
| 34 | Fmoc-Phe-OH (6.00 eq) | HATU (5.70 eq) and DIEA (12.0 eq) |
| 35 | Fmoc-Thr(tBu)-OH (6.00 eq) | HATU (5.70 eq) and DIEA (12.0 eq) |
| 36 | Fmoc-Gly-OH (6.00 eq) | HATU (5.70 eq) and DIEA (12.0 eq) |
| 37 | Fmoc-Glu(tBu)-OH (6.00 eq) | HOBT (6.00 eq) and DIC (6.00 eq) |
| 38 | Fmoc-Aib-OH (3.00 eq) | HATU (2.85 eq) and DIEA (6.00 eq) |
| 39 | Boc-Tyr(tBu)-OH (3.00 eq) | HATU (2.85 eq) and DIEA (6.00 eq) |

## Intermediate B-5

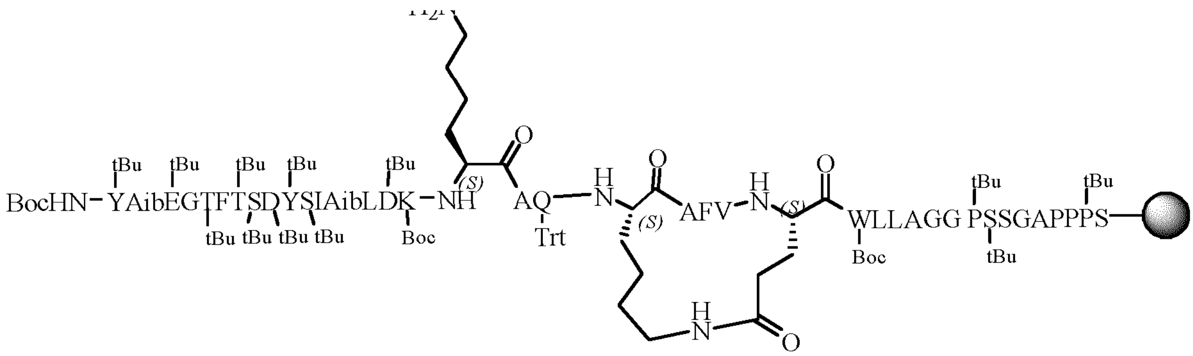

B-5

Referring to the synthesis method of **B-1**, **B-5** was obtained after the following operations such as coupling of amino acids, deprotection of Alloc and OAll, ring closure of amide, and deprotection of Dde.

| NO. | Raw Material | Coupling Reagent |
|---|---|---|
| 1 | Fmoc-Ser(tBu)-OH (3.00 eq) | HBTU (2.85 eq) and DIEA (6.00 eq) |
| 2 | Fmoc-Pro-OH (3.00 eq) | HBTU (2.85 eq) and DIEA (6.00 eq) |
| 3 | Fmoc-Pro-OH (3.00 eq) | HBTU (2.85 eq) and DIEA (6.00 eq) |
| 4 | Fmoc-Pro-OH (3.00 eq) | HBTU (2.85 eq) and DIEA (6.00 eq) |
| 5 | Fmoc-Ala-OH (3.00 eq) | HBTU (2.85 eq) and DIEA (6.00 eq) |
| 6 | Fmoc-Gly-OH (3.00 eq) | HBTU (2.85 eq) and DIEA (6.00 eq) |
| 7 | Fmoc-Ser(tBu)-OH (3.00 eq) | HBTU (2.85 eq) and DIEA (6.00 eq) |
| 8 | Fmoc-Ser(tBu)-OH (3.00 eq) | HBTU (2.85 eq) and DIEA (6.00 eq) |
| 9 | Fmoc-Pro-OH (3.00 eq) | HBTU (2.85 eq) and DIEA (6.00 eq) |
| 10 | Fmoc-Gly-OH (3.00 eq) | HBTU (2.85 eq) and DIEA (6.00 eq) |
| 11 | Fmoc-Gly-OH (3.00 eq) | HBTU (2.85 eq) and DIEA (6.00 eq) |

(continued)

| NO. | Raw Material | Coupling Reagent |
|---|---|---|
| 12 | Fmoc-Ala-OH (3.00 eq) | HBTU (2.85 eq) and DIEA (6.00 eq) |
| 13 | Fmoc-Leu-OH (3.00 eq) | HBTU (2.85 eq) and DIEA (6.00 eq) |
| 14 | Fmoc-Leu-OH (3.00 eq) | HBTU (2.85 eq) and DIEA (6.00 eq) |
| 15 | Fmoc-Trp(Boc)-OH (3.00 eq) | HBTU (2.85 eq) and DIEA (6.00 eq) |
| 16 | Fmoc-Glu(OAll)-OH (2.00 eq) | HBTU (1.90 eq) and DIEA (4.00 eq) |
| 17 | Fmoc-Val-OH (3.00 eq) | HBTU (2.85 eq) and DIEA (6.00 eq) |
| 18 | Fmoc-Phe-OH (3.00 eq) | HBTU (2.85 eq) and DIEA (6.00 eq) |
| 19 | Fmoc-Ala-OH (3.00 eq) | HBTU (2.85 eq) and DIEA (6.00 eq) |
| 20 | Fmoc-Lys(Alloc)-OH (2.00 eq) | HOBT (2.00 eq) and DIC (2.00 eq) |
| 21 | Fmoc-Gln(Trt)-OH (3.00 eq) | HBTU (2.85 eq) and DIEA (6.00 eq) |
| 22 | Fmoc-Ala-OH (3.00 eq) | HBTU (2.85 eq) and DIEA (6.00 eq) |
| 23 | Fmoc-Lys(Dde)-OH(3.00 eq) | HBTU (2.85 eq) and DIEA (6.00 eq) |
| 24 | Fmoc-Lys(Boc)-OH (3.00 eq) | HBTU (2.85 eq) and DIEA (6.00 eq) |
| 25 | Fmoc-Asp(tBu)-OH (6.00 eq) | HATU (5.70 eq) and DIEA (12.0 eq) |
| 26 | Fmoc-Leu-OH (6.00 eq) | HATU (5.70 eq) and DIEA (12.0 eq) |
| 27 | Fmoc-Aib-OH (6.00 eq) | HATU (5.70 eq) and DIEA (12.0 eq) |
| 28 | Fmoc-Ile-OH (6.00 eq) | HOAT (6.00 eq) and DIC (6.00 eq) |
| 29 | Fmoc-Ser(tBu)-OH (6.00 eq) | HATU (5.70 eq) and DIEA (12.0 eq) |
| 30 | Fmoc-Tyr(tBu)-OH (6.00 eq) | HATU (5.70 eq) and DIEA (12.0 eq) |
| 31 | Fmoc-Asp(tBu)-OH (6.00 eq) | HATU (5.70 eq) and DIEA (12.0 eq) |
| 32 | Fmoc-Ser(tBu)-OH (6.00 eq) | HATU (5.70 eq) and DIEA (12.0 eq) |
| 33 | Fmoc-Thr(tBu)-OH (6.00 eq) | HATU (5.70 eq) and DIEA (12.0 eq) |
| 34 | Fmoc-Phe-OH (6.00 eq) | HATU (5.70 eq) and DIEA (12.0 eq) |
| 35 | Fmoc-Thr(tBu)-OH (6.00 eq) | HATU (5.70 eq) and DIEA (12.0 eq) |
| 36 | Fmoc-Gly-OH (6.00 eq) | HATU (5.70 eq) and DIEA (12.0 eq) |
| 37 | Fmoc-Glu(tBu)-OH (6.00 eq) | HOBT (6.00 eq) and DIC (6.00 eq) |
| 38 | Fmoc-Aib-OH (3.00 eq) | HATU (2.85 eq) and DIEA (6.00 eq) |
| 39 | Boc-Tyr(tBu)-OH (3.00 eq) | HATU (2.85 eq) and DIEA (6.00 eq) |

## Intermediate B-6

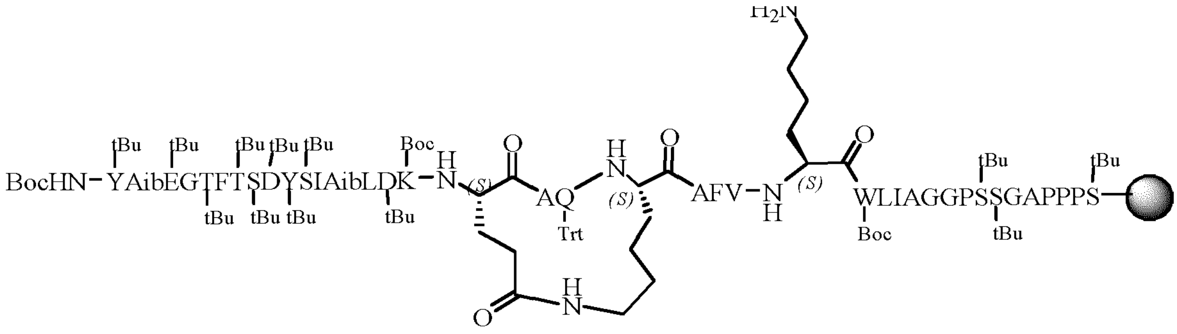

**B-6**

Referring to the synthesis method of **B-1**, **B-6** was obtained after the following operations such as coupling of amino acids, deprotection of Alloc and OAll, ring closure of amide, and deprotection of Dde.

| NO. | Raw Material | Coupling Reagent |
|---|---|---|
| 1 | Fmoc-Ser(tBu)-OH (3.00 eq) | HBTU (2.85 eq) and DIEA (6.00 eq) |
| 2 | Fmoc-Pro-OH (3.00 eq) | HBTU (2.85 eq) and DIEA (6.00 eq) |
| 3 | Fmoc-Pro-OH (3.00 eq) | HBTU (2.85 eq) and DIEA (6.00 eq) |
| 4 | Fmoc-Pro-OH (3.00 eq) | HBTU (2.85 eq) and DIEA (6.00 eq) |
| 5 | Fmoc-Ala-OH (3.00 eq) | HBTU (2.85 eq) and DIEA (6.00 eq) |
| 6 | Fmoc-Gly-OH (3.00 eq) | HBTU (2.85 eq) and DIEA (6.00 eq) |
| 7 | Fmoc-Ser(tBu)-OH (3.00 eq) | HBTU (2.85 eq) and DIEA (6.00 eq) |
| 8 | Fmoc-Ser(tBu)-OH (3.00 eq) | HBTU (2.85 eq) and DIEA (6.00 eq) |
| 9 | Fmoc-Pro-OH (3.00 eq) | HBTU (2.85 eq) and DIEA (6.00 eq) |
| 10 | Fmoc-Gly-OH (3.00 eq) | HBTU (2.85 eq) and DIEA (6.00 eq) |
| 11 | Fmoc-Gly-OH (3.00 eq) | HBTU (2.85 eq) and DIEA (6.00 eq) |
| 12 | Fmoc-Ala-OH (3.00 eq) | HBTU (2.85 eq) and DIEA (6.00 eq) |
| 13 | Fmoc-Ile-OH (3.00 eq) | HBTU (2.85 eq) and DIEA (6.00 eq) |
| 14 | Fmoc-Leu-OH (3.00 eq) | HBTU (2.85 eq) and DIEA (6.00 eq) |
| 15 | Fmoc-Trp(Boc)-OH (3.00 eq) | HBTU (2.85 eq) and DIEA (6.00 eq) |
| 16 | Fmoc-Lys(Dde)-OH(3.00 eq) | HBTU (1.90 eq) and DIEA (4.00 eq) |
| 17 | Fmoc-Val-OH (3.00 eq) | HBTU (2.85 eq) and DIEA (6.00 eq) |
| 18 | Fmoc-Phe-OH (3.00 eq) | HBTU (2.85 eq) and DIEA (6.00 eq) |
| 19 | Fmoc-Ala-OH (3.00 eq) | HBTU (2.85 eq) and DIEA (6.00 eq) |
| 20 | Fmoc-Lys(Alloc)-OH (2.00 eq) | HOBT (2.00 eq) and DIC (2.00 eq) |
| 21 | Fmoc-Gln(Trt)-OH (3.00 eq) | HBTU (2.85 eq) and DIEA (6.00 eq) |
| 22 | Fmoc-Ala-OH (3.00 eq) | HBTU (2.85 eq) and DIEA (6.00 eq) |
| 23 | Fmoc-Glu(OAll)-OH (2.00 eq) | HBTU (2.85 eq) and DIEA (6.00 eq) |
| 24 | Fmoc-Lys(Boc)-OH (3.00 eq) | HBTU (2.85 eq) and DIEA (6.00 eq) |
| 25 | Fmoc-Asp(tBu)-OH (6.00 eq) | HATU (5.70 eq) and DIEA (12.0 eq) |
| 26 | Fmoc-Leu-OH (6.00 eq) | HATU (5.70 eq) and DIEA (12.0 eq) |
| 27 | Fmoc-Aib-OH (6.00 eq) | HATU (5.70 eq) and DIEA (12.0 eq) |

(continued)

| NO. | Raw Material | Coupling Reagent |
|---|---|---|
| 28 | Fmoc-Ile-OH (6.00 eq) | HOAT (6.00 eq) and DIC (6.00 eq) |
| 29 | Fmoc-Ser(tBu)-OH (6.00 eq) | HATU (5.70 eq) and DIEA (12.0 eq) |
| 30 | Fmoc-Tyr(tBu)-OH (6.00 eq) | HATU (5.70 eq) and DIEA (12.0 eq) |
| 31 | Fmoc-Asp(tBu)-OH (6.00 eq) | HATU (5.70 eq) and DIEA (12.0 eq) |
| 32 | Fmoc-Ser(tBu)-OH (6.00 eq) | HATU (5.70 eq) and DIEA (12.0 eq) |
| 33 | Fmoc-Thr(tBu)-OH (6.00 eq) | HATU (5.70 eq) and DIEA (12.0 eq) |
| 34 | Fmoc-Phe-OH (6.00 eq) | HATU (5.70 eq) and DIEA (12.0 eq) |
| 35 | Fmoc-Thr(tBu)-OH (6.00 eq) | HATU (5.70 eq) and DIEA (12.0 eq) |
| 36 | Fmoc-Gly-OH (6.00 eq) | HATU (5.70 eq) and DIEA (12.0 eq) |
| 37 | Fmoc-Glu(tBu)-OH (6.00 eq) | HOBT (6.00 eq) and DIC (6.00 eq) |
| 38 | Fmoc-Aib-OH (3.00 eq) | HATU (2.85 eq) and DIEA (6.00 eq) |
| 39 | Boc-Tyr(tBu)-OH (3.00 eq) | HATU (2.85 eq) and DIEA (6.00 eq) |

## Intermediate B-7

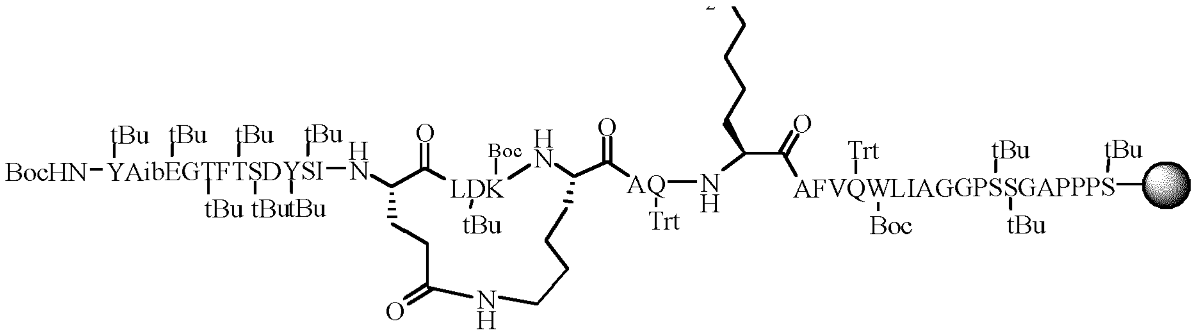

**B-7**

Referring to the synthesis method of **B-1**, **B-7** was obtained after the following operations such as coupling of amino acids, deprotection of Alloc and OAll, ring closure of amide, and deprotection of Dde.

| NO. | Raw Material | Coupling Reagent |
|---|---|---|
| 1 | Fmoc-Ser(tBu)-OH (3.00 eq) | HBTU (2.85 eq) and DIEA (6.00 eq) |
| 2 | Fmoc-Pro-OH (3.00 eq) | HBTU (2.85 eq) and DIEA (6.00 eq) |
| 3 | Fmoc-Pro-OH (3.00 eq) | HBTU (2.85 eq) and DIEA (6.00 eq) |
| 4 | Fmoc-Pro-OH (3.00 eq) | HBTU (2.85 eq) and DIEA (6.00 eq) |
| 5 | Fmoc-Ala-OH (3.00 eq) | HBTU (2.85 eq) and DIEA (6.00 eq) |
| 6 | Fmoc-Gly-OH (3.00 eq) | HBTU (2.85 eq) and DIEA (6.00 eq) |
| 7 | Fmoc-Ser(tBu)-OH (3.00 eq) | HBTU (2.85 eq) and DIEA (6.00 eq) |
| 8 | Fmoc-Ser(tBu)-OH (3.00 eq) | HBTU (2.85 eq) and DIEA (6.00 eq) |
| 9 | Fmoc-Pro-OH (3.00 eq) | HBTU (2.85 eq) and DIEA (6.00 eq) |
| 10 | Fmoc-Gly-OH (3.00 eq) | HBTU (2.85 eq) and DIEA (6.00 eq) |
| 11 | Fmoc-Gly-OH (3.00 eq) | HBTU (2.85 eq) and DIEA (6.00 eq) |

(continued)

| NO. | Raw Material | Coupling Reagent |
|---|---|---|
| 12 | Fmoc-Ala-OH (3.00 eq) | HBTU (2.85 eq) and DIEA (6.00 eq) |
| 13 | Fmoc-Ile-OH (3.00 eq) | HBTU (2.85 eq) and DIEA (6.00 eq) |
| 14 | Fmoc-Leu-OH (3.00 eq) | HBTU (2.85 eq) and DIEA (6.00 eq) |
| 15 | Fmoc-Trp(Boc)-OH (3.00 eq) | HBTU (2.85 eq) and DIEA (6.00 eq) |
| 16 | Fmoc-Gln(Trt)-OH (2.00 eq) | HBTU (1.90 eq) and DIEA (4.00 eq) |
| 17 | Fmoc-Val-OH (3.00 eq) | HBTU (2.85 eq) and DIEA (6.00 eq) |
| 18 | Fmoc-Phe-OH (3.00 eq) | HBTU (2.85 eq) and DIEA (6.00 eq) |
| 19 | Fmoc-Ala-OH (3.00 eq) | HBTU (2.85 eq) and DIEA (6.00 eq) |
| 20 | Fmoc-Lys(Dde)-OH(3.00 eq) | HOBT (2.00 eq) and DIC (2.00 eq) |
| 21 | Fmoc-Gln(Trt)-OH (3.00 eq) | HBTU (2.85 eq) and DIEA (6.00 eq) |
| 22 | Fmoc-Ala-OH (3.00 eq) | HBTU (2.85 eq) and DIEA (6.00 eq) |
| 23 | Fmoc-Lys(Alloc)-OH (2.00 eq) | HBTU (2.85 eq) and DIEA (6.00 eq) |
| 24 | Fmoc-Lys(Boc)-OH (3.00 eq) | HBTU (2.85 eq) and DIEA (6.00 eq) |
| 25 | Fmoc-Asp(tBu)-OH (6.00 eq) | HATU (5.70 eq) and DIEA (12.0 eq) |
| 26 | Fmoc-Leu-OH (6.00 eq) | HATU (5.70 eq) and DIEA (12.0 eq) |
| 27 | Fmoc-Glu(OAll)-OH (2.00 eq) | HATU (5.70 eq) and DIEA (12.0 eq) |
| 28 | Fmoc-Ile-OH (6.00 eq) | HOAT (6.00 eq) and DIC (6.00 eq) |
| 29 | Fmoc-Ser(tBu)-OH (6.00 eq) | HATU (5.70 eq) and DIEA (12.0 eq) |
| 30 | Fmoc-Tyr(tBu)-OH (6.00 eq) | HATU (5.70 eq) and DIEA (12.0 eq) |
| 31 | Fmoc-Asp(tBu)-OH (6.00 eq) | HATU (5.70 eq) and DIEA (12.0 eq) |
| 32 | Fmoc-Ser(tBu)-OH (6.00 eq) | HATU (5.70 eq) and DIEA (12.0 eq) |
| 33 | Fmoc-Thr(tBu)-OH (6.00 eq) | HATU (5.70 eq) and DIEA (12.0 eq) |
| 34 | Fmoc-Phe-OH (6.00 eq) | HATU (5.70 eq) and DIEA (12.0 eq) |
| 35 | Fmoc-Thr(tBu)-OH (6.00 eq) | HATU (5.70 eq) and DIEA (12.0 eq) |
| 36 | Fmoc-Gly-OH (6.00 eq) | HATU (5.70 eq) and DIEA (12.0 eq) |
| 37 | Fmoc-Glu(tBu)-OH (6.00 eq) | HOBT (6.00 eq) and DIC (6.00 eq) |
| 38 | Fmoc-Aib-OH (3.00 eq) | HATU (2.85 eq) and DIEA (6.00 eq) |
| 39 | Boc-Tyr(tBu)-OH (3.00 eq) | HATU (2.85 eq) and DIEA (6.00 eq) |

## Intermediate B-8

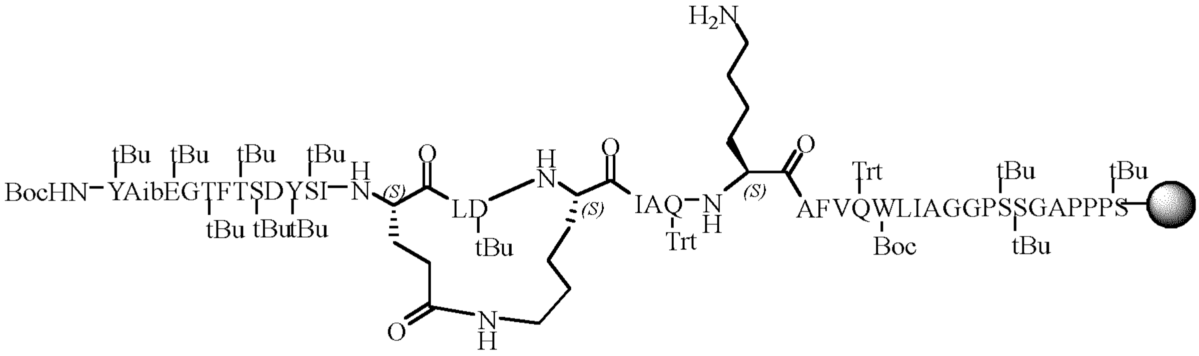

**B-7**

Referring to the synthesis method of **B-1**, **B-8** was obtained after the following operations such as coupling of amino acids, deprotection of Alloc and OAll, ring closure of amide, and deprotection of Dde.

| NO. | Raw Material | Coupling Reagent |
|---|---|---|
| 1 | Fmoc-Ser(tBu)-OH (3.00 eq) | HBTU (2.85 eq) and DIEA (6.00 eq) |
| 2 | Fmoc-Pro-OH (3.00 eq) | HBTU (2.85 eq) and DIEA (6.00 eq) |
| 3 | Fmoc-Pro-OH (3.00 eq) | HBTU (2.85 eq) and DIEA (6.00 eq) |
| 4 | Fmoc-Pro-OH (3.00 eq) | HBTU (2.85 eq) and DIEA (6.00 eq) |
| 5 | Fmoc-Ala-OH (3.00 eq) | HBTU (2.85 eq) and DIEA (6.00 eq) |
| 6 | Fmoc-Gly-OH (3.00 eq) | HBTU (2.85 eq) and DIEA (6.00 eq) |
| 7 | Fmoc-Ser(tBu)-OH (3.00 eq) | HBTU (2.85 eq) and DIEA (6.00 eq) |
| 8 | Fmoc-Ser(tBu)-OH (3.00 eq) | HBTU (2.85 eq) and DIEA (6.00 eq) |
| 9 | Fmoc-Pro-OH (3.00 eq) | HBTU (2.85 eq) and DIEA (6.00 eq) |
| 10 | Fmoc-Gly-OH (3.00 eq) | HBTU (2.85 eq) and DIEA (6.00 eq) |
| 11 | Fmoc-Gly-OH (3.00 eq) | HBTU (2.85 eq) and DIEA (6.00 eq) |
| 12 | Fmoc-Ala-OH (3.00 eq) | HBTU (2.85 eq) and DIEA (6.00 eq) |
| 13 | Fmoc-Ile-OH (3.00 eq) | HBTU (2.85 eq) and DIEA (6.00 eq) |
| 14 | Fmoc-Leu-OH (3.00 eq) | HBTU (2.85 eq) and DIEA (6.00 eq) |
| 15 | Fmoc-Trp(Boc)-OH (3.00 eq) | HBTU (2.85 eq) and DIEA (6.00 eq) |
| 16 | Fmoc-Gln(Trt)-OH (2.00 eq) | HBTU (1.90 eq) and DIEA (4.00 eq) |
| 17 | Fmoc-Val-OH (3.00 eq) | HBTU (2.85 eq) and DIEA (6.00 eq) |
| 18 | Fmoc-Phe-OH (3.00 eq) | HBTU (2.85 eq) and DIEA (6.00 eq) |
| 19 | Fmoc-Ala-OH (3.00 eq) | HBTU (2.85 eq) and DIEA (6.00 eq) |
| 20 | Fmoc-Lys(Dde)-OH(3.00 eq) | HOBT (2.00 eq) and DIC (2.00 eq) |
| 21 | Fmoc-Gln(Trt)-OH (3.00 eq) | HBTU (2.85 eq) and DIEA (6.00 eq) |
| 22 | Fmoc-Ala-OH (3.00 eq) | HBTU (2.85 eq) and DIEA (6.00 eq) |
| 23 | Fmoc-Ile-OH (3.00 eq) | HBTU (2.85 eq) and DIEA (6.00 eq) |
| 24 | Fmoc-Lys(Alloc)-OH (2.00 eq) | HBTU (2.85 eq) and DIEA (6.00 eq) |
| 25 | Fmoc-Asp(tBu)-OH (6.00 eq) | HATU (5.70 eq) and DIEA (12.0 eq) |
| 26 | Fmoc-Leu-OH (6.00 eq) | HATU (5.70 eq) and DIEA (12.0 eq) |
| 27 | Fmoc-Glu(OAll)-OH (2.00 eq) | HATU (5.70 eq) and DIEA (12.0 eq) |

(continued)

| NO. | Raw Material | Coupling Reagent |
|---|---|---|
| 28 | Fmoc-Ile-OH (6.00 eq) | HOAT (6.00 eq) and DIC (6.00 eq) |
| 29 | Fmoc-Ser(tBu)-OH (6.00 eq) | HATU (5.70 eq) and DIEA (12.0 eq) |
| 30 | Fmoc-Tyr(tBu)-OH (6.00 eq) | HATU (5.70 eq) and DIEA (12.0 eq) |
| 31 | Fmoc-Asp(tBu)-OH (6.00 eq) | HATU (5.70 eq) and DIEA (12.0 eq) |
| 32 | Fmoc-Ser(tBu)-OH (6.00 eq) | HATU (5.70 eq) and DIEA (12.0 eq) |
| 33 | Fmoc-Thr(tBu)-OH (6.00 eq) | HATU (5.70 eq) and DIEA (12.0 eq) |
| 34 | Fmoc-Phe-OH (6.00 eq) | HATU (5.70 eq) and DIEA (12.0 eq) |
| 35 | Fmoc-Thr(tBu)-OH (6.00 eq) | HATU (5.70 eq) and DIEA (12.0 eq) |
| 36 | Fmoc-Gly-OH (6.00 eq) | HATU (5.70 eq) and DIEA (12.0 eq) |
| 37 | Fmoc-Glu(tBu)-OH (6.00 eq) | HOBT (6.00 eq) and DIC (6.00 eq) |
| 38 | Fmoc-Aib-OH (3.00 eq) | HATU (2.85 eq) and DIEA (6.00 eq) |
| 39 | Boc-Tyr(tBu)-OH (3.00 eq) | HATU (2.85 eq) and DIEA (6.00 eq) |

**Example 1**

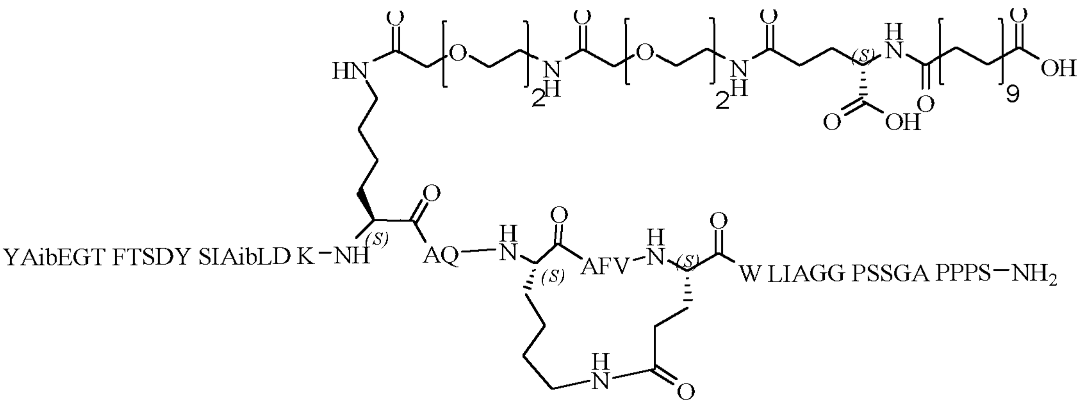

**WX-001**

**1. Coupling of long-chain fatty acids: Referring to** synthesis step 3.2 of **B-1** to complete the coupling of the following fragments:

| NO. | Raw Material | Coupling Reagent |
|---|---|---|
| 1 | Fmoc-AEEA-OH (3.00 eq) | HATU (2.85 eq) and DIEA (6.00 eq) |
| 2 | Fmoc-AEEA-OH (3.00 eq) | HATU (2.85 eq) and DIEA (6.00 eq) |
| 3 | Fmoc-Glu-OtBu (3.00 eq) | HATU (2.85 eq) and DIEA (6.00 eq) |
| 4 | 20-(tBu)-eicosanedioic acid (3.00 eq) | HATU (2.85 eq) and DIEA (6.00 eq) |

**2. Cleavage and Drying of Crude Peptide**

2.1. The cleavage solution was configured according to the following volume

| Reagent | Ratio |
|---|---|
| **Trifluoroacetic acid** | 91 |
| **Triisopropylsilane** | 3 |
| **$H_2O$** | 3 |
| **3-Mercaptopropionic acid** | 3 |

The dried peptide resin was added to the prepared cleavage solution. The mixture was shaken on a shaker for 2.5 hours, and filtered, and the filtrate was added to ice isopropyl ether with 10 times the volume of the filtrate, centrifuged, and washed 3 times with isopropyl ether. After drying in vacuum for 2 hours, the crude peptide was obtained, and the polypeptide compound **WX-001** was obtained after purification. The molecular weight of the polypeptide was confirmed by ESI-MS, calculated for 4811.4, found 4812.0.

**Example 2**

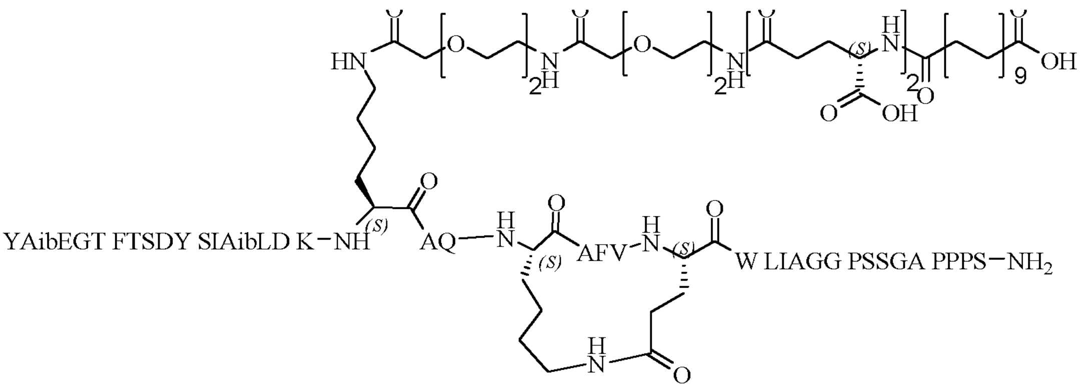

**WX-002**

**1. Coupling of long-chain fatty acids: Referring to** synthesis step 3.2 of **B-1** to complete the coupling of the following fragments:

| NO. | Raw Material | Coupling Reagent |
|---|---|---|
| 1 | Fmoc-AEEA-OH (3.00 eq) | HATU (2.85 eq) and DIEA (6.00 eq) |
| 2 | Fmoc-AEEA-OH (3.00 eq) | HATU (2.85 eq) and DIEA (6.00 eq) |
| 3 | Fmoc-Glu-OtBu (3.00 eq) | HATU (2.85 eq) and DIEA (6.00 eq) |
| 4 | Fmoc-Glu-OtBu (3.00 eq) | HATU (2.85 eq) and DIEA (6.00 eq) |
| 5 | 20-(tBu)-eicosanedioic acid (3.00 eq) | HATU (2.85 eq) and DIEA (6.00 eq) |

**2. Cleavage and Drying of Crude Peptide**

2.1. The cleavage solution was prepared according to the following volume

| Reagent | Ratio |
|---|---|
| **Trifluoroacetic acid** | 91 |
| **Triisopropylsilane** | 3 |
| **$H_2O$** | 3 |
| **3-Mercaptopropionic acid** | 3 |

The dried peptide resin was added to the prepared cleavage solution. The mixture was shaken on a shaker for 2.5 hours, and filtered, and the filtrate was added to ice isopropyl ether with 10 times the volume of the filtrate, centrifuged, and washed 3 times with isopropyl ether. After drying in vacuum for 2 hours, the crude peptide was obtained, and the polypeptide compound **WX-002** was obtained after purification. The molecular weight of the polypeptide was confirmed by ESI-MS, calculated for 4940.5, found 4940.6.

**Example 3**

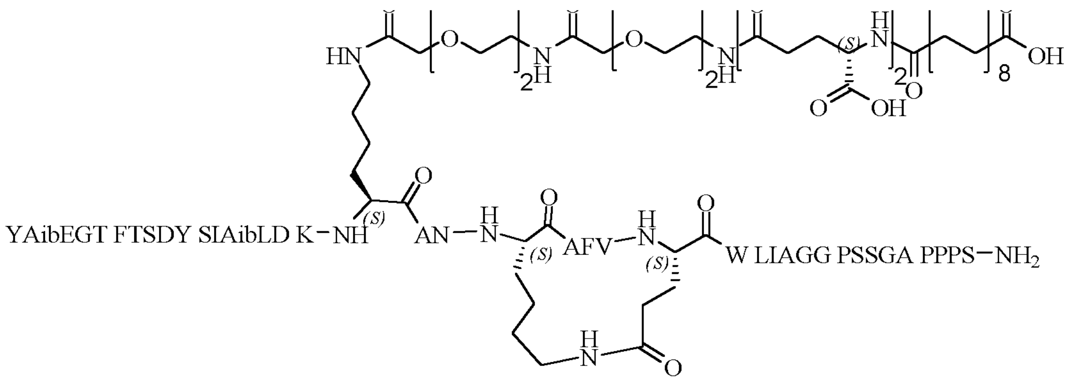

**WX-003**

**1. Coupling of long-chain fatty acids: Referring to** synthesis step 3.2 of **B-1** to complete the coupling of the following fragments:

| NO. | Raw Material | Coupling Reagent |
|---|---|---|
| 1 | Fmoc-AEEA-OH (3.00 eq) | HATU (2.85 eq) and DIEA (6.00 eq) |
| 2 | Fmoc-AEEA-OH (3.00 eq) | HATU (2.85 eq) and DIEA (6.00 eq) |
| 3 | Fmoc-Glu-OtBu (3.00 eq) | HATU (2.85 eq) and DIEA (6.00 eq) |
| 4 | Fmoc-Glu-OtBu (3.00 eq) | HATU (2.85 eq) and DIEA (6.00 eq) |
| 5 | 20-(tBu)-octadecanedioic acid (3.00 eq) | HATU (2.85 eq) and DIEA (6.00 eq) |

**2. Cleavage and Drying of Crude Peptide**

2.1. The cleavage solution was configured according to the following volume

| Reagent | Ratio |
|---|---|
| **Trifluoroacetic acid** | 91 |
| **Triisopropylsilane** | 3 |
| **$H_2O$** | 3 |
| **3-Mercaptopropionic acid** | 3 |

The dried peptide resin was added to the prepared cleavage solution. The mixture was shaken on a shaker for 2.5 hours, and filtered, and the filtrate was added to ice isopropyl ether with 10 times the volume of the filtrate, centrifuged, and washed 3 times with isopropyl ether. After drying in vacuum for 2 hours, the crude peptide was obtained, and the polypeptide compound WX-003 was obtained after purification. The molecular weight of the polypeptide was confirmed by ESI-MS, calculated for 4912.5, found 4913.1.

**Example 4**

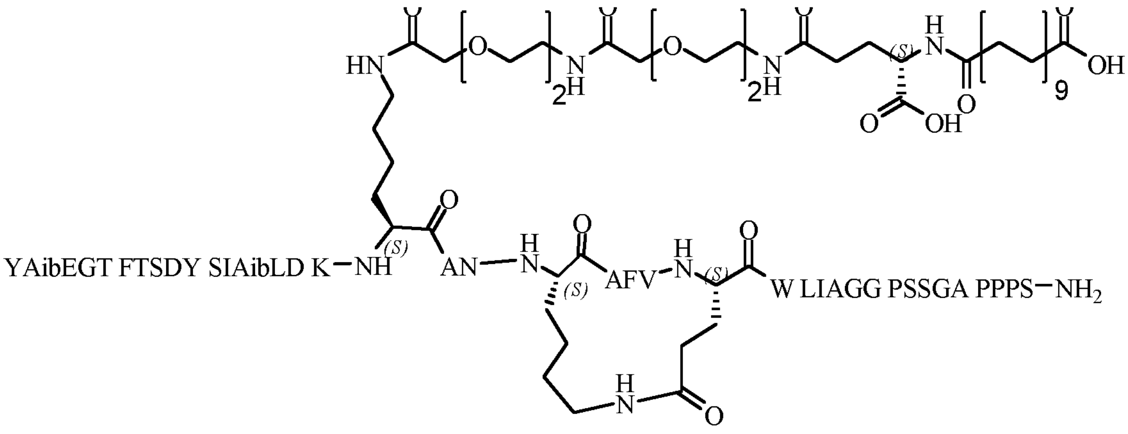

**WX-004**

Referring to the synthesis of **WX-001, WX-004** was obtained through intermediate **B-2**. The molecular weight of the polypeptide was confirmed by ESI-MS, calculated for 4797.4, found 4797.3.

Example 5

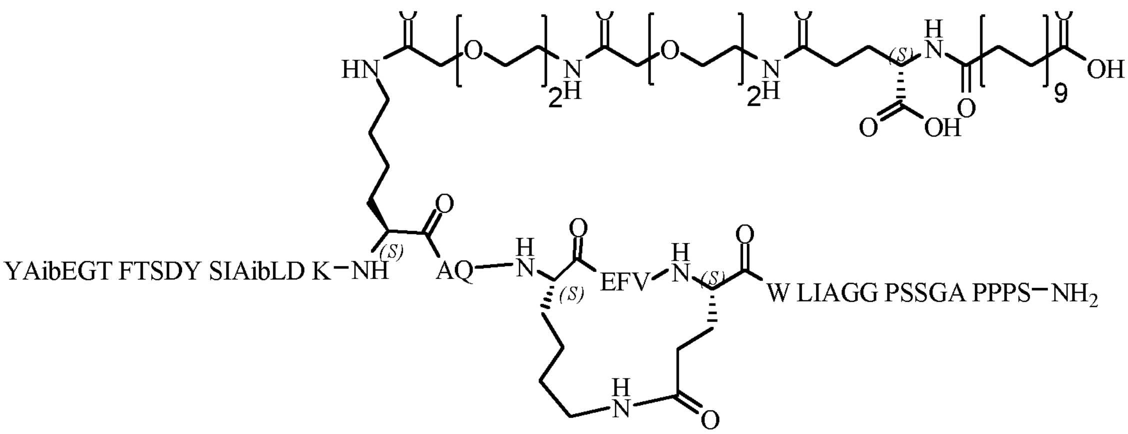

**WX-005**

Referring to the synthesis of **WX-001, WX-005** was obtained through intermediate **B-3**. The molecular weight of the polypeptide was confirmed by ESI-MS, calculated for 4869.5, found 4869.3.

**Example 6**

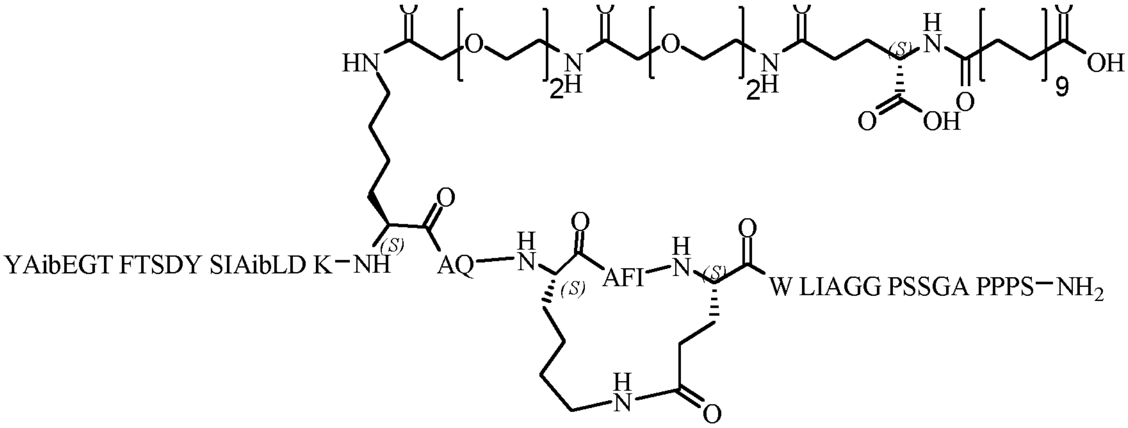

**WX-006**

Referring to the synthesis of **WX-001, WX-006** was obtained through intermediate **B-4**. The molecular weight of the polypeptide was confirmed by ESI-MS, calculated for 4825.5, found 4825.5.

**Example 7**

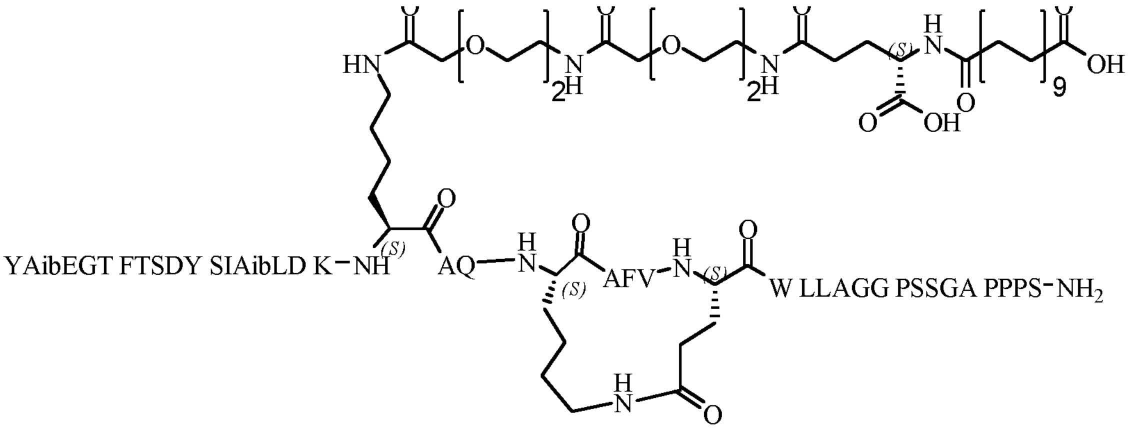

**WX-007**

Referring to the synthesis of **WX-001, WX-007** was obtained through intermediate **B-5**. The molecular weight of the polypeptide was confirmed by ESI-MS, calculated for 4811.4, found 4811.1.

**Example 8**

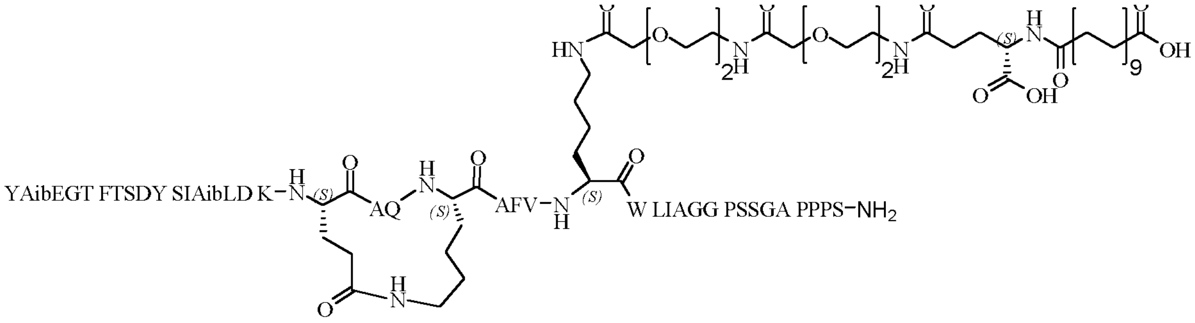

**WX-008**

Referring to the synthesis of **WX-001, WX-008** was obtained through intermediate **B-6**. The molecular weight of the polypeptide was confirmed by ESI-MS, calculated for 4811.4, found 4811.4.

**Example 9**

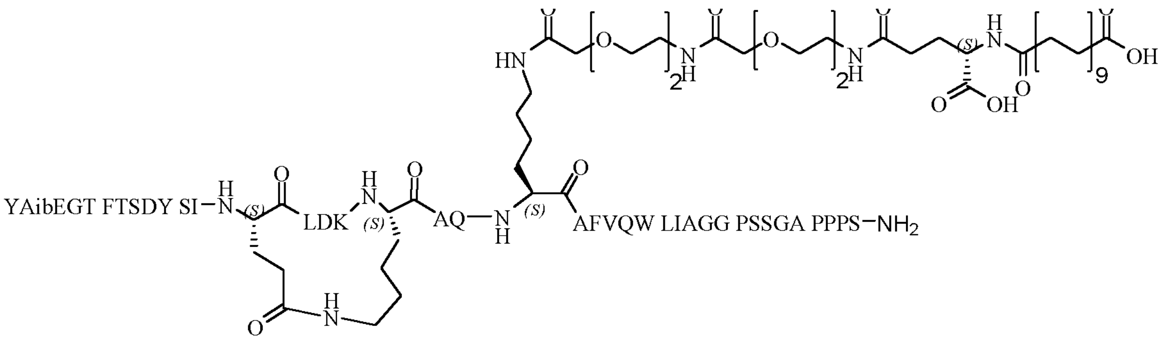

**WX-009**

Referring to the synthesis of **WX-001, WX-009** was obtained through intermediate **B-7**. The molecular weight of the polypeptide was confirmed by ESI-MS, calculated for 4854.5, found 4854.9.

**Example 10**

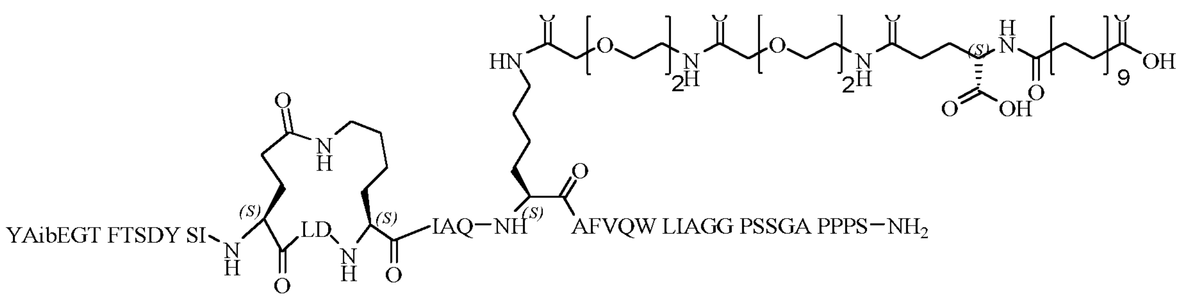

**WX-010**

Referring to the synthesis of **WX-001, WX-010** was obtained through intermediate **B-8**. The molecular weight of the polypeptide was confirmed by ESI-MS, calculated for 4839.4, found 4839.6.

**Biological Test Data**

**Experimental Example 1: GLP-1R/GIPR Agonist Activity Test *in vitro***

**A. Main Material:**

**1) Cell Strain**

The cell strains were constructed by Shanghai WuXi AppTec. See the table below for details.

| Target | Host Cell | Clone |
|---|---|---|
| GLP-1R | HEK293 | N/A |
| GIPR | CHO | N/A |

**2) Reagents and Consumables**

| Name | Batch. | Item NO. | Manufacturer |
|---|---|---|---|
| cAMP assay kit | 29F | 62AM4PEJ | Cisbio |
| 1M HEPES | 2120919 | 15630-106 | Invitrogen |
| Hanks balanced salt solution (HB S S) | 2185775 | 14025 | Invitrogen |
| Human serum albumin (HSA) | SLCF7301 | A1653-10G | Sigma |
| Casein | SLCC9458 | C4765-10mL | Sigma |
| 3-Isobutyl-1-methylxanthine (IBMX) | STBF6061V | I5879-5G | Sigma |
| ECHO qualified 384-well plate | 0006433672 | PP-0200 | Labcyte |
| OptiPlate-384 | 8210-19481 | 6007299 | PerkinElmer |

**3) Instruments**

| Name | Model | Manufacturer |
|---|---|---|
| EnVision | envision2014 | PerkinElmer |
| Vi-cell counter | Vi-CELL™ XR Cell Viability Analyzer | Beckman |
| Bravo | Bravo V11 | Agilent |
| ECHO | ECHO 555 | Labcyte |
| Centrifuge | Allegra™ 25R Centrifuge | Beckman |

**B. Method**

1) Experimental Materials

**Experimental Buffer**

| Reagent | Storage Concentration | Volume | Final Concentration |
|---|---|---|---|
| Hanks balanced salt solution | 1x | 48.7 mL/ 44.7 mL | About 1x |
| HEPES buffer | 1 mol/L | 250 μL | 5 mmol/L |
| 5% casein solution (HEPES)/ 10% human serum albumin solution (HSA) | 5%/ 10% | 1000 μL/ 5000 μL | 0.10%/ 1% |
| 3-Isobutyl-1-methylxanthine (IBMX) | 500 mmol/L | 50 μL | 0.5 mmol/L |

**Detection Reagent Preparation**

| Reagent | Storage Concentration | Volume | Final Concentration |
|---|---|---|---|
| Cell lysate | 1x | 9.5 mL | About 1x |
| D2-cAMP solution | 40x | 250 μL | 1x |
| cAMP-antibody solution | 40x | 250 μL | 1x |

**2) Experimental Method**

a) Preparation of the Compound Plate:

The test compound was serially diluted 4-fold with 10 points. The initial concentration was 30 μM, and the dilution was completed with Bravo.

b) Compound Transference:

1) 100 nL of the compound was transferred to OptiPlate-384 plate using Echo.
2) The OptiPlate-384 plate was centrifuged at 1000 rpm for 5 seconds.

c) Preparation of Cell Suspension:

1) A GLP-1R/GIPR cell cryopreservation tube was quickly thawed in warm water at 37°C.
2) The cell suspension was transferred to a Transfer15 mL centrifuge tube and gently rinsed with 10 mL of HBSS.
3) The centrifuge tube was centrifuged at 1000 rpm for 1 minute at room temperature.
4) The supernatant was discarded.
5) The bottom cells were gently dispersed and gently rinsed with 10 mL of HBSS. The cells were centrifuged to settle, and finally resuspended with the experimental buffer.
6) Vi-cell was used to measure cell density and activity.
7) The GLP-1R/GIPR cell concentration was diluted to $2.0*10^5$ /mL with the experimental buffer.
8) 100 nL of the diluted cell suspension was transferred into the OptiPlate-384 plate.
9) The diluted cell suspension was incubated at room temperature for 30 minutes.

d) Addition of Detection Reagent:

1) 10 μL of 800 nM gradient-diluted cAMP standard was added to the empty well of the OptiPlate-384 plate.
2) 10 μL of cAMP detection reagent was added.

3) The OptiPlate-384 plate was covered with TopSeal-A film and incubated at room temperature for 60 minutes.

TopSeal-A was removed and read in EnVision.

**C. Experimental Results**

The experimental results are shown in Table 1.

Table 1 GLP-1R/GIPR Agonist Activity Test Results *in vitro*

| **Polypeptid e Drugs** | **GLP-1R Agonist Activity** | | | **GIPR Agonist Activity** | | |
|---|---|---|---|---|---|---|
| | $EC_{50}$ (nM) | | Ratio | $EC_{50}$ (nM) | | Ratio |
| | (-) 1% HSA | (+) 1% HSA | | (-) 1% HSA | (+) 1% HSA | |
| **WX-001** | 0.014 | 3.664 | 265 | 0.44 | 23.04 | 53 |
| **WX-002** | 0.052 | NA | NA | 0.29 | NA | NA |
| **WX-003** | 0.090 | NA | NA | 0.30 | NA | NA |
| **WX-005** | 0.042 | NA | NA | 0.22 | NA | NA |
| **WX-006** | 0.051 | NA | NA | 0.45 | NA | NA |
| **WX-007** | 0.14 | NA | NA | 0.13 | NA | NA |
| **WX-008** | 0.08 | NA | NA | 0.32 | NA | NA |
| **WX-010** | 0.03448 | 33.96 | 984.9 | 11.86 | 443 | 37.4 |

**Conclusion:** The compounds of the present disclosure have strong agonist activity on GLP-1R/GIPR.

**Experimental Example 2: Pharmacokinetic Evaluation of Compounds in Rats**

**A. Experimental Purpose**

To test the pharmacokinetics of compounds in SD rats *in vivo*

**B. Experimental Operation**

The pharmacokinetic characteristics of the compounds in rodents after subcutaneous injection were tested according to the standard protocol. In the experiment, the candidate compounds were formulated into clear solutions, and a single subcutaneous injection (SC, 0.048 mpk) was given to the rats. The injection vehicle was citrate buffer (20 mM, pH=7). Whole blood was collected and prepared to obtain plasma. Drug concentration was analyzed by LC-MS/MS method, and pharmacokinetic parameters were calculated by Phoenix WinNonlin software.

**C. Experimental Results**

The experimental results are shown in Table 2.

Table 2 Pharmacokinetic Test Results

| **Compound Number** | **Maximum Plasma Concentration (nM)** | **Half-life $T_{1/2}$ (h)** | **SC Concentration Integral $AUC_{0-72\ h}$ (nM.hr)** |
|---|---|---|---|
| **WX001** | 22 | 12 | 728 |
| **WX002** | 14 | 17 | 1316 |
| **WX005** | 14 | 17 | 1329 |
| **WX006** | 16 | 10 | 846 |

Conclusion: The compounds of the present disclosure have excellent pharmacokinetic properties in rats.

**Experimental Example 3: Pharmacokinetic Evaluation of Compounds in Rats**

**A. Experimental Purpose**

To test the pharmacokinetics of compounds in C57BL/6 mice *in vivo*

**B. Experimental Operation**

The pharmacokinetic characteristics of the compounds in rodents after intravenous injection and subcutaneous injection were tested according to the standard protocol. In the experiment, the candidate compounds were formulated into clear solutions, and a single intravenous injection (IV, 0.048 mpk) and subcutaneous injection (SC, 0.048 mpk) were given to the mice. The vehicle for intravenous injection was PBS buffer (pH=7), and the vehicle for subcutaneous injection was citrate buffer (20 mM, pH=7). Whole blood was collected and prepared to obtain plasma. Drug concentration was analyzed by LC-MS/MS method, and pharmacokinetic parameters were calculated by Phoenix WinNonlin software.

**C. Experimental Results**

The experimental results are shown in Table 3.

Table 3 Pharmacokinetic Test Results

| Compound Number | Clearance (mL/min/kg) | Half-life $T_{1/2}$ (h) | SC Concentration Integral $AUC_{0-72h}$ (nM.hr) | Bioavailability F (%) |
|---|---|---|---|---|
| **WX001** | 0.088 | 15.5 | 1554 | 85.5% |

Conclusion: The compounds of the present disclosure have excellent pharmacokinetic properties in mice.

**Experimental Example 4: Pharmacokinetic Evaluation of Compounds in Cynomolgus Monkeys**

**A. Experimental Purpose**

To test the pharmacokinetics of compounds in cynomolgus monkeys *in vivo*

**B. Experimental Operation**

The pharmacokinetic characteristics of the compounds in mammals after intravenous injection and subcutaneous injection were tested according to the standard protocol. In the experiment, the candidate compounds were formulated into clear solutions, and a single subcutaneous injection (SC, 0.02 mpk) was given to the cynomolgus monkeys. The vehicle for subcutaneous injection was citrate buffer (20 mM, pH=7). Whole blood was collected and prepared to obtain plasma. Drug concentration was analyzed by LC-MS/MS method, and pharmacokinetic parameters were calculated by Phoenix WinNonlin software.

**C. Experimental results**

The experimental results are shown in Table 4.

Table 4 Pharmacokinetic Test Results

| Compound Number | $C_{max}$ (nM) | $T_{max}$ (h) | Half-life $T_{1/2}$ (h) | SC Concentration Integral $AUC_{0-240h}$ (nM.hr) |
|---|---|---|---|---|
| **WX001** | 30 | 21 | 80 | 3973 |
| **WX002** | 33 | 29 | 81 | 4582 |
| **WX005** | 31 | 40 | 98 | 4553 |
| **WX006** | 30 | 29 | 72.4 | 3640 |

Conclusion: The compounds of the present disclosure have excellent pharmacokinetic properties in monkeys.

**Experimental Example 5: Plasma Stability (PLS) Test**

**A. Experimental Purpose**

To study the stability of test compounds in the plasma of normal mice.

**B. Experimental Operation**

1. Before the experiment, the coagulated frozen plasma was thawed in a water bath at 37°C. Plasma was centrifuged at 4000 rpm for 5 min. If there was blood clot, the blood clot was removed and the pH value was adjusted to 7.4±0.1.
2. Preparation of the test compound solution: 100 μM of the solution was prepared by dilution with DMSO.
3. 98 μL of blank control plasma was added to 2 μL of the test compound solution (100 μM), so that the final concentration of the mixed solution of the two reached 2 μM, and the mixed solution was placed in a 37°C water bath for incubation.
4. At each time point (0, 10, 30, 60, and 120 min), 100 μL of $H_3PO_4$ solution and 800 μL of stop solution (200 ng/mL of tolbutamide and 200 ng/mL of labetalol in 100% methanol solution) were added to precipitate protein, and mixed thoroughly.
5. The samples were centrifuged at 4000 rpm for 20 min, and 100 μL of supernatant was taken from each well for LC-MS/MS analysis.

**C. Experimental Results**

The experimental results are shown in Table 5.

Table 5 PLS Test Results

| Compound Number | WX001 | WX002 | WX005 | WX006 |
|---|---|---|---|---|
| PLS (H/M) $T_{1/2}$ (min) | > 289/> 289 | > 289/> 289 | > 289/> 289 | > 289/> 289 |

Conclusion: The compounds of the present disclosure have excellent plasma stability.

**Experimental Example 6: Plasma Protein Binding (PPB) Test**

**A. Experimental Purpose**

To study the binding of the test compounds to human/mouse plasma albumin.

**B. Experimental Operation**

1. Matrix preparation: On the day of the experiment, the plasma was thawed in cold water and centrifuged at 3220 rpm for 5 min to remove all blood clots. The pH of the obtained plasma was measured and adjusted to 7.4±0.1 using 1% phosphoric acid or 1N sodium hydroxide as needed.
2. Dilution steps of the test compounds: The test compounds were dissolved in dimethyl sulfoxide (DMSO) to prepare stock solutions at concentrations of 10 mM and 2 mM, respectively. A 40 μM working solution was prepared by diluting 2 μL of the stock solution (2 mM) with 98 μL of DMSO. A 400 μM working solution of the control compound was prepared by diluting 10 μL of the stock solution with 240 μL of DMSO. The working solution of the compound (5 μL) was mixed with the blank matrix (995 μL) at a ratio of 1:200 to prepare the loading matrix.
3. Analysis Steps

3.1 An equal amount of 30 μL of the loading matrix (n=2) was transferred to the sample collection plate to prepare samples of time 0 (T0) for residue determination. The samples were immediately matched to the

corresponding blank buffer in a final volume of 60 μL with a 1:1 volume ratio of plasma to buffer in each well. Then, 60 μL of 4% $H_3PO_4$ in $H_2O$ and 480 μL of stop solution containing an internal standard were then added to the T0 samples of the test compounds. They were then stored at 2-8°C with other samples pending for further processing.
3.2 The remaining plasma samples were pre-incubated in a carbon dioxide incubator at 37±1°C for 30 min. The prepared protein-free samples (F samples) and loading matrix samples (230 μL) were transferred to polycarbonate tubes (n = 2) and ultracentrifuged at 37°C and 155000 × g (35000 rpm) for 4 hours.
3.3 To prepare T samples (test samples), an additional matrix-containing sample was transferred to a separate 96-well plate (sample incubation plate) and incubated at 37°C for 4 hours.
3.4 After centrifugation, 30 μL of protein-free sample and 30 μL of T sample were transferred from the second layer of supernatant (below the upper layer) to a new sample collection plate. Each sample was mixed with the corresponding blank buffer or matrix in a final volume of 60 μL with a matrix:buffer volume ratio of 1:1. 60 μL of 4% $H_3PO_4$ aqueous solution and 480 μL of stop solution (containing internal standard) were added to all samples. The mixture was centrifuged at 4000 rpm for 20 min, and 100 μL of the supernatant of each sample was taken for LC-MS/MS analysis.

**C. Experimental Results**

The experimental results are shown in Table 6. Note: NA means the plasma-protein binding is too high, and no free drug can be detected under a normal concentration of plasma protein.

Table 6 PPB Test Results

| Compound Number | WX001 | WX002 | WX005 | WX006 |
|---|---|---|---|---|
| PPB% unbound (HIM) | NA/NA | NA/0.21% | NA/0.22% | NA/0.35% |

Conclusion: The compounds of the present disclosure have extremely high plasma-protein binding.

**Experimental Example 7: Kidney Homogenate Stability (BHS) Test**

**A. Experimental Purpose**

To study the stability of the test compounds in normal mouse kidney homogenate.

**B. Experimental Operation**

1. Before the experiment, the frozen kidney homogenate was thawed in a 37°C water bath.
2. Test compounds: 10 mM stock solution was diluted with DMSO to prepare 1 mM intermediate solution;
3. test compounds: 1 mM intermediate solution was diluted with DMSO to prepare 50 μM dosing solution;
4. at each time point (0, 10, 30, 60, 120, 180, 240 min), 100 μL of 4% $H_3PO_4$ and 800 μL of stop solution were added to precipitate the protein, and mixed thoroughly.
5. The samples were centrifuged at 4000 rpm for 10 min, and 100 μL of supernatant was taken from each well to the plate. The samples were shaken at 800 rpm for approximately 10 min before submitting for LC-MS/MS test.

**C. Experimental Results**

The experimental results are shown in Table 7.

Table 7 BHS Test Results

| Compound Number | WX00 1 | WX002 | WX005 | WX006 |
|---|---|---|---|---|
| BHS ($T_{1/2}$ (min)) | 14 | 20 | 21 | 234 |

**Experimental Example 8: Intraperitoneal Glucose Tolerance Test (ipGTT) - *in vivo* Pharmacodynamic Evaluation of Compounds in Mice**

**A. Experimental Purpose**

To study the improvement effect of the test compounds on the glucose tolerance of normal mice

**B. Experimental Operation**

1. After the mice were grouped according to body weight and blood glucose, each group of animals was injected with the test compound (0.3 nmol/kg) and vehicle (20 mM citrate buffer). The mice were fasted overnight, and intraperitoneally injected with glucose solution (2 g/kg, 10 mL/kg) after 18 hours;
2. a glucometer was used to measure blood glucose concentrations at -60, 0, 15, 30, 60 and 120 minutes after glucose administration.

**C. Experimental Results**

The experimental results are shown in Table 8.

Table 8 ipGTT Test Results

| Compound Number | Vehicle | WX001 | WX002 | WX005 | WX006 |
|---|---|---|---|---|---|
| Blood glucose $AUC_{0-120min}$ (mmol.min) | 1916 | 1277 | 1150 | 1143 | 1164 |

Conclusion: The compounds of the present disclosure have excellent effects on improving glucose tolerance.

**Experimental Example 9: Pharmacodynamic study - *in vivo* Pharmacodynamic Evaluation in db/db Mice**

**A. Experimental Purpose**

To study the glycemic control effect of the test compounds on type II diabetic db/db mice

**B. Experimental Operation**

1. After the db/db mice arrived at the facility, they were kept in an animal breeding room with strictly controlled environmental conditions. The temperature in the breeding room was maintained at 20-24°C and the humidity was maintained at 30-70%. The temperature and humidity in the breeding room were monitored in real-time by a hygrothermograph, and the temperature and humidity were recorded twice a day (one time in the morning and one time in the afternoon). The daylighting in the animal breeding room was controlled by an electronic timing lighting system, and the lights were turned on for 12 hours and turned off for 12 hours every day (turned on at 7:00 in the morning and turned off at 19:00 in the afternoon). During the experiment, the animals were kept in single cages, and toys were provided for each cage. During the experiment, the animals had free access to food (feed for growth/breeding of rats and mice) and drinking water.
2. The vehicle and the test compound (15 nmol/kg) were subcutaneously injected into each group of animals. The administration time: 9:30-11:00 in the morning, and the administration frequency was once a day, and the administration was continuous for 4 weeks.

**C. Experimental Results**

The experimental results are shown in Table 9.

Table 9 Glucose-lowering Effect of the Test Compounds in db/db Mice

| Compound Number | Vehicle | WX001 | WX002 | WX005 | WX006 |
|---|---|---|---|---|---|
| ΔHbA1c% (compared with Day 1 after four weeks of continuous administration) | 15% | -28% | -21% | -21% | -23% |

Conclusion: The compounds of the present disclosure exhibit excellent glucose-lowering efficacy in db/db mice.

**Experimental Example 10: Pharmacodynamic study - *in vivo* Pharmacodynamic Evaluation in DIO Mice**

**A. Experimental Purpose**

To study the weight loss effect of the test compounds in DIO mice

**B. Experimental Operation**

1. After the DIO mice arrived at the WuXi AppTec facility, they were kept in an animal breeding room with strictly controlled environmental conditions. The temperature in the breeding room was maintained at 20-24°C, and the humidity was maintained at 30-70%. The temperature and humidity in the breeding room were monitored in real-time by a hygrothermograph, and the temperature and humidity were recorded twice a day (one time in the morning and one time in the afternoon). The daylighting in the animal breeding room was controlled by an electronic timing lighting system, and the lights were turned on for 12 hours and turned off for 12 hours every day (turned on at 7:00 in the morning and turned off at 19:00 in the afternoon). During the experiment, the animals were kept in single cages, and toys were provided for each cage. During the experiment, the animals had free access to food (feed for the growth/breeding of rats and mice) and drinking water.
2. The vehicle and the test compound (10 nmol/kg) were subcutaneously injected into each group of animals, respectively. The administration time: 9:30 in the morning, and the administration frequency was once every three days, and the administration cycle was 22 days.

**C. Experimental Results**

The experimental results are shown in Table 10.

Table 10 Pharmacodynamics of the Test Compounds in DIO Mice

| Compound Number | Vehicle | WX001 | WX002 | WX005 | WX006 |
|---|---|---|---|---|---|
| Δ Body weight % (after 22 days compared with day 1) | 0.2% | -29% | -26% | -24% | -32% |
| Δ Adipose tissue % (after 22 days compared to day 1) | 2% | -54% | -48% | -47% | -62% |
| Δ Non-adipose tissue % (after 22 | -3% | -11% | -10% | -8% | -18% |
| days compared with day 1) | | | | | |

Conclusion: The compounds of the present disclosure exhibit excellent weight loss efficacy in DIO mice.

## Claims

**1.** A compound represented by the following formula or a pharmaceutically acceptable salt thereof,

SEQ ID NO1: YAibEGT FTSDY SIAibLD $KK^1AZ_0K_0$ $Z_1FZ_2E_0W$ $LZ_3AGG$ PSSGA $PPPS_0$
SEQ ID NO2: YAibEGT FTSDY SIAibLD $KE_0AQK_0$ AFVK'W LIAGG PSSGA $PPPS_0$
SEQ ID NO3: YAibEGT FTSDY $SIE_0LD$ $KK_0AQK^1$ AFVQW LIAGG PSSGA PPPSo
SEQ ID NO4: YAibEGT FTSDY $SIE_0LD$ $K_0IAQK^1$ AFVQW LIAGG PSSGA PPPSo

wherein

Aib has a structure of

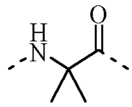

;

$S_0$ is selected from

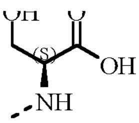

and

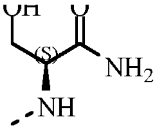

;

$Z_0$ is selected from glutamine (Q) and asparagine (N);
$Z_1$ is selected from alanine (A) and glutamic acid (E);
$Z_2$ is selected from valine (V) and isoleucine (I);
$Z_3$ is selected from isoleucine (I) and leucine (L);
$E_0$ and $K_0$ indicate that the carboxyl group on the side chain of the glutamic acid and the amino group on the side chain of the lysine jointly form a lactam structure, and in this case,
$K_0Z_1FZ_2E_0$ has a structure of

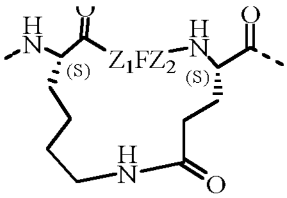

,

$E_0AQK_0$ has a structure of

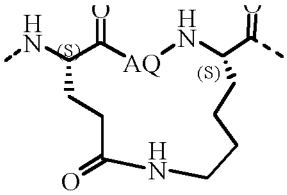

,

$E_0LDKK_0$ has a structure of

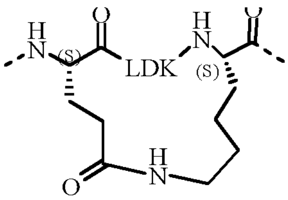

,

and $E_0LDK_0$ has a structure of

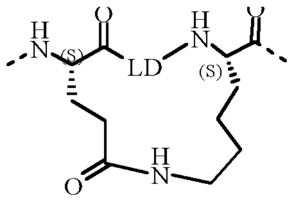

;

K' indicates that the amino group on the side chain of the lysine is connected to -X-$X_1$-$X_2$, and has a structure of

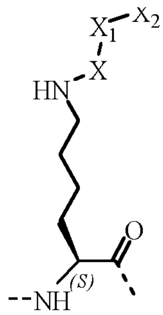

;

X is selected from

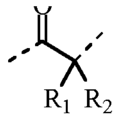

;

$R_1$ and $R_2$ are each independently selected from H and $CH_3$;
$X_1$ is selected from

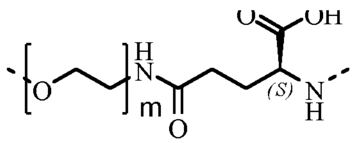

,

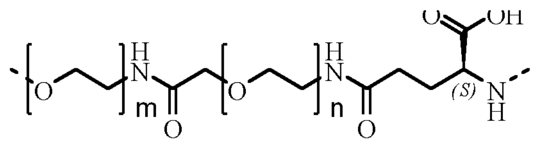

,

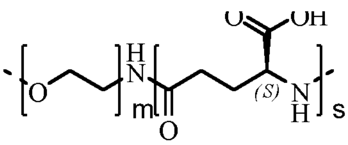

,

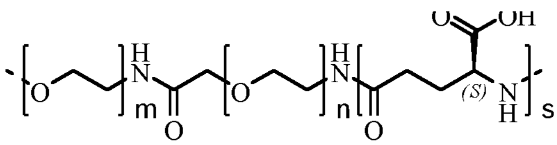

and

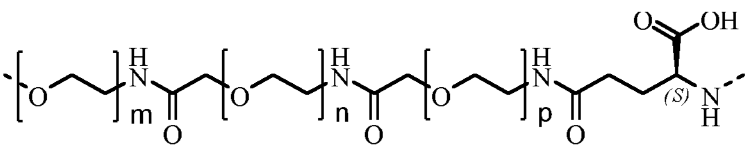

;

$X_2$ is selected from

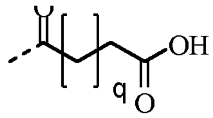

;

m, n and p are each independently selected from 2 and 3;
s is selected from 2 and 3;
q is selected from 15, 16, 17, 18 and 19.

**2.** The compound or the pharmaceutically acceptable salt thereof according to claim 1, which is selected from:

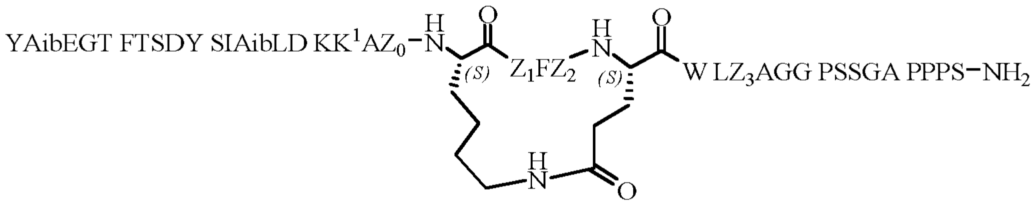

**(P-1)**

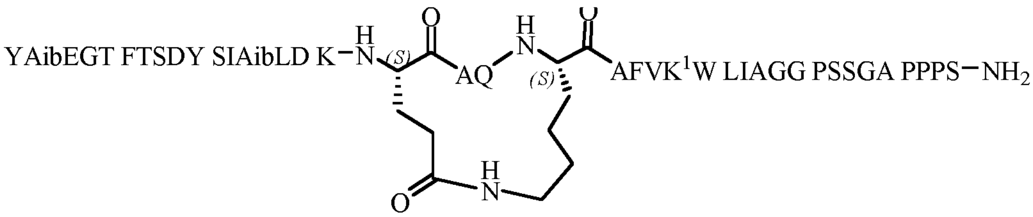

**(P-2)**

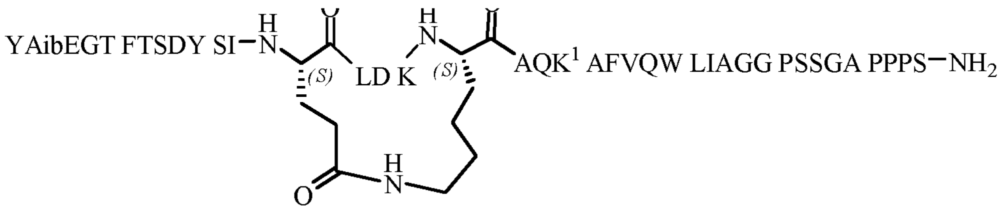

**(P-3)**

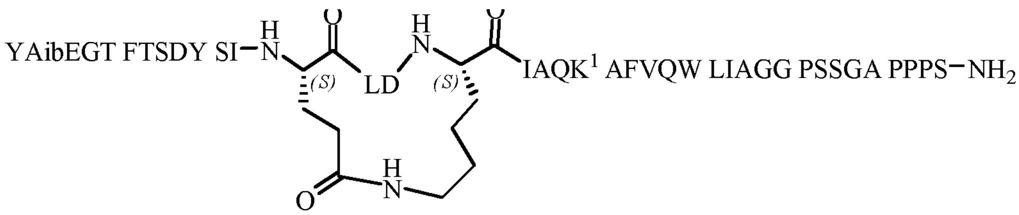

**(P-4)**

wherein
Aib, $Z_0$, $Z_1$, $Z_2$, $Z_3$ and $K^1$ are as defined in claim 1.

3. The compound or the pharmaceutically acceptable salt thereof according to claim 1 or 2, wherein X is selected from $-C(=O)-CH_2-$.

4. The compound or the pharmaceutically acceptable salt thereof according to claim 1 or 2, wherein m, n and p are each independently selected from 2.

5. The compound or the pharmaceutically acceptable salt thereof according to claim 1 or 2, wherein s is independently selected from 2.

6. The compound or the pharmaceutically acceptable salt thereof according to claim 1 or 2, wherein q is independently selected from 15 and 17.

7. The compound or the pharmaceutically acceptable salt thereof according to claim 1 or 2, wherein $X_1$ is selected from

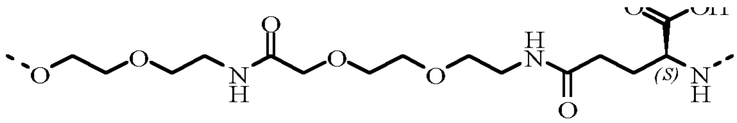

and

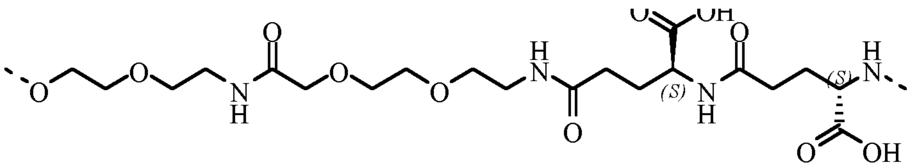

.

8. The compound or the pharmaceutically acceptable salt thereof according to claim 1 or 2, wherein $X_2$ is selected from

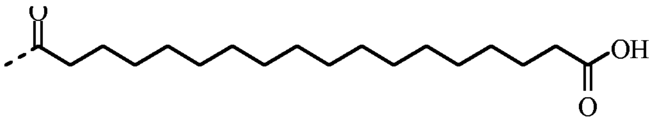

and

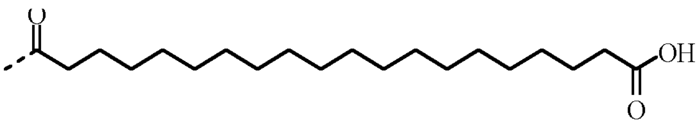

.

9. The compound or the pharmaceutically acceptable salt thereof according to claim 1 or 2, wherein $-X-X_1-X_2$ is selected from

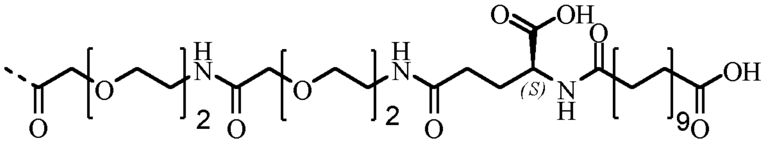

,

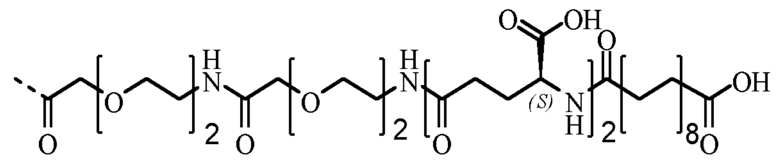

and

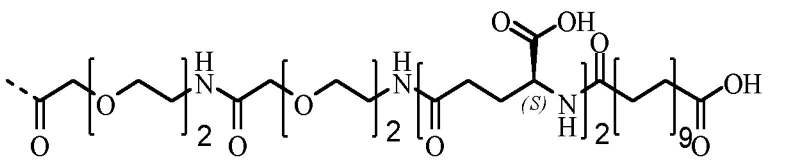

**10.** A compound represented by the following formula or a pharmaceutically acceptable salt thereof,

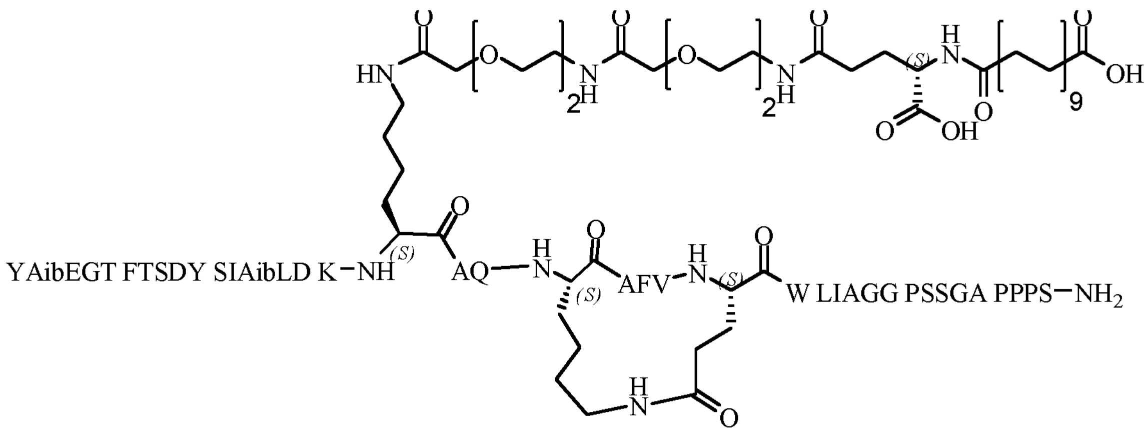

**WX-001**

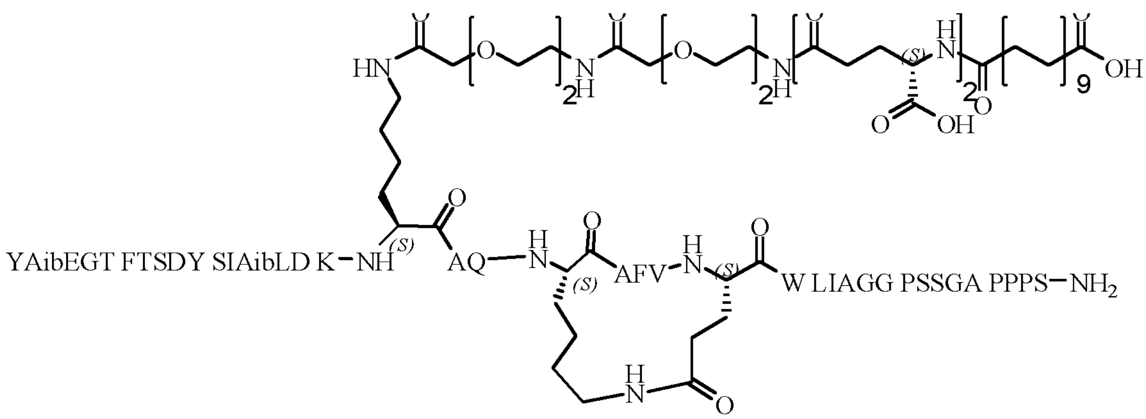

**WX-002**

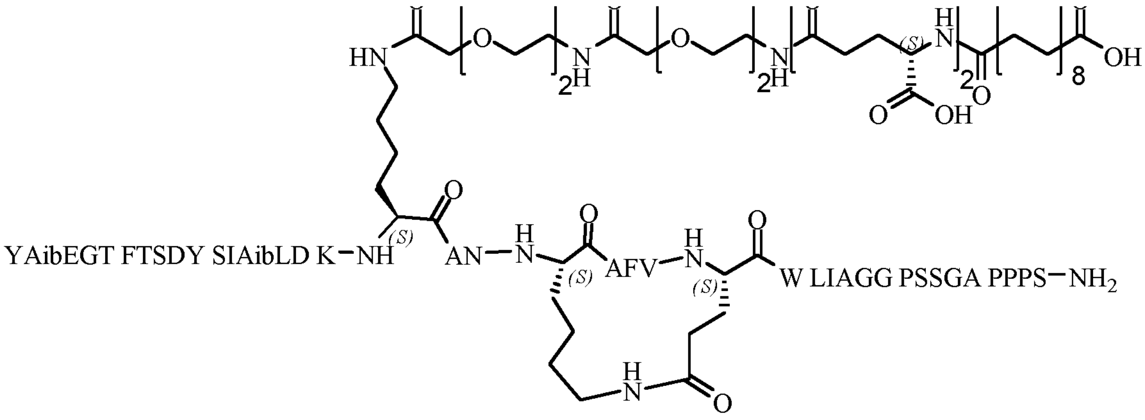

**WX-003**

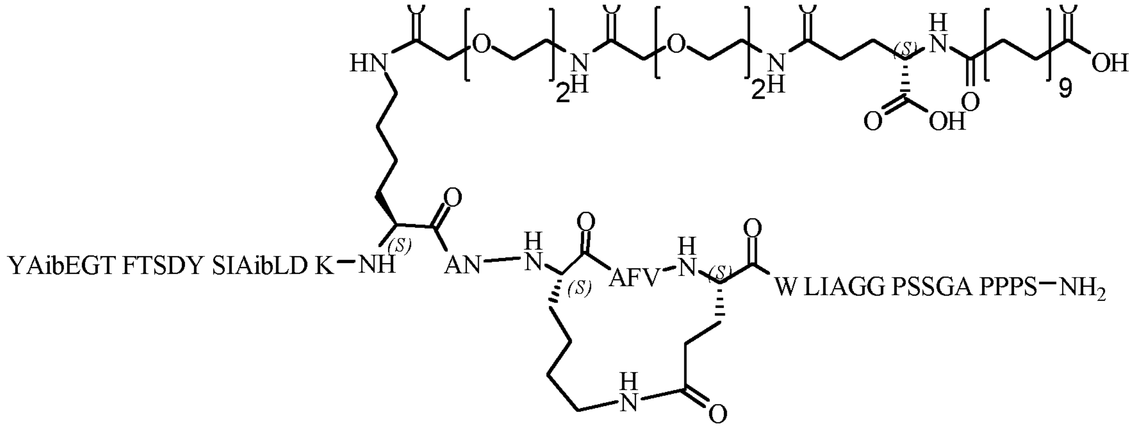

**WX-004**

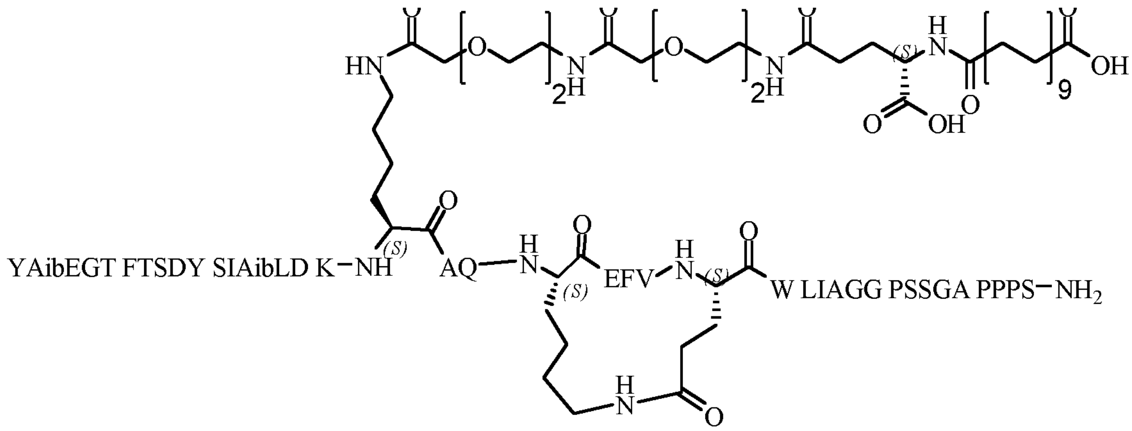

**WX-005**

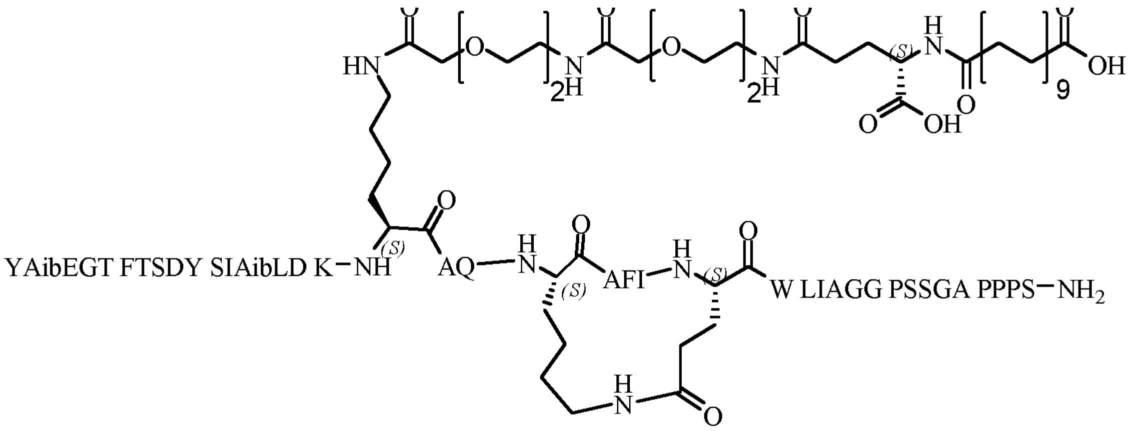

**WX-006**

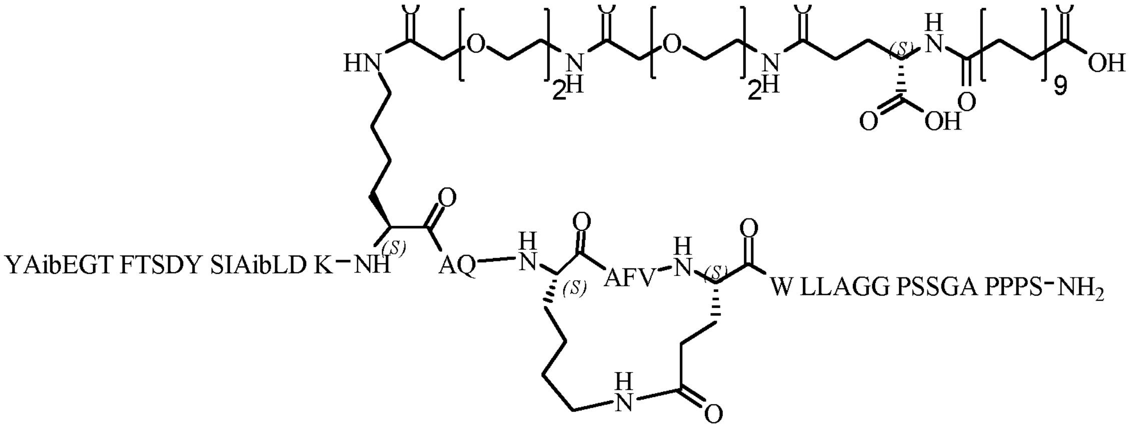

**WX-007**

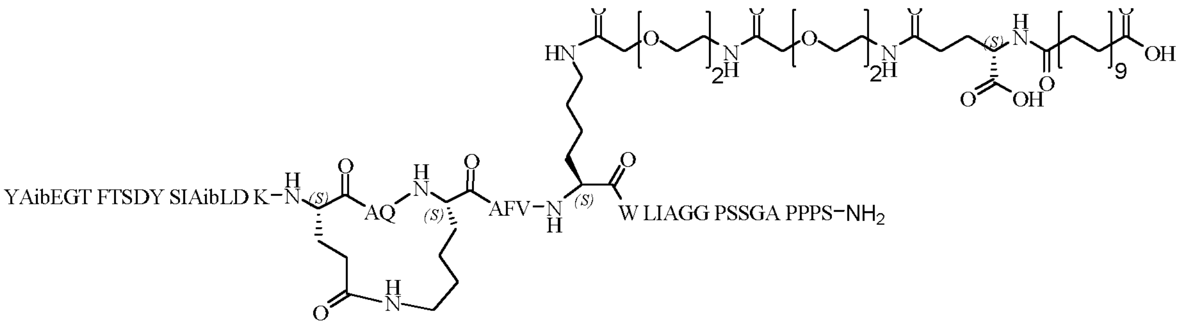

**WX-008**

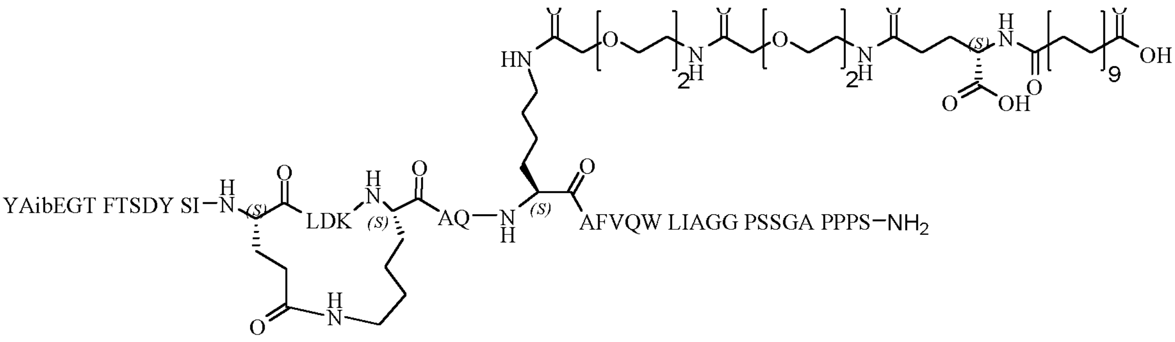

WX-009

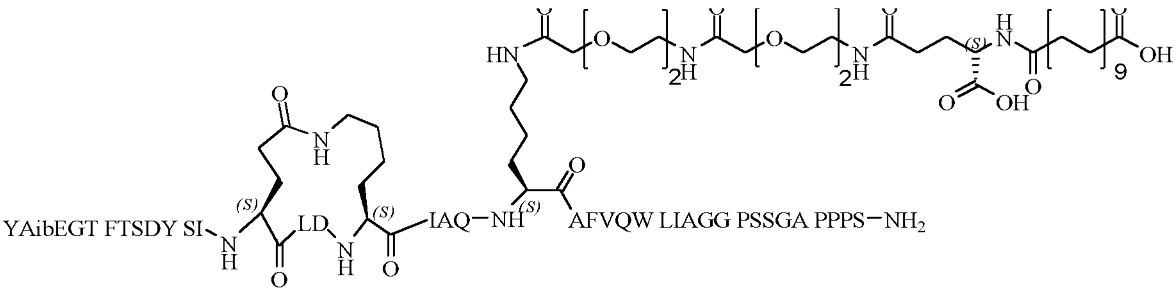

WX-010

11. A use of the compound or the pharmaceutically acceptable salt thereof according to any one of claims 1 to 10 in the manufacture of a medicament for treating obesity and diabetes.

## INTERNATIONAL SEARCH REPORT

International application No. **PCT/CN2021/135180**

**A. CLASSIFICATION OF SUBJECT MATTER**

C07K 14/605(2006.01)i；A61K 38/26(2006.01)i；A61P 3/00(2006.01)i；A61P 3/10(2006.01)i

According to International Patent Classification (IPC) or to both national classification and IPC

**B. FIELDS SEARCHED**

Minimum documentation searched (classification system followed by classification symbols)

C07K，A61K，A61P

Documentation searched other than minimum documentation to the extent that such documents are included in the fields searched

Electronic data base consulted during the international search (name of data base and, where practicable, search terms used)

CNABS, SIPOABS, DWPI, CNTXT, EPTXT, USTXT, WOTXT, CNKI, 万方, EBI, NCBI, 百度, ISI Web of Knowledge, PUBMED, STN: 南京明德新药研发有限公司, 潘志祥, 江志赶, 贺海鹰, 黎健, 陈曙辉, GIP, GIPR, 肥胖症, 糖尿病, Aib, 内酰胺, Lactam Modifications, 谷氨酸, 羧基, 赖氨酸, 氨基, WX-001, GIP/GLP1双重激动剂肽, tirzepatide, 替尔泊肽, GIP/GLP1共激动剂, GIP和GLP-1双激动多肽, 双重肠促胰岛素肽, GLP 1, SEQ ID NOs: 1-4

**C. DOCUMENTS CONSIDERED TO BE RELEVANT**

| Category* | Citation of document, with indication, where appropriate, of the relevant passages | Relevant to claim No. |
|---|---|---|
| A | CN 110684082 A (SINOPEP JIANGSU INC. et al.) 14 January 2020 (2020-01-14)<br>entire document | 1-11 |
| A | CN 107207576 A (ELI LILLY AND COMPANY) 26 September 2017 (2017-09-26)<br>entire document | 1-11 |
| A | WO 2020159949 A1 (ELILILLY AND COMPANY) 06 August 2020 (2020-08-06)<br>entire document | 1-11 |
| A | CN 110903355 A (CHENGDU SHENGNUO BIOPHARM CO., LTD.) 24 March 2020 (2020-03-24)<br>entire document | 1-11 |
| A | CN 110642935 A (CHENGDU AODA BIOTECHNOLOGY CO., LTD.) 03 January 2020 (2020-01-03)<br>entire document | 1-11 |
| A | CN 104470948 A (ZEALAND PHARMA A/S) 25 March 2015 (2015-03-25)<br>entire document | 1-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 January 2022** | **28 February 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

INTERNATIONAL SEARCH REPORT

International application No.
PCT/CN2021/135180

C. DOCUMENTS CONSIDERED TO BE RELEVANT

| Category* | Citation of document, with indication, where appropriate, of the relevant passages | Relevant to claim No. |
| --- | --- | --- |
| A | CN 108699125 A (HANMI PHARMACEUTICAL CO., LTD.) 23 October 2018 (2018-10-23) entire document | 1-11 |

Form PCT/ISA/210 (second sheet) (January 2015)

## INTERNATIONAL SEARCH REPORT

International application No.
**PCT/CN2021/135180**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (January 2015)

**INTERNATIONAL SEARCH REPORT**
**Information on patent family members**

International application No.
**PCT/CN2021/135180**

| Patent document cited in search report | Publication date (day/month/year) | Patent family member(s) | Publication date (day/month/year) |
|---|---|---|---|
| CN 110684082 A | 14 January 2020 | WO 2021068251 A1 | 15 April 2021 |
| CN 107207576 A | 26 September 2017 | AU 2016205435 A1 | 08 June 2017 |
| | | AU 2016205435 B2 | 29 March 2018 |
| | | WO 2016111971 A1 | 14 July 2016 |
| | | IL 276492 D0 | 30 September 2020 |
| | | IL 276492 A | 29 April 2021 |
| | | BR 112017010596 A2 | 06 March 2018 |
| | | HU E045860 T2 | 28 January 2020 |
| | | AR 103242 A1 | 26 April 2017 |
| | | DO P2017000153 A | 15 July 2017 |
| | | CO 2017006737 A2 | 29 September 2017 |
| | | LT 3242887 T | 11 November 2019 |
| | | KR 20170092661 A | 11 August 2017 |
| | | KR 101957620 B1 | 13 March 2019 |
| | | ZA 201703930 B | 26 June 2019 |
| | | MD 3242887 T2 | 30 November 2019 |
| | | CA 2973352 A1 | 14 July 2016 |
| | | CA 2973352 C | 26 March 2019 |
| | | EA 201892057 A1 | 28 February 2019 |
| | | EA 035055 B1 | 22 April 2020 |
| | | TN 2017000198 A1 | 19 October 2018 |
| | | IL 252499 D0 | 31 July 2017 |
| | | IL 252499 A | 31 August 2020 |
| | | EP 3597662 A1 | 22 January 2020 |
| | | JO P20200119 A1 | 16 June 2017 |
| | | EA 202090392 A2 | 31 May 2020 |
| | | EA 202090392 A3 | 31 August 2020 |
| | | MA 50422 A | 26 August 2020 |
| | | JP 2018052933 A | 05 April 2018 |
| | | JP 6545766 B2 | 17 July 2019 |
| | | SI 3242887 T1 | 30 October 2019 |
| | | IL 281545 D0 | 31 May 2021 |
| | | HR P20191614 T1 | 13 December 2019 |
| | | MX 2017008927 A | 11 October 2017 |
| | | UA 118239 C2 | 10 December 2018 |
| | | JO 3575 B1 | 05 July 2020 |
| | | JP 2017507124 A | 16 March 2017 |
| | | JP 6219534 B2 | 25 October 2017 |
| | | JP 2019203000 A | 28 November 2019 |
| | | JP 6754867 B2 | 16 September 2020 |
| | | SV 2017005453 A | 27 August 2018 |
| | | CL 2017001760 A1 | 16 March 2018 |
| | | SG 11201705603 Y A | 30 August 2017 |
| | | PE 20170954 A1 | 13 July 2017 |
| | | EA 201791281 A1 | 30 November 2017 |
| | | EA 031591 B1 | 31 January 2019 |
| | | DK 3242887 T3 | 02 September 2019 |
| | | PL 3242887 T3 | 28 February 2020 |
| | | TW 201636362 A | 16 October 2016 |
| | | TW I582109 B | 11 May 2017 |

Form PCT/ISA/210 (patent family annex) (January 2015)

**INTERNATIONAL SEARCH REPORT**
**Information on patent family members**

International application No.
**PCT/CN2021/135180**

| Patent document cited in search report | | | Publication date (day/month/year) | Patent family member(s) | | | Publication date (day/month/year) |
|---|---|---|---|---|---|---|---|
| WO | 2020159949 | A1 | 06 August 2020 | EP | 3917950 | A1 | 08 December 2021 |
| | | | | KR | 20210110349 | A | 07 September 2021 |
| | | | | TW | 202043260 | A | 01 December 2020 |
| | | | | EC | SP21056124 | A | 31 August 2021 |
| | | | | AU | 2020216922 | A1 | 15 July 2021 |
| | | | | CN | 113330024 | A | 31 August 2021 |
| | | | | SG | 11202107123 T | A | 30 August 2021 |
| | | | | IL | 284446 | D0 | 31 August 2021 |
| | | | | CA | 3128023 | A1 | 06 August 2020 |
| | | | | CO | 2021009662 | A2 | 09 August 2021 |
| | | | | PE | 20211992 | A1 | 18 October 2021 |
| CN | 110903355 | A | 24 March 2020 | | None | | |
| CN | 110642935 | A | 03 January 2020 | CN | 111320683 | A | 23 June 2020 |
| CN | 104470948 | A | 25 March 2015 | TW | 201348252 | A | 01 December 2013 |
| | | | | TW | I689515 | B | 01 April 2020 |
| | | | | AR | 090937 | A1 | 17 December 2014 |
| | | | | EA | 201491918 | A1 | 30 July 2015 |
| | | | | EA | 028665 | B1 | 29 December 2017 |
| | | | | CA | 2872314 | A1 | 07 November 2013 |
| | | | | CA | 2872314 | C | 31 August 2021 |
| | | | | TR | 201815338 | T4 | 21 November 2018 |
| | | | | BR | 112014027348 | A2 | 27 June 2017 |
| | | | | EP | 2844669 | A1 | 11 March 2015 |
| | | | | EP | 2844669 | B1 | 01 August 2018 |
| | | | | PH | 12014502452 | A1 | 02 February 2015 |
| | | | | KR | 20150003910 | A | 09 January 2015 |
| | | | | KR | 102184241 | B1 | 01 December 2020 |
| | | | | JP | 2015517459 | A | 22 June 2015 |
| | | | | JP | 6228187 | B2 | 08 November 2017 |
| | | | | IN | 2304MUN2014 | A | 07 August 2015 |
| | | | | NZ | 702333 | A | 30 June 2017 |
| | | | | HK | 1208232 | A1 | 26 February 2016 |
| | | | | MX | 2014013318 | A | 28 September 2015 |
| | | | | MX | 356641 | B | 07 June 2018 |
| | | | | US | 2019135886 | A1 | 09 May 2019 |
| | | | | US | 2015299281 | A1 | 22 October 2015 |
| | | | | US | 10100097 | B2 | 16 October 2018 |
| | | | | AU | 2013255751 | A1 | 18 December 2014 |
| | | | | AU | 2013255751 | B2 | 05 October 2017 |
| | | | | WO | 2013164483 | A1 | 07 November 2013 |
| | | | | IL | 235463 | D0 | 31 December 2014 |
| | | | | SG | 11201407137 P | A | 27 November 2014 |
| CN | 108699125 | A | 23 October 2018 | EA | 201891359 | A1 | 31 January 2019 |
| | | | | EA | 038524 | B1 | 09 September 2021 |
| | | | | CO | 2018006982 | A2 | 10 July 2018 |
| | | | | PE | 20181494 | A1 | 18 September 2018 |
| | | | | DO | P2018000157 | A | 15 October 2018 |
| | | | | SG | 11201805586 S | A | 30 July 2018 |
| | | | | DO | P2018000159 | A | 30 September 2018 |
| | | | | JP | 2020171287 | A | 22 October 2020 |

Form PCT/ISA/210 (patent family annex) (January 2015)

**INTERNATIONAL SEARCH REPORT**
**Information on patent family members**

International application No.
**PCT/CN2021/135180**

| Patent document cited in search report | Publication date (day/month/year) | Patent family member(s) | | | Publication date (day/month/year) |
|---|---|---|---|---|---|
| | | TW | 201730207 | A | 01 September 2017 |
| | | PH | 12018501411 | A1 | 08 April 2019 |
| | | HK | 1258177 | A1 | 08 November 2019 |
| | | US | 2019153060 | A1 | 23 May 2019 |
| | | US | 10981967 | B2 | 20 April 2021 |
| | | IL | 260318 | D0 | 30 August 2018 |
| | | KR | 20190062344 | A | 05 June 2019 |
| | | BR | 112018013525 | A2 | 04 December 2018 |
| | | PH | 12018501409 | A1 | 08 April 2019 |
| | | KR | 20200096184 | A | 11 August 2020 |
| | | KR | 102285377 | B1 | 04 August 2021 |
| | | CA | 3010265 | A1 | 06 July 2017 |
| | | ZA | 201804997 | B | 29 May 2019 |
| | | IL | 260310 | D0 | 30 August 2018 |
| | | AU | 2019203888 | A1 | 20 June 2019 |
| | | AU | 2019203888 | B2 | 13 May 2021 |
| | | KR | 20190104958 | A | 11 September 2019 |
| | | KR | 102179392 | B1 | 16 November 2020 |
| | | US | 2019218269 | A1 | 18 July 2019 |
| | | CR | 20180380 | A | 07 December 2018 |
| | | EP | 3398961 | A1 | 07 November 2018 |
| | | EP | 3398961 | A4 | 12 June 2019 |
| | | AU | 2016382394 | A1 | 02 August 2018 |
| | | AU | 2016382394 | B2 | 04 July 2019 |
| | | CL | 2020002034 | A1 | 09 October 2020 |
| | | TN | 2018000228 | A1 | 04 October 2019 |
| | | US | 2018311315 | A1 | 01 November 2018 |
| | | US | 10400020 | B2 | 03 September 2019 |
| | | EA | 201891360 | A1 | 28 December 2018 |
| | | EA | 038544 | B1 | 13 September 2021 |
| | | KR | 20170080521 | A | 10 July 2017 |
| | | JP | 2019504055 | A | 14 February 2019 |
| | | JP | 6712322 | B2 | 17 June 2020 |
| | | HK | 1255834 | A1 | 30 August 2019 |
| | | US | 2021188937 | A1 | 24 June 2021 |
| | | TW | 201737945 | A | 01 November 2017 |
| | | TW | I731015 | B | 21 June 2021 |
| | | SG | 11201805573 R | A | 30 July 2018 |
| | | AU | 2019203891 | A1 | 20 June 2019 |
| | | AU | 2019203891 | B2 | 13 May 2021 |
| | | KR | 20200095436 | A | 10 August 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202011402979
- CN 202011409947X
- CN 202110432060
- CN 202110587056